# EUROPEAN PATENT SPECIFICATION

(11) **EP 3 699 175 B1**
(45) Date of publication and mention of the grant of the patent: **04.05.2022**
(21) Application number: 20158145.1
(22) Date of filing: 19.02.2020
(51) Int. Cl.: C07D 313/04, C08G 63/00, C09J 167/00

(54) **FUNCTIONALIZED ALIPHATIC POLYESTERS AND PROCESS FOR PRODUCING THE SAME**
FUNKTIONALISIERTE ALIPHATISCHE POLYESTER UND VERFAHREN ZU IHRER HERSTELLUNG
POLYESTERS ALIPHATIQUES FONCTIONNALISÉS ET LEUR PROCÉDÉ DE FABRICATION

(30) Priority: 19.02.2019 LU 101132
(43) Date of publication of application: 26.08.2020
(73) Proprietor: Kemijski Institut, 1000 Ljubljana (SI)
(72) Inventor: Zagar, Ema, 4000 Kranj (SI); Toplishek, Maria, 8250 Brezice (SI); Pahovnik, David, 4000 Kranj (SI)
(74) Representative: Zacco GmbH

(56) References cited:
- WO-A1-2007/106997
- WO-A2-2009/118538

## Description

The present invention relates to lactone monomers, which may be biomass-derived substituted caprolactone monomers, to a synthetic process for producing the same, to functionalized aliphatic polyester homopolymer, to functionalized aliphatic polyester copolymers of random, gradient, or block microstructure and to a respective process of ROP. The present invention is defined by the appended claims.

### FIELD OF THE INVENTION

The invention relates to substituted caprolactone monomers, to functionalized aliphatic polyesters prepared by ROP of said substituted caprolactone monomers, which are applicable in engineering and biomedical applications.

### BACKGROUND OF THE INVENTION

Due to the growing concern about plastic's environmental footprint, one of the key trends in modern polymer science is the development of materials which do not possess only useful properties, but also demonstrate inherent (bio)degradability and/or are derived from a renewable resource platform. For this reason, one of the most rapidly developing fields in synthetic polymer chemistry is the synthesis of polyesters with special accent on functionalized aliphatic polyesters. Namely, aliphatic polyesters are degradable, however, the major drawback of conventional aliphatic polyesters is the absence of functional groups along the polymer backbone, which limits broadening of their application fields. Moreover, introduction of functional groups in the aliphatic polyester backbone would make it possible to find the substitutes for many conventional nondegradable polymers. Most of the work in the field of aliphatic polyesters is pointed at their design for biomedical applications. At the same time, there are many examples of application of aliphatic polyesters in packaging and engineering purposes [1].

The most effective way to adjust the polymer properties for certain application is the introduction of functional groups in the monomer structure. For example, polar functional groups such as the ester, amide, and halogen are present in the structure of mass production polymers like polyacrylates, polyacrylamide, polyvinylchloride, etc. Among other functionalities, a nitrile group (for example in polyacrylonitrile) is known to improve polymer thermal stability, mechanical properties, polarity, and at the same time it enables the post-polymerization modifications.

Conventional cyano-polymers, like polyacrylonitrile (PAN) and cyanoacrylate, have found wide and diverse applications in engineering field. Repeating unit of polyacrylonitrile is present in the styrene acrylonitrile and acryl butadiene styrene (ABS) commodity copolymers [2], which are widely used in automotive industry, for manufacturing of medical devices, household and consumer goods, etc. Additionally, polyacrylonitrile is used for the production of textiles, paints, glues, while both cyano-polymers have been shown to be beneficial also for more demanding purposes. For example, PAN holds a leading role in carbon fiber synthesis, while the other carbon fiber precursors are used in much smaller share. Despite of intensive research on the production of carbon fibers from the lignocellulosic biomass derived chemicals, PAN-derived fibers still demonstrate superior mechanical properties [3].

Examples can be seen in WO 2007/106997 A1 and WO 2009/118538 A2 (2009-10-01) as these disclose homopolymers and block-copolymers, which are obtained from lactones via ring-opening polymerization.

Possible solution of the above-mentioned problems is the synthesis of biomass-derived functionalized aliphatic polyesters bearing polar functional groups in their structure. Therefore, the functionalized polyesters derived from a renewable resource platform represent an alternative for the oil-derived conventional polymers.

The most efficient way of aliphatic polyester synthesis is a ROP of the cyclic monomers (lactones, lactides). Extensive research has been performed on the synthesis of monosubstituted lactones (mostly caprolactones); i.e., with alkyl [4], halogen [5], carboxyl [6], ether [5], and other type of substituents, however, there are limited reports on the synthesis of lactone monomers, especially disubstituted caprolactone monomers bearing polar substituents. Additionally, a lot of success has been achieved in the development of (functionalized) lactone monomers from the bio-based chemicals [7], but all the products have been developed mainly for biomedical applications. Therefore, the purpose of the present invention relies on designing the synthetic pathway to the lactone monomers, especially disubstituted lactone monomers bearing polar substituents in the structure by using the bio-based chemicals. Moreover, the synthetic conditions for ROP of such monomers have been optimized to produce the bio-based and degradable alternatives to the nondegradable polyacrylonitrile, polyacrylates, and polyacrylamide polymers, for the use in engineering and biomedical fields.

Among all the known representatives of this class, the δ- and ε-lactones are most commonly used for the polyester synthesis due to, i.e. optimal kinetics of polymerization, relative simplicity of monomer synthesis, and stability of monomers during purification and storage conditions. One of the most common synthetic procedures to lactones is the Baeyer-Villiger oxidation of the corresponding cyclic ketone precursors [8].

### SUMMARY OF THE INVENTION

This invention now offers a solution for the problems described above.

A central aspect of the invention rests in a lactone monomer having a structure of general formula (I) 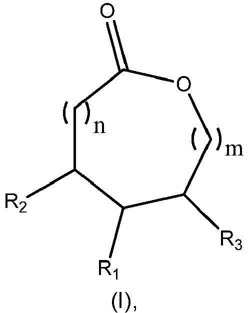 wherein
n is selected from 0, 1 or 2;
m is selected from 0, 1 or 2; and
m + n is 2;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
   if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
alternatively, R₅ and R₆ taken together with the nitrogen atom to which they are attached, may form a substituted or unsubstituted heterocyclyl.

These lactone monomers - which may even be derived from bio-mass - may be used to produce various polymers by undergoing ROP in a highly effective way delivering high quality polymers. These polymers may be easily degradable.

In a preferred embodiment the lactone monomer according to the invention has a structure of general formula (Ia)

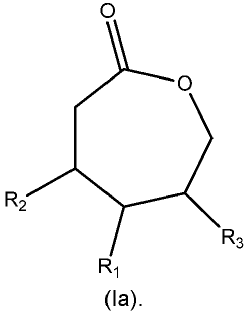

In another preferred embodiment of the invention the lactone monomer of the invention has a structure of either general formula (II) or of general formula (III)

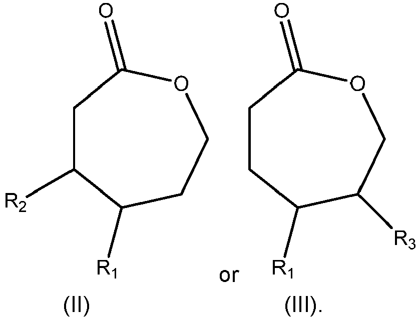

Another aspect of the invention refers to a process for producing a lactone monomer of the invention according to general formulas (I) and (Ia) comprising the final step of a Baeyer-Villiger oxidation of a ketone to the lactone monomer of Formula (I) following the below reaction-scheme: 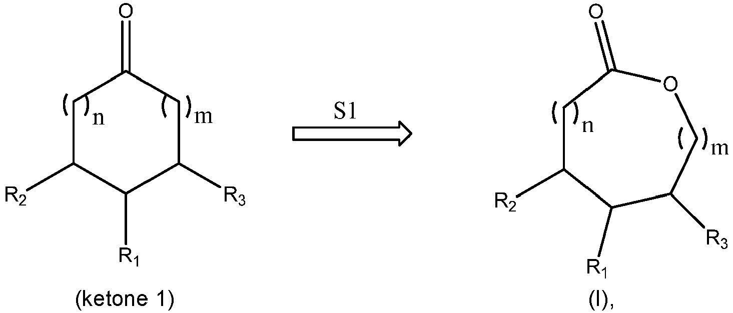 wherein the substituents in the ketone 1 and in the compound of formula (I) are as described below and in the reaction step S1
- an oxidant is added, preferably an oxidant selected from mCPBA, hydrogen peroxide, sodium perborate, peracetic acid, trifluoroperacetic acid, performic acid, 4-nitroperbenzoic acid is added, more preferably mCPBA is added;
- the solvent is an organic solvent, preferably is CH₂Cl₂, and
- the temperature is selected between -5° C and 90° C, preferably is room temperature.

In a preferred embodiment of this process for producing a lactone monomer of either general Formula (II) or (III), comprising the final step of a Baeyer-Villiger oxidation of a ketone to the lactone monomer of Formula (II) and (III) following the below reaction-scheme:

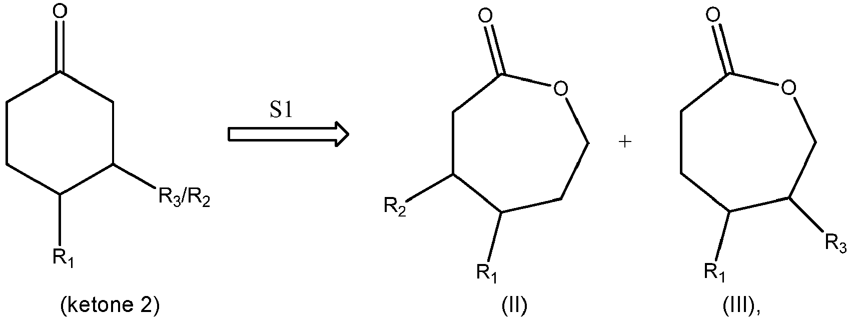

Another aspect of the invention is drawn to a functionalized aliphatic polyester homopolymer of general formula (IV) 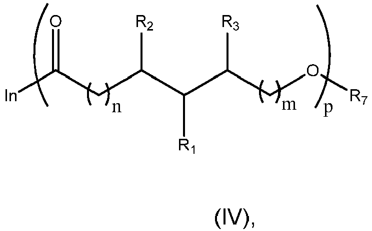 wherein the substituents in the compound of formula (IV) are as described below.

This aspect of the invention is also drawn to a functionalized aliphatic polyester homopolymer of general formula (IV), wherein the substituents in the compound of formula (IV) are as described below.

Yet, a further aspect of the invention is directed towards a functionalized aliphatic polyester random copolymer of general formula (VII) 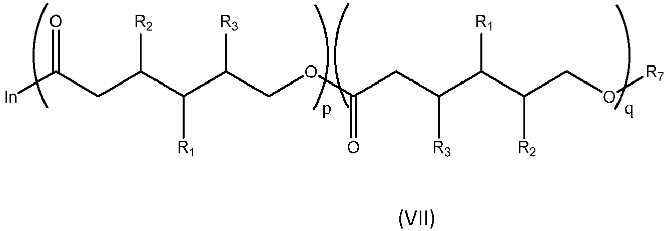 wherein the substituents in the compound of formula (VII) are as described below.

This aspect of the invention is also directed towards a functionalized aliphatic polyester random copolymer of general formula (VII), wherein the substituents in the compound of formula (VII) are as described below.

Another further aspect of the invention is drawn to a functionalized aliphatic polyester copolymer of random and block microstructure of general formula (VIII) or (IX) 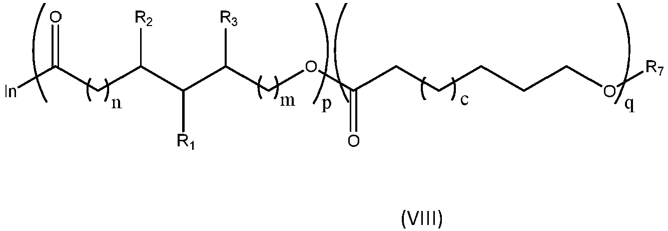 or 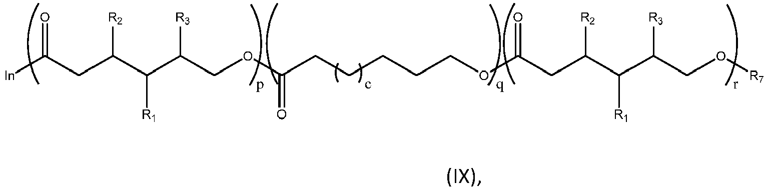 wherein the substituents in the compounds of formulas (VIII) and (IX) are as described below.

This further aspect of the invention is also drawn to a functionalized aliphatic polyester copolymer of random and block microstructure of general formula (VIII) or (IX), wherein the substituents in the compounds of formulas (VIII) and (IX) are as described below.

A final further aspect of the invention is focused on a process of ROP for the production of a functionalized aliphatic polyester homopolymer according to the invention, an aliphatic polyester random copolymer according to the invention or of a functionalized aliphatic polyester copolymer of random and block microstructure according to the invention, wherein the ROP is executed
in the presence of a basic, acidic, tin or aluminum catalyst; preferably in the presence of an aluminum alkyl or aluminum alkoxide catalyst;
preferably
in the presence of primary alcohol as initiator.

In general, this invention provides a synthetic method to produce lactone monomers like the preferred disubstituted caprolactones of the invention bearing electron-withdrawing groups (cyano, ester, amide, halogen, nitro), preferably by using biobase-derived chemicals. It also provides polymerization methods using such prepared lactone monomers/disubstituted caprolactones to prepare the respective functionalized aliphatic polyesters.

The synthetic scheme for the synthesis of the lactone monomers/disubstituted caprolactone monomers with different type of substituents was optimized. In principle, it includes two steps: i) Diels-Alder reaction followed by *in-situ* TMS-enol cleavage, and in the next step ii) the Baeyer-Villiger oxidation.

In a preferred example of the invention, the Diels-Alder solvent-free cycloaddition of 2-trimethylsililoxy-1,4-butadiene (enol ether of methyl vinyl ketone, which could be obtained from the biomass processing product - levulinic acid), and fumaronitrile (derivative of fumaric acid, which is also obtained from the lignocellulosic biomass), followed by acidic cleavage of the TMS-enol, results in the 3,4-dicyanosubstituted cyclohexanone formation in high yield. The ketone is then oxidized with mCPBA via the Baeyer-Villiger reaction. It was established, that the thus produced dicyanosubstituted caprolactone is a solid with a high melting point (Tₘ = ~180°C). It can only be dissolved in very polar solvents like acetonitrile, acetone, dimethylformamide, and dimethylsulfoxide. Compared to ε-caprolactone, these thus produced and preferred disubstituted monomers of the invention are found to be less active in acid catalyzed ROP, but much more active in anionic or pseudo-anionic conditions. The best polymerization conditions leading to no side reactions were achieved with aluminum alkoxide catalysts - diethyl aluminum ethoxide, methylaluminum bis(2,6-diphenylphenolate) and methylaluminum bis(2,6-di-tert-butyl-4-methylphenolate). The second and third catalyst yielded the best results since the obtained polymers had narrow molecular weight distributions.

The thus obtained and preferred cyano disubstituted polycaprolactone of the invention demonstrates distinctive properties: high glass transition temperature (T_{g}: ~70°C for Mₙ = ~20 kDa) and thermal stability up to 220 °C, which is much higher compared to other substituted polycaprolactones known in the art, making the new polymer suitable candidate for various engineering applications. On the other hand, the presence of cyano groups in the polymer enables specific post-polymerization modifications.

### DETAILED DESCRIPTION OF THE INVENTION

In a Main aspect (A) the invention is directed to a lactone monomer having a structure of general formula (I) 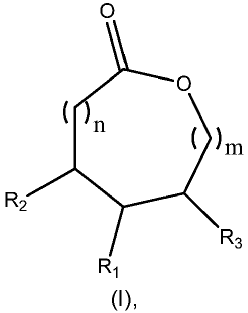 wherein
n is selected from 0, 1 or 2;
m is selected from 0, 1 or 2; and
m + n is 2;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl.

These lactone monomers - which may even be derived from biomass - may be used to produce various polymers by undergoing ROP in a highly effective way delivering high quality polymers. These polymers may show a good degree of biodegradation by selecting the fitting lactone monomers and their substitution pattern.

For clarity purposes, all groups and definitions described in the description and referring to compounds of general Formula (I), also apply to compounds of other general Formulas were applicable and if they are included in the respected other general Formula (I). The same applies *mutatis mutandis* also in case of other general formulas if they are included in a respected other general basic Formula.

In the context of this invention, alkyl is understood as meaning saturated, linear or branched hydrocarbons, which may be unsubstituted or substituted once or several times. It encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc. Preferably alkyl is understood in the context of this invention as C₁₋₈alkyl like methyl, ethyl, propyl, butyl, pentyl, hexyl, heptyl, or octyl; preferably is C₁₋₆alkyl like methyl, ethyl, propyl, butyl, pentyl, or hexyl; more preferably is C₁₋₄alkyl like methyl, ethyl, propyl or butyl.

Alkenyl is understood as meaning unsaturated, linear or branched hydrocarbons, which may be unsubstituted or substituted once or several times. It encompasses groups like e.g. -CH=CH-CH₃. The alkenyl radicals are preferably vinyl (ethenyl), allyl (2-propenyl). Preferably in the context of this invention alkenyl is C₂₋₁₀-alkenyl or C₂₋₈-alkenyl like ethylene, propylene, butylene, pentylene, hexylene, heptylene or octylene; or is C₂₋₆-alkenyl like ethylene, propylene, butylene, pentylene, or hexylene; or is C₂₋₄-alkenyl, like ethylene, propylene, or butylenes.

Alkynyl is understood as meaning unsaturated, linear or branched hydrocarbons, which may be unsubstituted or substituted once or several times. It encompasses groups like e.g. -C=C-CH₃ (1-propinyl). Preferably alkynyl in the context of this invention is C₂₋₁₀-alkynyl or C₂₋₈-alkynyl like ethyne, propyne, butyene, pentyne, hexyne, heptyne, or octyne; or is C₂₋₆-alkynyl like ethyne, propyne, butyene, pentyne, or hexyne; or is C₂₋₄-alkynyl like ethyne, propyne, butyene, pentyne, or hexyne.

Aliphatic or aliphatic group is understood as meaning a linear or branched hydrocarbon (group), which may be unsubstituted or substituted once or several times. Aliphatic encompasses alkyl, alkenyl and alkynyl.

In connection with aliphatic, alkyl (also in alkylaryl, alkylheterocyclyl or alkylcycloalkyl), alkenyl, alkynyl and O-alkyl - unless defined otherwise - the term substituted in the context of this invention is understood as meaning replacement of at least one hydrogen radical on a carbon atom by halogen (F, CI, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c'}, or a linear or branched, substituted or unsubstituted -OC₁₋₆ alkyl, with R_{c} and R_{c'} being represented by H or by a linear or branched, substituted or unsubstituted C₁₋₆-alkyl, wherein when different radicals Rc or Rc' are present simultaneously in the compound of the same general Formula they may be identical or different.

More than one replacement on the same molecule and also on the same carbon atom is possible with the same or different substituents. This includes for example 3 hydrogens being replaced on the same C atom, as in the case of CF₃, or at different places of the same molecule, as in the case of e.g. -CH(OH)-CH=CH-CHCl₂.

Aryl is understood as meaning 5 to 18 membered mono or polycyclic ring systems with at least one aromatic ring but without heteroatoms even in only one of the rings. Examples are phenyl, naphthyl, fluoranthenyl, fluorenyl, tetralinyl, indanyl, 9H-fluorenyl or anthracenyl radicals, which can be unsubstituted or once or several times substituted. Most preferably aryl is understood in the context of this invention as phenyl, naphthyl or anthracenyl, preferably is phenyl.

A heterocyclyl radical or group (also called heterocyclyl hereinafter) is understood as meaning 5 to 18 membered mono or poly heterocyclic ring systems, with at least one saturated or unsaturated ring which contains one or more heteroatoms selected from the group consisting of nitrogen, oxygen and/or sulfur in the ring. A heterocyclic group can also be substituted once or several times.

A heterocyclyl is a heterocyclic ring system of one or more saturated or unsaturated rings of which at least one ring contains one or more heteroatoms selected from the group consisting of nitrogen, oxygen and/or sulfur in the ring; preferably is a heterocyclic ring system of one or two saturated or unsaturated rings of which at least one ring contains one or more heteroatoms selected from the group consisting of nitrogen, oxygen and/or sulfur in the ring, more preferably is selected from oxazepan, pyrrolidine, imidazole, oxadiazole, tetrazole, azetidine, pyridine, pyrimidine, piperidine, piperazine, benzofuran, benzimidazole, indazole, benzothiazole, benzodiazole, thiazole, benzothiazole, tetrahydropyran, morpholine, indoline, furan, triazole, isoxazole, pyrazole, thiophene, benzothiophene, pyrrole, pyrazine, pyrrolo[2,3b]pyridine, quinoline, quinolone, isoquinoline, tetrahydrothienopyridine, phthalazine, benzo-1,2,5-thiadiazole, indole, benzotriazole, benzoxazole oxopyrrolidine, benzodioxolane, benzodioxane, carbazole, oxaspirodecan or thiazole.

In the context of this invention cycloalkyl is understood as meaning saturated and unsaturated (but not aromatic) cyclic hydrocarbons (without a heteroatom in the ring), which can be unsubstituted or once or several times substituted. Furthermore, C₃₋₄-cycloalkyl represents C3- or C4-cycloalkyl, C₃₋₅-cycloalkyl represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C5-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. Examples are cyclopropyl, 2-methylcyclopropyl, cyclopropylmethyl, cyclobutyl, cyclopentyl, cyclopentylmethyl, cyclohexyl, cycloheptyl, cyclooctyl, and also adamantly. Preferably in the context of this invention cycloalkyl is C₃₋₈cycloalkyl like cyclopropyl, cyclobutyl, cyclopentyl, cyclohexyl, cycloheptyl, or cyclooctyl; or is C₃₋₇cycloalkyl like cyclopropyl, cyclobutyl, cyclopentyl, cyclohexyl, or cycloheptyl; or is C₃₋₆cycloalkyl like cyclopropyl, cyclobutyl, cyclopentyl or cyclohexyl, especially cyclopentyl or cyclohexyl.

In the context of this invention alkylaryl is understood as meaning an aryl group (see above) being connected to another atom through a C1-6-alkyl (see above) which may be branched or linear and is unsubstituted or substituted once or several times. Preferably alkylaryl is understood as meaning an aryl group (see above) being connected to another atom through 1 to 4 (-CH2-) groups. Most preferably alkylaryl is benzyl (i.e. -CH2-phenyl).

In the context of this invention alkylheterocyclyl is understood as meaning an heterocyclyl group (see above) being connected to another atom through a C₁₋₆-alkyl (see above) which may be branched or linear and is unsubstituted or substituted once or several times. Preferably alkylheterocyclyl is understood as meaning a heterocyclyl group (see above) being connected to another atom through 1 to 4 (-CH₂-) groups.

In the context of this invention alkylcycloalkyl is understood as meaning an cycloalkyl group (see above) being connected to another atom through a C₁₋₆-alkyl (see above) which may be branched or linear and is unsubstituted or substituted once or several times. Preferably alkylcycloalkyl is understood as meaning a cycloalkyl group (see above) being connected to another atom through 1 to 4 (-CH₂-) group.

Aromatic or aromatic group is understood as meaning an aromatic ring system containing at least on aromatic ring, which may be unsubstituted or substituted once or several times. Aromatic encompasses aryl and may encompass heterocyclyl (if it contains an aromatic ring)..

In connection with aromatic, aryl (including alkyl-aryl), cycloalkyl (including alkyl-cycloalkyl), or heterocyclyl (including alkyl-heterocyclyl), substituted is understood - unless defined otherwise - as meaning substitution of the ring-system of the aryl or alkyl-aryl, cycloalkyl or alkyl-cycloalkyl; heterocyclyl or alkyl-heterocyclyl with one or more of halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , -NR_{c}S(O)₂R_{c'} , =O, -OCH₂CH₂OR_{c}, - NR_{c}C(O)NR_{c'}R_{c"}, -S(O)₂NR_{c}R_{c'}, -NR_{c}S(O)₂NR_{c'}H_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl, wherein when different radicals Rc, Rc' or Rc" are present simultaneously in the compound of the same general Formula they may be identical or different.

Moreover, in connection with cycloalkyl (including alkyl-cycloalkyl), or heterocyclyl (including alkylheterocyclyl) namely non-aromatic heterocyclyl (including non-aromatic alkyl-heterocyclyl), substituted is also understood - unless defined otherwise - as meaning substitution of the ring-system of the cycloalkyl or alkyl-cycloalkyl; non-aromatic heterocyclyl or non-aromatic alkyl-heterocyclyl with =O.

In the context of this invention the term "acyl is understood as meaning a chemical substituent, obtained by the removal of hydroxylic group from carboxylic acid. The "acyl" may be an aliphatic or aromatic acyl depending on which chemical structure the substituent carrying the acyl group has. Preferably, acyl is selected from acetyl.

In the context of this invention the term "alkoxide" is understood as meaning a conjugate base of an alcohol consisting of an alkyl (or in case of an "aliphatic alkoxide and aliphatic group) bonded to a negatively charged oxygen atom. Preferably, the alkoxide is methoxide, ethoxide, propoxide and butoxide, more preferably it is ethoxide.

In the context of this invention the term "primary alcohol" is understood as meaning a chemical substance with a primary alcohol moiety. Preferably, the "primary alcohol is selected from methanol, ethanol, propanol and butanol.

In the context of this invention the term "oxidant" (also "oxidizing agent") is understood as meaning a substance that has the ability to oxidize other substances being reduced in return. Preferably, the oxidant is seleted from mCPBA, hydrogen peroxide, sodium perborate, peracetic acid, trifluoroperacetic acid, performic acid, and 4-nitroperbenzoic acid; more preferably is mCPBA.

In the context of this invention the term "catalyst" is understood as meaning any catalyst that ameliorates the reaction conditions of a process according to the inventions. Preferably, the catalyst is selected from basic, acidic, tin or aluminum catalysts; more preferably, the catalyst is an aluminum catalyst; most preferably, the catalyst is an aluminum catalyst, selected from an aluminum alkyl catalyst or aluminum alkoxide catalysts.

In the context of this invention the term "aluminium alkoxide catalyst" is understood as meaning a catalyst showing an alkoxide moiety and an aluminium ion. Preferably, the aluminium alkoxide catalyst is methylaluminum bis(2,6-diphenylphenolate)

In the context of this invention the term "aluminum alkyl catalyst" is understood as meaning a catalyst showing an alkyl moiety and an aluminum ion. Preferably, the aluminium alkyl catalyst is selected from diethyl aluminum ethoxide.

In the context of this invention the term "aliphatic amino group" is understood as meaning an aliphatic group substituting an amino group, being either a primary or secondary amine, preferably being a primary or secondary aliphatic amine (or amino group), more preferably being a primary aliphatic amine (or amino group).

In the context of this invention the term "Initiator" (or "In") is understood as meaning a molecule with which the first monomer ring is opened. Preferably, the initiator is a substituted or unsubstituted aliphatic alkoxide or a primary or secondary substituted or unsubstituted aliphatic amino group, preferably is ethoxide.

In a preferred embodiment of the lactone monomer of the invention (Aspect A)
one of R₂ or R₃ is H
with the condition that
   a) if R₂ is H, m is not 0; or
   b) if R₃ is H, n is not 0.

In a preferred embodiment of the lactone monomer of the invention (Aspect A) the lactone monomer has a structure of general formula (Ia) 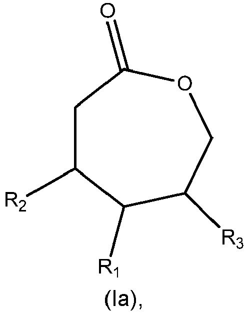 wherein
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl or substituted, unsubstituted O-alkyl, or substituted or unsubstituted O-aryl;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
alternatively, R₅ and R₆ taken together with the nitrogen atom to which they are attached, may form a substituted or unsubstituted heterocyclyl.

In another preferred embodiment (Aspect A') of the lactone monomer of the invention (Aspect A) the lactone monomer has a structure of either general formula (II) or of general formula (III) 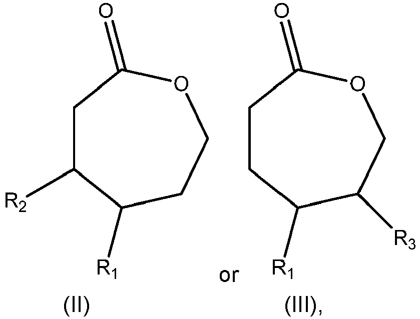 wherein
R₁ is selected from CN, COOR₄, CONR₅R₆, halogen or NO₂;
R₂ is selected from CN, COOR₄, CONR₅R₆, halogen or NO₂;
R₃ is selected from CN, COOR₄, CONR₅R₆, halogen or NO₂;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
alternatively, R₅ and R₆ taken together with the nitrogen atom to which they are attached, may form a substituted or unsubstituted heterocyclyl.

In a further aspect of the invention (Aspect B) the invention is directed at a process for producing a lactone monomer of the invention (Aspect A) comprising the final step of a Baeyer-Villiger oxidation of a ketone to the lactone monomer of Formula (I) following the below reaction-scheme: 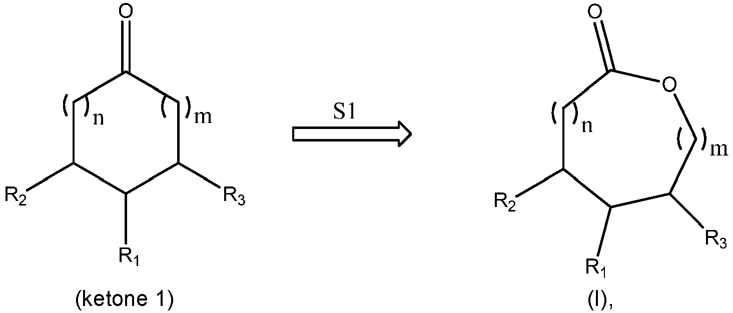 wherein in the reaction step S1
- an oxidant is added;
- the solvent is an organic solvent; and
- the temperature is selected between -5° C and 90° C;
and wherein in the ketone 1 and in the compound of formula (I)
n is selected from 0, 1 or 2;
m is selected from 0, 1 or 2; and
m + n is 2;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
   if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
alternatively, R₅ and R₆ taken together with the nitrogen atom to which they are attached, may form a substituted or unsubstituted heterocyclyl.

Preferably in the process according to the invention of Aspect B one of R₂ or R₃ is H, with the condition that if R₂ is H, m is not 0; or if R₃ is H, n is not 0 and/or
in the process according to the invention of Aspect B n and m are 1.

Preferably, in the process according to the invention of Aspect B in the reaction step S1 an oxidant selected from mCPBA, hydrogen peroxide, sodium perborate, peracetic acid, trifluoroperacetic acid, performic acid, 4-nitroperbenzoic acid is added; preferably m(mCPBA is added.

Preferably, in the process according to the invention of Aspect B in the reaction step S1 the solvent is CH₂Cl₂.

Preferably, in the process according to the invention of Aspect B in the reaction step S1 the temperature is selected between 15° C and 30° C, preferably is room temperature.

A preferred embodiment (Aspect B') of the process according to the invention (Aspect B) for producing a lactone monomer of one of either Formulas (II) or (III) (Aspect A') comprises the final step of a Baeyer-Villiger oxidation of a ketone to the lactone monomer of Formula (II) and (III) following the below reaction-scheme: 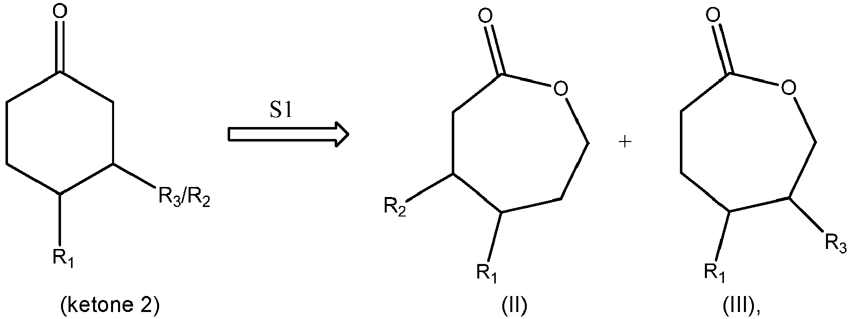 wherein in the reaction step S1
- an oxidant is added;
- the solvent is an organic solvent; and
- the temperature is selected between -5° C and 90° C;
and wherein in the ketone 2 and in the compounds of formulas (II) and (III)
R₁ is selected from CN, COOR₄, CONR₅R₆, halogen or NO₂;
R₂ is selected from CN, COOR₄, CONR₅R₆, halogen or NO₂;
R₃ is selected from CN, COOR₄, CONR₅R₆, halogen or NO₂;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl.

Preferably, in the process according to the invention of Aspect B' in the reaction step S1 an oxidant selected from mCPBA, hydrogen peroxide, sodium perborate, peracetic acid, trifluoroperacetic acid, performic acid, 4-nitroperbenzoic acid is added, preferably mCPBA is added.

Preferably, in the process according to the invention of Aspect B' in the reaction step S1 the solvent is CH₂Cl₂.

Preferably, in the process according to the invention of Aspect B' in the reaction step S1 the temperature is selected between 15° C and 30° C, preferably is room temperature.

In a preferred embodiment (Aspect B") of the process according to the invention (Aspect B') the ketone 2 is provided by a sequence of a Diels-Alder reaction (S2) followed by TMS-enol cleavage, following the below reaction-scheme: 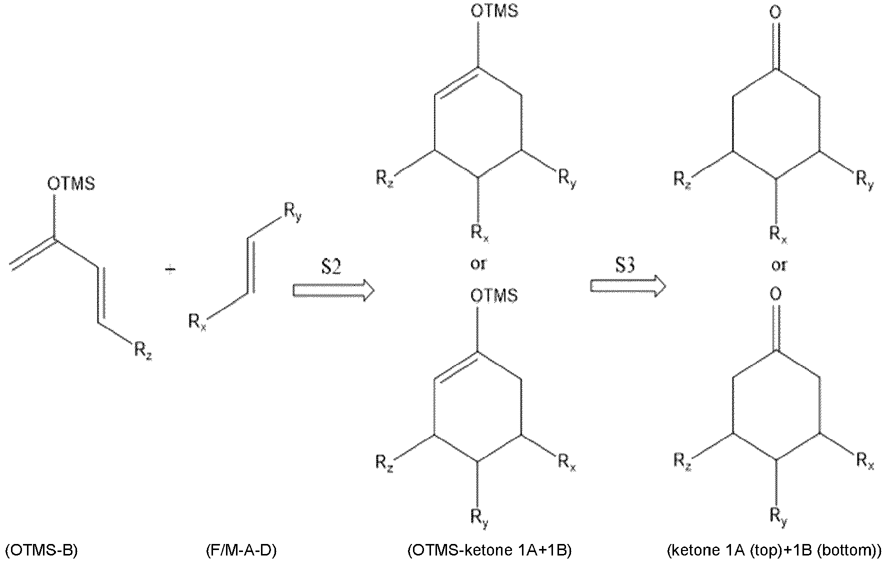 wherein (OTMS-B) is 3 or 2-trimethylsililoxy-substituted diene, preferably is 2-trimethylsililoxy-1,4-butadiene, (F/M-A-D) is a fumaric/maleic acid derivative (F/M-A-D), preferably wherein the 2-Trimethylsililoxy-1,4-butadiene (OTMS-B) and/or the fumaric/maleic acid derivative (F/M-A-D) are synthesized from chemicals, derived from oil- or biomass;
wherein
Rₓ is selected from CN, COOR₄, CONR₅R₆, halogen or NO₂;
R_{y} is selected from CN, COOR₄, CONR₅R₆, halogen or NO₂;
R_{z} is selected from H, substituted or unsubstituted alkyl or substituted, unsubstituted O-alkyl, or substituted or unsubstituted O-aryl
wherein in the reaction step S2
   - the temperature is selected between 25° C and 180° C, preferably is 120°C to 140°C, most preferably is 130°C;
   - the reaction time is selected from 1 hour to 24 hours, preferably from 3 hours to 5 hours, most preferably is 4 hours;
wherein in the reaction step S3
   - acetonitrile, ethanol or methanol, formic acid or sodium carbonate is added,
   - the reaction mixture is filtered and evaporated
   - and optionally the final product is additionally purified by a vacuum distillation or by chromatography.

Then finally, in this preferred embodiment (Aspect B") of the process according to the invention (Aspect B'), when R_{z} is H, the ketone 2 (above; Aspect B') is provided from the ketone 1A (see the formula in the top right corner of the respective reaction-scheme above) in which Rₓ is R₁ and R_{y} is R₃/R₂.

It might be preferred if in the overall reaction/process of (Aspect B"; above) in step S2 hydroquinone is added.

Preferably in the process according to Aspect B" (OTMS-B) is 2-trimethylsililoxy-1,4-butadiene; and/or (F/M-A-D) is a fumaric acid derivative.

Preferably in the process according to Aspect B" the (OTMS-B) and/or the (F/M-A-D) are synthesized from chemicals, derived from oil- or biomass, more preferably the 2-Trimethylsililoxy-1,4-butadiene (OTMS-B) and/or the fumaric/maleic acid derivative (F/M-A-D) are synthesized from chemicals, derived from oil- or biomass; most preferably the 2-Trimethylsililoxy-1,4-butadiene (OTMS-B) and the fumaric/maleic acid derivative (F/M-A-D) are synthesized from chemicals, derived from oil- or biomass.

Preferably, in the process according to the invention of Aspect B" in the reaction step S2 the reaction time is selected from 3 hours to 5 hours, preferably is 4 hours.

Preferably, in the process according to the invention of Aspect B" in the reaction step S2 the temperature is selected between 120°C to 140°C, preferably is selected as 130°C.

In a further aspect of the invention (Aspect C) the invention is directed at a functionalized aliphatic polyester homopolymer of general formula (IV) 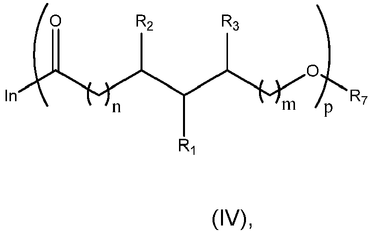 wherein
n is selected from 0, 1 or 2;
m is selected from 0, 1 or 2; and
m + n is 2;
p is selected from 5-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
   if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl;
and

In is substituted or unsubstituted aliphatic alkoxide or a primary or secondary substituted or unsubstituted aliphatic amino group.

This further aspect of the invention (Aspect C) is also directed at a functionalized aliphatic polyester homopolymer of general formula (IV), wherein the substituents in the compounds of formula (IV) are as described immediately above.

In a preferred embodiment of the functionalized aliphatic polyester homopolymer of general formula (IV) of the invention (Aspect C) one of R₂ or R₃ is H, with the condition that if R₂ is H, m is not 0; or if R₃ is H, n is not 0.

In another preferred embodiment of the functionalized aliphatic polyester homopolymer of general formula (IV) of the invention (Aspect C) In is ethoxide.

In another preferred embodiment of the functionalized aliphatic polyester homopolymer of general formula (IV) of the invention (Aspect C)
a) the functionalized aliphatic polyester homopolymer (Aspect C) is of general formula (IVa) 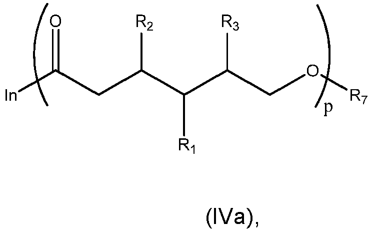 wherein
   p is selected from 5-500;
   R₁ is selected from CN, COOR₄, CONR₅R₆, halogen or NO₂;
   one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
   R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
   R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
   alternatively, R₅ and R₆ taken together with the nitrogen atom to which they are attached, may form a substituted or unsubstituted heterocyclyl;
   R₇ is selected from H, substituted or unsubstituted aliphatic or aromatic acyl, substituted or unsubstituted alkyl; or
b) the functionalized aliphatic polyester homopolymer is of general formula (V) or (VI) (Aspect C') 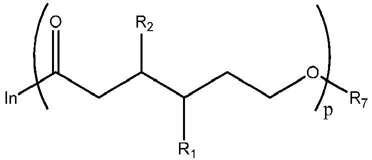 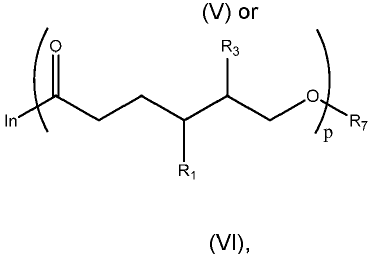 wherein
   p is selected from 5-500;
   R₁ is selected from CN, COOR₄, CONR₅R₆, halogen or NO₂;
   R₂ is selected from CN, COOR₄, CONR₅R₆, halogen or NO₂;
   R₃ is selected from CN, COOR₄, CONR₅R₆, halogen or NO₂;
   R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
   R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
   alternatively, R₅ and R₆ taken together with the nitrogen atom to which they are attached, may form a substituted or unsubstituted heterocyclyl;
   R₇ is selected from H, substituted or unsubstituted aliphatic or aromatic acyl, substituted or unsubstituted alkyl.

This preferred embodiment of the functionalized aliphatic polyester homopolymer of general formula (IV) of the invention (Aspect C) is also drawn to a functionalized aliphatic polyester homopolymer (Aspect C) of general formula (IVa) (see option a)), wherein the substituents in the compounds of formula (IVa) are as described immediately above for option a); or
this preferred embodiment of the functionalized aliphatic polyester homopolymer of general formula (IV) of the invention (Aspect C) is also drawn to a functionalized aliphatic polyester homopolymer is of general formula (V) or (VI) (Aspect C').(see option b)), wherein the substituents in the compounds of formula (IVa) are as described immediately above for Option b).

In a further aspect of the invention (Aspect D) the invention is directed at a functionalized aliphatic polyester random copolymer of general formula (VII) 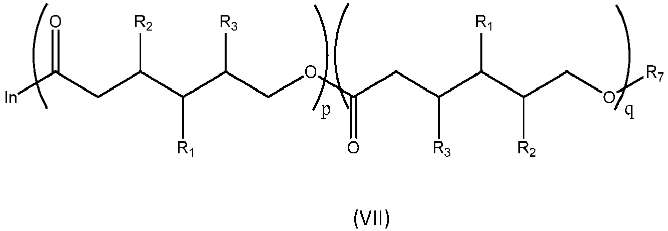
p is selected from 5-500,
q is selected from 5-500,
p+q is 10-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl.

This further aspect of the invention (Aspect D) is also directed at a functionalized aliphatic polyester random copolymer of general formula (VII), wherein the substituents in the compounds of formula (VII) are as described immediately above.

In a further aspect of the invention (Aspect E) the invention is directed at a functionalized aliphatic polyester copolymer of random and block microstructure of general formula (VIII) or (IX) 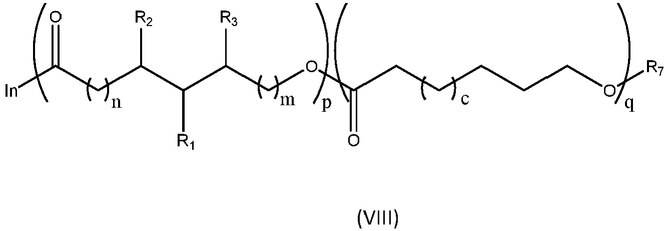 or 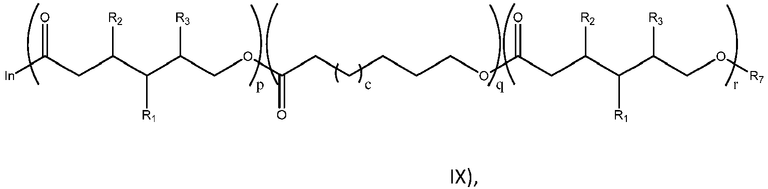 wherein
n is selected from 0, 1 or 2,
m is selected from 0, 1 or 2, and
m + n is 2,
c is selected from 0 or 1;
p is selected from 5-500,
q is selected from 5-500, and
r is selected from 5-500,
p+q+r is 15-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
   if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl.

This further aspect of the invention (Aspect E) is also directed at a functionalized aliphatic polyester copolymer of random and block microstructure of general formula (VIII) or (IX), wherein the substituents in the compounds of formula (VIII) or (IX) are as described immediately above.

In a preferred embodiment of Aspect D one of R₂ or R₃ is H,
with the condition that if R₂ is H, m is not 0; or if R₃ is H, n is not 0;

In another preferred embodiment of the functionalized aliphatic polyester copolymer of random and block microstructure according to Aspect E
a) the functionalized aliphatic polyester copolymer of random and block microstructure (Aspect E) is of general formula (VIIIa) or (IXa) 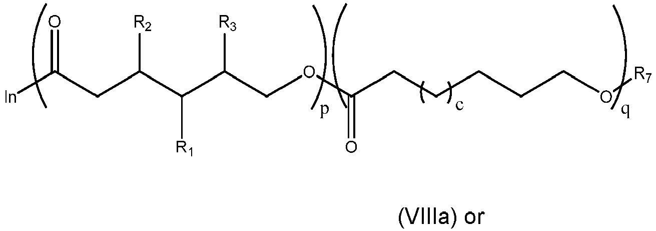 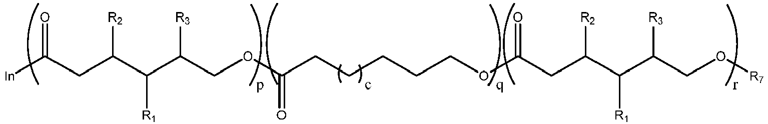 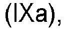 wherein
   c is selected from 0 or 1,
   p is selected from 5-500,
   q is selected from 5-500, and
   r is selected from 5-500,
   p+q+r is 15-500;
   R₁ is selected from CN, COOR₄, CONR₅R₆, halogen or NO₂;
   one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
   R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
   R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
   R₇ is selected from H, substituted or unsubstituted aliphatic or aromatic acyl, substituted or unsubstituted alkyl; or
b) the functionalized aliphatic polyester copolymer of random and block microstructure (Aspect E') is of general formula (X) or (XI) and (XII) or (XIII) 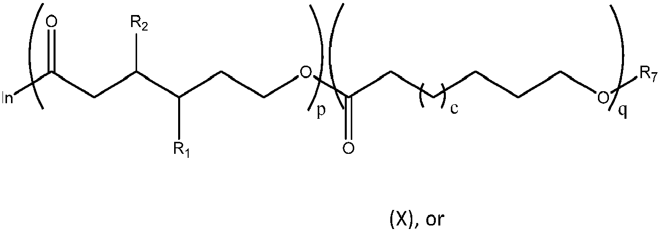 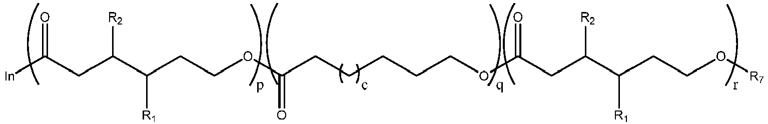 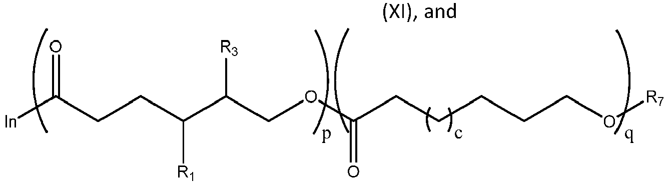 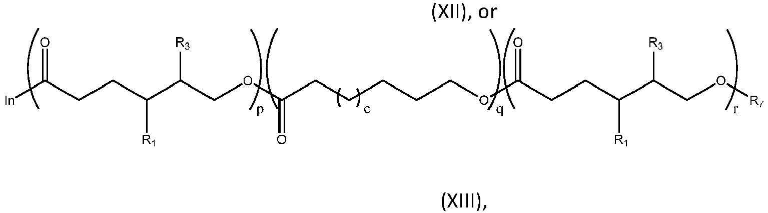 wherein
   c is selected from 0 or 1;
   p is selected from 5-500,
   q is selected from 5-500, and
   r is selected from 5-500,
   p+q+r is 15-500,
   R₁ is selected from CN, COOR₄, CONR₅R₆, halogen or NO₂;
   R₂ is selected from CN, COOR₄, CONR₅R₆, halogen or NO₂;
   R₃ is selected from CN, COOR₄, CONR₅R₆, halogen or NO₂;
   R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
   R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl,
   alternatively, R₅ and R₆ taken together with the nitrogen atom to which they are attached, may form a substituted or unsubstituted heterocyclyl;
   R₇ is selected from H, substituted or unsubstituted aliphatic or aromatic acyl, substituted or unsubstituted alkyl.

This preferred embodiment of the functionalized aliphatic polyester copolymer of random and block microstructure according to Aspect E is also drawn to a functionalized aliphatic polyester copolymer of random and block microstructure (Aspect E) of general formula (VIIIa) or (IXa) (see option a)), wherein the substituents in the compounds of formula (VIIIa) or (IXa) are as described immediately above for option a); or
this preferred embodiment of the functionalized aliphatic polyester copolymer of random and block microstructure according to Aspect E is also drawn to a functionalized aliphatic polyester copolymer of random and block microstructure (Aspect E') of general formula (X) or (XI) and (XII) or (XIII), wherein the substituents in the compounds of formula formula (X) or (XI) and (XII) or (XIII) are as described immediately above for option b).

In a further preferred embodiment of the invention of the lactone monomer according to Aspect A, the process according to aspect B, the functionalized aliphatic polyester homopolymer according to Aspect C, the functionalized aliphatic polyester random copolymer of Aspect D or the functionalized aliphatic polyester copolymer of random and block microstructure according to Aspect E
R₄ is selected from substituted or unsubstituted C₁₋₁₀-alkyl, substituted or unsubstituted aryl, or substituted or unsubstituted C₁₋₄-alkyl-aryl;
R₅ and R₆ are independently of each other selected from substituted or unsubstituted C₁₋₁₀-alkyl, substituted or unsubstituted aryl, substituted or unsubstituted C₁₋₄-alkyl-aryl, substituted or unsubstituted mono- or bi-cyclic heterocyclyl, or substituted or unsubstituted mono- or bi-cyclic C₁₋₄-alkyl-heterocyclyl,
alternatively, R₅ and R₆ taken together with the nitrogen atom to which they are attached, may form a substituted or unsubstituted mono- or bi-cyclic heterocyclyl;
preferably
R₄ is selected from substituted or unsubstituted C₁₋₄-alkyl, substituted or unsubstituted aryl, or substituted or unsubstituted C₁₋₄-alkyl-aryl;
R₅ and R₆ are independently of each other selected from substituted or unsubstituted C₁₋₄-alkyl, substituted or unsubstituted phenyl, substituted or unsubstituted benzyl or substituted or unsubstituted mono- or bi-cyclic heterocyclyl,
alternatively, R₅ and R₆ taken together with the nitrogen atom to which they are attached, may form a substituted or unsubstituted morpholine;
more preferably
R₄ is selected from unsubstituted C₁₋₄-alkyl, substituted or unsubstituted phenyl or substituted or unsubstituted benzyl;
R₅ and R₆ are independently of each other selected from substituted or unsubstituted C₁₋₄-alkyl, substituted or unsubstituted phenyl or substituted or unsubstituted benzyl, alternatively, R₅ and R₆ taken together with the nitrogen atom to which they are attached, may form an unsubstituted morpholine.

In a further preferred embodiment of the invention of the functionalized aliphatic polyester homopolymer according to Aspect C, the functionalized aliphatic polyester random copolymer of Aspect D or the functionalized aliphatic polyester copolymer of random and block microstructure according to Aspect E
R₇ is selected from H, substituted or unsubstituted aliphatic or aromatic acyl, substituted or unsubstituted alkyl;
preferably R₇ is selected from H, acetyl or unsubstituted alkyl;
more preferably R₇ is H.

In a further preferred embodiment of the invention of the lactone monomer according to Aspect A, of the process according to Aspect B, a functionalized aliphatic polyester homopolymer according to Aspect C, the functionalized aliphatic polyester random copolymer of Aspect D, or a functionalized aliphatic polyester copolymer of random and block microstructure according to any one of claims Aspect E, wherein
R₁ is selected from CN, COOR₄, CONR₅R₆; and
R₂ is selected from CN, COOR₄, CONR₅R₆, while R₃ is selected from H, substituted or unsubstituted alkyl; substituted or unsubstituted O-alkyl or substituted or unsubstituted O-aryl; or
R₃ is selected from CN, COOR₄, CONR₅R₆, while R₂ is selected from H, substituted or unsubstituted alkyl, substituted or unsubstituted O-alkyl or substituted or unsubstituted O-aryl;
preferably wherein
R₁ is selected from CN, COOR₄, CONR₅R₆; and
R₂ is selected from CN, COOR₄, CONR₅R₆, while R₃ is selected from H, substituted or unsubstituted C₁₋₄-alkyl, substituted or unsubstituted O-C₁₋₄-alkyl or substituted or unsubstituted O-aryl; or
R₃ is selected from CN, COOR₄, CONR₅R₆, while R₂ is selected from H, substituted or unsubstituted C₁₋₄-alkyl, substituted or unsubstituted O-C₁₋₄-alkyl or substituted or unsubstituted O-aryl;
more preferably wherein
R₁ is selected from CN, COOR₄, CONR₅R₆; and
R₂ is selected from CN, COOR₄, CONR₅R₆, while R₃ is selected from H, unsubstituted C₁₋₄-alkyl, unsubstituted O-C₁₋₄-alkyl or substituted or unsubstituted O-phenyl; or
R₃ is selected from CN, COOR₄, CONR₅R₆, while R₂ is selected from H, substituted or unsubstituted C₁₋₄-alkyl or substituted or unsubstituted O-C₁₋₄-alkyl or substituted or unsubstituted O-phenyl;
most preferably wherein
R₁ is selected from CN, COOR₄, CONR₅R₆; and
R₂ is selected from CN, COOR₄, CONR₅R₆, while R₃ is H; or
R₃ is selected from CN, COOR₄, CONR₅R₆, while R₂ is H.

In a further preferred embodiment of the invention of the lactone monomer according to Aspect A', of the process according to Aspect B', of the functionalized aliphatic polyester homopolymer according to Aspect C', of the functionalized aliphatic polyester random copolymer according to Aspect D', or of the functionalized aliphatic polyester copolymer of random and block microstructure according to Aspect D, wherein
R₄ is selected from substituted or unsubstituted C₁₋₁₀-alkyl, substituted or unsubstituted aryl, or substituted or unsubstituted C₁₋₄-alkyl-aryl;
R₅ and R₆ are independently of each other selected from substituted or unsubstituted C₁₋₁₀-alkyl, substituted or unsubstituted aryl, substituted or unsubstituted C₁₋₄-alkyl-aryl, substituted or unsubstituted mono- or bi-cyclic heterocyclyl, or substituted or unsubstituted mono- or bi-cyclic C₁₋₄-alkyl-heterocyclyl,
alternatively, R₅ and R₆ taken together with the nitrogen atom to which they are attached, may form a substituted or unsubstituted mono- or bi-cyclic heterocyclyl;
preferably wherein
R₄ is selected from substituted or unsubstituted C₁₋₄-alkyl, substituted or unsubstituted aryl, or substituted or unsubstituted C₁₋₄-alkyl-aryl;
R₅ and R₆ are independently of each other selected from substituted or unsubstituted C₁₋₄-alkyl, substituted or unsubstituted phenyl, substituted or unsubstituted benzyl or substituted or unsubstituted mono- or bi-cyclic heterocyclyl,
alternatively, R₅ and R₆ taken together with the nitrogen atom to which they are attached, may form a substituted or unsubstituted morpholine;
more preferably wherein
R₄ is selected from unsubstituted C₁₋₄-alkyl, substituted or unsubstituted phenyl or substituted or unsubstituted benzyl;
R₅ and R₆ are independently of each other selected from substituted or unsubstituted C₁₋₄-alkyl, substituted or unsubstituted phenyl or substituted or unsubstituted benzyl, alternatively, R₅ and R₆ taken together with the nitrogen atom to which they are attached, may form an unsubstituted morpholine;
and/or
wherein
R₁ is selected from CN, COOR₄, CONR₅R₆;
R₂ is selected from CN, COOR₄, CONR₅R₆, and
R₃ is selected from CN, COOR₄, CONR₅R₆.
and/or
wherein in a functionalized aliphatic polyester homopolymer according to Aspect C', in a functionalized aliphatic polyester random copolymer according to Aspect D, or in a functionalized aliphatic polyester copolymer of random and block microstructure according to Aspect E'
R₇ is selected from H, substituted or unsubstituted aliphatic or aromatic acyl, substituted or unsubstituted alkyl;
preferably wherein in a functionalized aliphatic polyester homopolymer according to claim 9 (option b)), in a functionalized aliphatic polyester random copolymer according to claim 10, or a functionalized aliphatic polyester copolymer of random and block microstructure according to claim 12 (option b))
R₇ is selected from H, acetyl or unsubstituted alkyl;
more preferably wherein in a functionalized aliphatic polyester homopolymer according to claim 9 (option b)), in a functionalized aliphatic polyester random copolymer according to claim 10, or in a functionalized aliphatic polyester copolymer of random and block microstructure according to claim 12 (option b))
R₇ is H.

In a further aspect of the invention (Aspect F) the invention is directed at a process of ROP for the production of a functionalized aliphatic polyester homopolymer according to Aspect C, a functionalized aliphatic polyester random copolymer according to Aspect D or a functionalized aliphatic polyester copolymer of random and block microstructure according to Aspect E, wherein the ROP is executed
in the presence of an basic, acidic, tin or aluminum catalyst; preferably in the presence of an aluminum alkyl and alkoxide catalyst; more preferably in the presence of an aluminum alkyl and aluminum alkoxide catalyst selected from diethylaluminum ethoxide, methylaluminum bis(2,6-di-tert-butyl-4-methylphenolate), or methylaluminum bis(2,6-di-phenylphenolate); most preferably in the presence of methylaluminum bis(2,6-di-phenylphenolate)
with/without
the presence of primary alcohol as initiator; preferably in the presence of ethanol as initiator.

### EXPERIMENTAL PART

***General.*** Unless otherwise specified, reagents were used as received from the vendor without further purification. Anhydrous solvents were bought from Sigma-Aldrich. 2-Trimethylsiloxy-1,3-butadiene was obtained by method, described in literature [1] or bought from Sigma-Aldrich. Solutions of methylaluminum bis(2,6-di-tert-butyl-4-methylphenolate) and methylaluminum bis(2,6-di-phenylphenolate) were obtained by addition of 2.0M toluene solution trimethylaluminum to the solution of 2 equivalents of the corresponding phenol in such amount of dry dichloromethane, so the concentration of the resulting complex was 0.2M. ¹H (299.9 MHz) and ¹³C NMR (75.4 MHz) spectra were recorded on an Agilent Technologies DD2 spectrometer in the pulse Fourier transform mode with both a relaxation delay and an acquisition time of 5 s. Chemical shifts are reported in parts-per-million (ppm) relative to TMS (δ = 0.00 ppm) for ¹H and to CHCl₃ solvent signal (δ = 77.0 ppm) for ¹³C NMR spectra. FT-IR spectra were obtained on a Perkin Elmer Spectrum One spectrometer in ATR mode in the range of 4000 - 650 cm⁻¹. HRMS measurements were obtained on a Waters Micromass Q-TOF Premier instrument equipped with an orthogonal Z-spray ESI interface.

As already explained above for the very preferred disubstituted caprolactone monomers with different type of substituents according to this invention an optimized synthetic pathway was found. It includes two steps: i) Diels-Alder reaction followed by *in-situ* TMS-enol cleavage, and in the next step ii) the Baeyer-Villiger oxidation. In the below example, the Diels-Alder solvent-free cycloaddition of 2-trimethylsililoxy-1,4-butadiene (enol ether of methyl vinyl ketone, which could be obtained from the biomass processing product - levulinic acid), and fumaronitrile (derivative of fumaric acid, which is also obtained from the lignocellulosic biomass), followed by acidic cleavage of the TMS-enol, results in the 3,4-dicyanosubstituted cyclohexanone formation in high yield. The ketone is then oxidized with (mCPBA) via the Baeyer-Villiger reaction.

One step assembling of the substituted ketone is possible via the Diels-Alder reaction if the proper substituents are present either in a diene, or in a dienophile, together with the masked ketone function. It was found that 2-trimethylsililoxy-1,4-butadiene **1** (Scheme 1) - enol ether of methyl vinyl ketone can serve as an appropriate diene for this purpose. Its precursor methyl vinyl ketone could be obtained from levulinic acid that is one of the mass products occurring during lignocellulosic biomass processing [9]. The further enol ether synthesis leads to the corresponding diene 1 [10]. Due to electron demands of the Diels-Alder reaction, the dienophile must contain electron-deficient substituents and it was found that this criterion is surprisingly well met by another group of biomass related chemicals - fumaric and maleic acid derivatives.

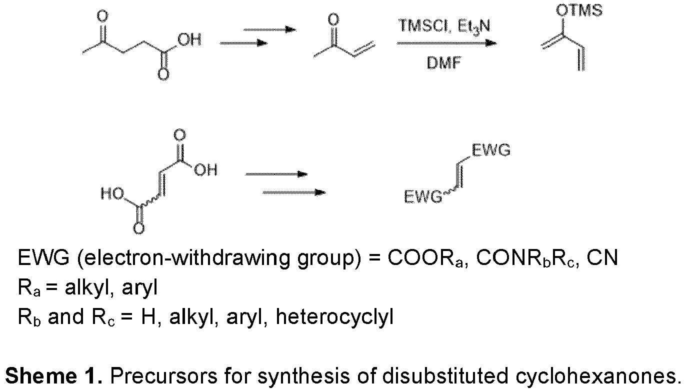

The synthesis was performed with a fumaronitrile, which could be easily obtained from the diethyl fumarate in two steps [11]. The dienophiles such as fumaro/maleoamides or esters could also be used. Diels-Alder reaction of the diene **1** (Scheme 2) with diethyl fumarate was previously reported [12]. This method was optimized for the reaction with fumaronitrile **2.** Heating of a mixture of diene **1** and dienophile **2** with a small addition of hydroquinone at 130 °C for 4 hours leads to the formation of cyclohexenone **3** (Scheme 2, R = CN). After the cycloaddition is completed, the mixture is diluted with acetonitrile and ethanol, and a small amount of formic acid is added. Further evaporation of reaction mixture and vacuum distillation affords the ketone **4** in high yield (80.5%) (Scheme 2). This method is also appropriate for other dienophiles (Scheme 2).

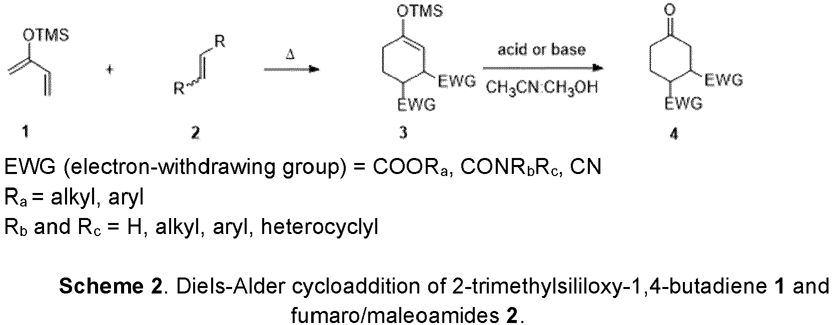

***Synthesis of di-3,4-cyano cyclohexanone (1).*** A mixture of 2-trimethylsiloxy-1,3-butadiene (9.18 g (90% pure), 64.6 mmol), fumaronitrile (5.04 g, 64.6 mmol) and hydroquinone (20 mg) was heated to 130 °C for 3 hours. The reaction mixture was dissolved in mixture of methanol (30 mL) and acetonitrile (15 mL). Then formic acid (100 µl) was added and the mixture was stirred overnight at room temperature. Solution was filtered and the solvent was evaporated under reduced pressure. Distillation on kuglerohr (3 Torr/200°C) afforded ketone (7.70 g, 80.5%) as pale yellow oil.

The substituted cyclohexanone **4** (with EWG being CN) was characterized by NMR and FTIR. In ¹H NMR spectrum there are two signals for the protons neighboring the two cyano groups, which are shifted to the lower magnetic field (3.25 - 3.5 ppm, Fig.1) compared to the nonsubstituted CH₂ fragments of the ring. ¹³C NMR spectrum also shows two distinctive signals for the carbons of the cyano group (117.3 and 117.0 ppm) as well as for the carbonyl carbon (201.2 ppm, Fig.2).

In the next step, the cyclohexanone **4** is oxidized by the Baeyer-Villiger method with mCPBA in dichloromethane (Scheme 3, R = CN). Lactone **5a** slowly crystallizes from the reaction mixture as a single regioisomer, so it can be filtered and washed with diethyl ether in order to remove admixture of m-chlorobenzoic acid. During this step, a small amount of the polymeric side product also forms. In order to remove it, the lactone is dissolved in acetonitrile and it is precipitated with diethyl ether. This procedure is repeated several times. Despite of poorer solubility of the side product, it doesn't crystallize and it stays in the form of milky dispersion while the lactone gets precipitated. After this purification procedure, the monomer of high purity is obtained. In NMR spectra the signals of the CH₂O fragment appeared (4.42 - 4.20 ppm in ¹H NMR and 64.6 ppm in ¹³C NMR, Figs. 4 and 5). The Scheme 2 was tested also for the synthesis of further caprolactones of the invention, being disubstituted with the ester and amide groups, and this resulted in the formation of a mixture of the two regioisomers with **5a** as the most abundant.

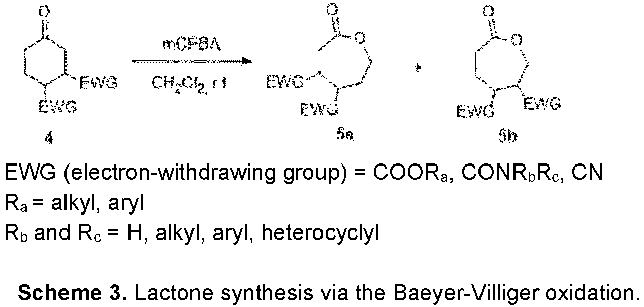

***Synthesis of di-3,4-cyano e-caprolactone (2).* 1** (11.8 g, 79.7 mmol) was dissolved in 350 ml of dichloromethane. Then 77% m-chloroperbenzoic acid (44.6 g, 199.0 mmol) was added and reaction was stirred for 10 days. Precipitate, which formed during reaction, was filtered and washed with 300 ml of ether, then washed down into flask with 300 ml of acetonitrile (byproduct stayed on the filter). Acetonitrile solution was evaporated under reduced pressure to ~80mL volume and the product was precipitated with 500 mL of ether. After 3 hours the milky solution was decanted, precipitate redissolved in 80 mL of acetonitrile and the procedure was repeated. After the vacuum-drying clean **2** (6.8 g, 52%) was obtained.

Residual water from the monomer 5 was removed as follows:
The substance was dissolved in minimal amount of dry acetonitrile (120mg/ml) at 65°C, then the solvent was removed under reduced pressure. The procedure was repeated three times.

The new monomer (with R = CN) has quite high melting point (Tₘ = 180°C) and it is only soluble in very polar solvents like acetone, acetonitrile, and less in dimethylformamide. Acetonitrile was chosen for the polymerization of the monomer **5a** (Scheme 4).

Different catalysts were tried for optimizing the polymerization. Dicyanocaprolactone was polymerized with methanesulfonic and trifluoromethanesulphonic acids as the catalysts. While working, considerable side reactions occurred. Efforts with another acidic catalyst, diphenylphosphate, did not show major improvement. The polymerization process was relatively slow. In reaction catalyzed by a classic Lewis acid stannous(II) 2-ethylhexanoate, a polymer formation was observed, but again side reactions occurred. Then basic catalysts were tested with another polymerization mechanism. The basic catalysts, i.e., DBU, potassium methoxide, and complexes of substituted thioureas with potassium methoxide or 1,3-dimesitylimidazol-2-ylidene, which are known to be very selective in experiments with ε-caprolactone and L-lactide [13] were tested. The monomer **5a** polymerizes vigorously almost in all these cases, but at the same time considerably side reactions were seen. Therefore, it seems that basic catalysts are also not an optimal selection. Then, transitional metal alkyl and alkoxide catalysts, which promote polymerization through a coordination-insertion mechanism, were tested. Using diethylaluminium ethoxide **7** (Scheme 5) in acetonitrile worked very well with the monomer **5a** (Scheme 5, R = CN). The reaction proceeds fast and without the formation of side products. Still, the molecular weight dispersity of the obtained polymer is relatively high. Polymerization with aluminum triisopropoxide improved the polymer molecular weight dispersity only slightly and the reproducibility of polymerization was not optimal. Aluminum catalysts with BHT (Catalyst **8)** and 2,4-diphenylphenol ligands (Catalyst **9)** [14] were used (Scheme 5). Both catalysts improved the control over ROP of the monomer **5**. Catalyst **9** shows the narrowest molecular weight distribution of the product (M_{w}/Mn = ~1.1 as determined by SEC-MALS). In ¹H NMR spectrum of the polymer there are signals of the C*H*₂O (4.40-4.12 ppm) and CNCHCHCN (3.75 - 3.65 and 3.52 - 3.40 ppm) fragments that are slightly shifted to the lower magnetic field in comparison to the corresponding signals of the cyclic monomer, which is ascribed to the relief of a ring strain due to the formation of a linear structure. In ¹³C NMR spectrum these signals can be found at 61.8 ppm and 118.7 ppm, respectively. The polymer demonstrates quite high glass transition temperature (T_{g} = ~70°C for Mₙ = ~20 kDa) and thermal stability up to 220 °C (Figs. 11 and 12).

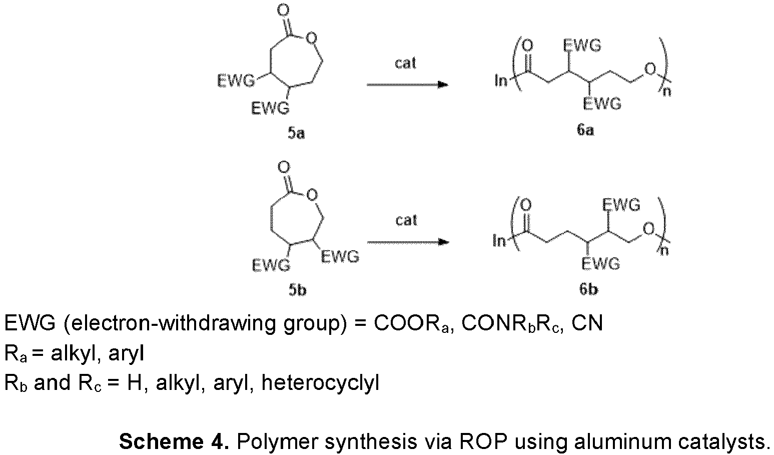

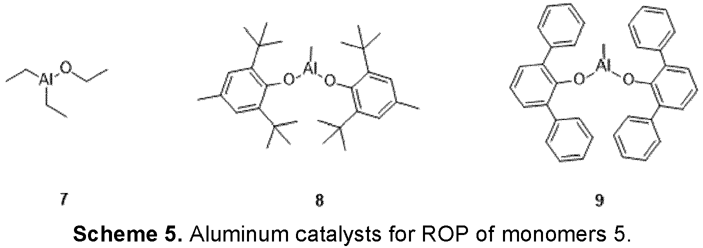

***General polymerization procedure.*** All procedures were done in a N₂-filled glove box.

Procedure 1. Dry acetonitrile was added to monomer **5** (1 ml per 120 mg) at 25°C, followed by the solution of diethylaluminum ethoxide (25% wt in toluene). The reaction mixture was stirred at 25°C, conversion was monitired with NMR and then quenched with 0.1M solution of acetic acid in acetonitrile. The solvent was evaporated under reduced pressure, then the polymer was redissolved in small amount of acetone and precipitated in hexane. The precipitate was centrifuged and then dried under reduced pressure at 130°C.

Procedure 2. Dry acetonitrile was added to monomer **5** (1 ml per 120 mg) at 25 °C. Then ethanol was added, followed by catalyst solution (bis(2,6-di-tert-butyl-4-methylphenolate) or methylaluminum bis(2,6-di-phenylphenolate)) in dichloromethane. Further steps are identical to previous procedure.

*Copolymerization procedure.*

***Block copolymer.*** ε-Caprolactone (1 ml per 85 mg) was dissolved in dry acetonitrile at 25°C, ehtanol was added, followed by the solution of methylaluminum bis(2,6-di-phenylphenolate) in dichloromethane. After conversion reached >97%, dry monomer **5** was added in equimolar amount to ε-caprolactone and reaction was stirred at ambient temperature until concersion of **5** was >95% (according to NMR spectrum), then quenched with 0.1M solution of acetic acid in acetonitrile. Reaction mixture was precipitated in cold methanol, the precipitate was centrifuged and then dried under reduced pressure at 130 °C.

***Random-copolymer.*** A mixture of dry acetonitrile and dichloromethane (2:1) was added to equimolar amounts of ε-caprolactone and monomer **5** (140 mg of combined monomers per 1 mL). Then ethanol was added, followed by the solution of methylaluminum bis(2,6-di-phenylphenolate) in dichloromethane. After the conversion of both monomers reached >94%, reaction mixture was quenched with 0.1M and isolated in the same way as block-copolymer.

### Bibliography

[1] http://docs.european-bioplastics.org/publications/EUBP_Facts_and_figures.pdf
[2]
   a. Wu, M. M. (2001). Acrylonitrile Polymers, Survey and Styrene-Acrylonitrile (SAN). In Kirk-Othmer Encyclopedia of Chemical Technology, (Ed.).
   b. J.M. Kelley, W.F. Hanzl, M.R. Stepanian (1968), Styrene-acrylonitrile copolymers and process for their preparation, Patent US3560418A, Dart Industries Inc.
[3] A. A. Ogale, M. Zhang, J. Jin, Recent advances in carbon fibers derived from biobased precursors, J. Appl. Polym. Sci., 2016, 133, 43794.
[4]
   a. J.R. Briggs, A.S. Guram, J.M. Maher, E.B. Tjaden (1997), Process for producing epsilon caprolactones and/or hydrates and/or esters thereof, Patent EP0872482A3, Union Carbide Chemicals and Plastics Technology LLC.
   b. H.C. Quilter, M. Hutchby, M.G. Davidson, M.D. Jones, Polymerisation of a terpene-derived lactone: a bio-based alternative to ε-caprolactone, *Polym. Chem.,* **2017,** 8, 833-837
[5]
   a. S. Lenoir, R. Riva, X. Lou, Ch. Detrembleur, R. Jérôme, Ph. Lecomte, Ring-Opening Polymerization of α-Chloro-ε-caprolactone and Chemical Modification of Poly(α-chloro-ε-caprolactone) by Atom Transfer Radical Processes, Macromolecules, 2004, 37, 4055-4061
   b. S. Gautier, V. D'Aloia, O. Halleux, M. Mazza, P. Lecomte, R. Jérôme, Amphiphilic copolymers of ε-caprolactone and γ-substituted ε-caprolactone. Synthesis and functionalization of poly(D,L-lactide) nanoparticles, J. Biomater. Sci. Polym. Ed, 2003, 14, 63-85
[6] A. Lavasanifar, A. Mahmud **(2006),** Functionalized caprolactone monomers useful for making poly(ethylene oxide)-block-poly(ester) block copolymers, EP2730604A3, University of Alberta.
[7] R.P. Babu, K. O'Connor, R. Seeram, Current progress on bio-based polymers and their future trends, *Prog. Biomater.,* **2013,** 2:8
[8] J.A. Dumesic, R.M. West **(2010),** Production of methyl-vinyl ketone from levulinic acid, Patent US7960592B1, Wisconsin Alumni Research Foundation
[9] R.T. Uyeda , P. Vu, D.D. Holsworth, Improved large scale synthesis of 2-trimethylsilyloxi-1,3-butadiene, Org. Prep. Proced. Int., 2002, 34, 540-543
[10] Mowry, D. T., Butler, J. M., Price, C. C. and Gilbert, R. D. (2003). Fumaronitrile. In Organic Syntheses
[11] M.E. Jung, C.A. McCombs, Y. Takeda, Y.-G. Pan, Use of silyloxydienes in synthesis. Total syntheses of the sesquiterpene (±)-seychellene, J. Am. Chem. Soc., 1981, 103, 6677-6685
[12] H. Wang, W. Wu, Z. Li, X. Zhi, C. Chen, C. Zhao, X. Li, Q. Zhang, K. Guo, 2,4-Dinitrobenzenesulfonic acid in an efficient Bronsted acid-catalyzed controlled/living ring-opening polymerization of ε-caprolactone, RSC Adv., 2014, 55716-55722
[13] M. Akatsuka, T. Aida, S. Inoue, Alcohol/methylaluminum diphenolate systems as novel, versatile initiators for synthesis of narrow molecular weight distribution polyester and polycarbonate, Macromolecules, 1995, 28, 1320-1322.
[14]
   a. M. Renz, B. Meunier, 100 Years of Baeyer-Villiger oxidations, Eur. J. Org. Chem., 1999, 737
   b. M. Uyanik, K. Ishihara, Baeyer-Villiger oxidation using hydrogen peroxide, ACS Catal., 2013, 3, 513

## Claims

1. A lactone monomer having a structure of general formula (I) 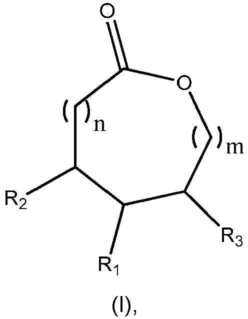 wherein
n is selected from 0, 1 or 2;
m is selected from 0, 1 or 2; and
m + n is 2;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl,
wherein alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c',} or a linear or branched, substituted or unsubstituted -OC₁₋₆ alkyl, with R_{c} and R_{c'} being represented by H or by a linear or branched, substituted or unsubstituted C₁₋₆-alkyl; and
wherein aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂, -NR_{c}R_{c'}, -C(O)OR_{c}, NR_{c}C(O)R_{c'} , -C(O)NR_{c}R_{c'} , -NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

2. A lactone monomer of claim 1, wherein
one of R₂ or R₃ is H
with the condition that
a) if R₂ is H, m is not 0; or
b) if R₃ is H, n is not 0.

3. A lactone monomer of claim 1, having a structure of general formula (Ia) 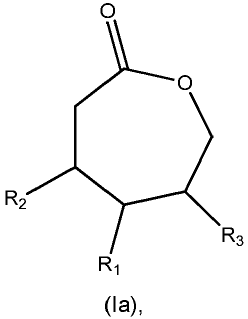 wherein
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl or substituted, unsubstituted O-alkyl, or substituted or unsubstituted O-aryl;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl.

4. A lactone monomer of claim 1 or 3, having a structure of general formula (II) or (III) 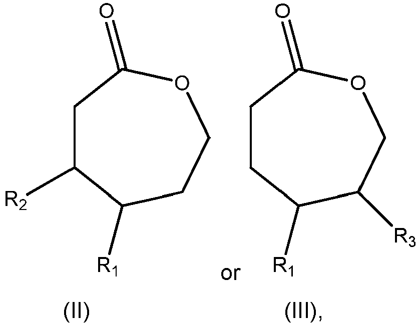 wherein
R₁ is selected from CN, COOR₄, CONR₅R₆, halogen or NO₂;
R₂ is selected from CN, COOR₄, CONR₅R₆, halogen or NO₂;
R₃ is selected from CN, COOR₄, CONR₅R₆, halogen or NO₂;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl alternatively, R₅ and R₆ taken together with the nitrogen atom to which they are attached, may form a substituted or unsubstituted heterocyclyl.

5. A process for producing a lactone monomer of claims 1, 2 or 3, comprising the final step of a Baeyer-Villiger oxidation of a ketone to the lactone monomer of Formula (I) following the below reaction-scheme: 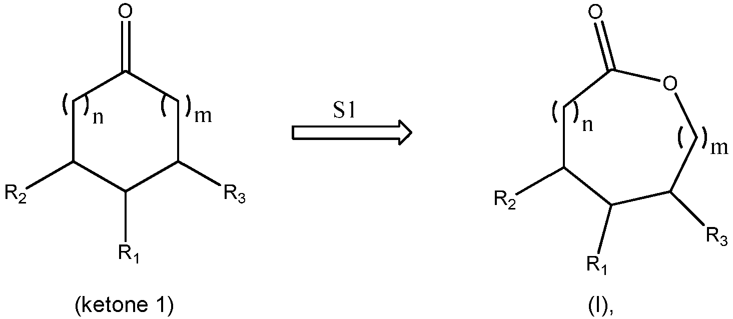 wherein
in the reaction step S1
- an oxidant is added, preferably an oxidant selected from m-chloroperbenzoic acid (mCPBA), hydrogen peroxide, sodium perborate, peracetic acid, trifluoroperacetic acid, performic acid, 4-nitroperbenzoic acid is added, more preferably mCPBA is added;
- the solvent is an organic solvent, preferably is CH₂Cl₂, and
- the temperature is selected between -5° C and 90° C, preferably is selected between 15° C and 30° C, more preferably is room temperature;
and wherein in the ketone 1 and in the compound of formula (I)
n is selected from 0, 1 or 2;
m is selected from 0, 1 or 2; and
m + n is 2;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl,
wherein alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c',} or a linear or branched, substituted or unsubstituted -OC₁₋₆ alkyl, with R_{c} and R_{c'} being represented by H or by a linear or branched, substituted or unsubstituted C₁₋₆-alkyl; and
wherein aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂, -NR_{c}R_{c'}, -C(O)OR_{c}, NR_{c}C(O)R_{c'} , -C(O)NR_{c}R_{c'} , -NR_{c}S(O)₂R_{c'} , =O, -OCH₂CH₂OR_{c}, -NR_{c}C(O)NR_{c'}H_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl;
preferably
wherein one of R₂ or R₃ is H,
with the condition that if R₂ is H, m is not 0; or if R₃ is H, n is not 0;
and/or
wherein n and m are 1.

6. A process for producing a lactone monomer of claim 4, comprising the final step of a Baeyer-Villiger oxidation of a ketone to the lactone monomer of Formula (II) and (III) following the below reaction-scheme: 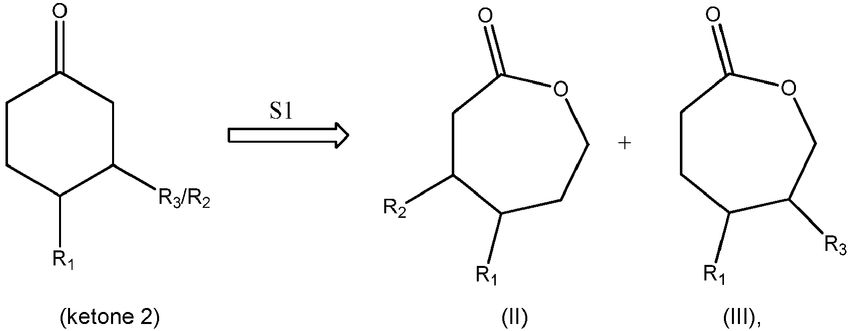 wherein in the reaction step S1
- an oxidant is added, preferably an oxidant selected from mCPBA, hydrogen peroxide, sodium perborate, peracetic acid, trifluoroperacetic acid, performic acid, 4-nitroperbenzoic acid is added, more preferably mCPBA is added,
- the solvent is an organic solvent, preferably is CH₂Cl₂, and
- the temperature is selected between -5° C and 90° C, preferably is selected between 15° C and 30° C, more preferably is room temperature;
and wherein in the ketone 2 and in the compounds of formulas (II) and (III)
R₁ is selected from CN, COOR₄, CONR₅R₆, halogen or NO₂;
R₂ is selected from CN, COOR₄, CONR₅R₆, halogen or NO₂;
R₃ is selected from CN, COOR₄, CONR₅R₆, halogen or NO₂;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl
alternatively, R₅ and R₆ taken together with the nitrogen atom to which they are attached, may form a substituted or unsubstituted heterocyclyl,
wherein alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c',} or a linear or branched, substituted or unsubstituted -OC₁₋₆ alkyl, with R_{c} and R_{c'} being represented by H or by a linear or branched, substituted or unsubstituted C₁₋₆-alkyl; and
wherein aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂, -NR_{c}R_{c'}, -C(O)OR_{c}, NR_{c}C(O)R_{c'} , -C(O)NR_{c}R_{c'} , -NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

7. A process according to claim 6, in which the ketone 2 is provided by a sequence of a Diels-Alder reaction (S2) followed by TMS-enol cleavage, following the below reaction-scheme: 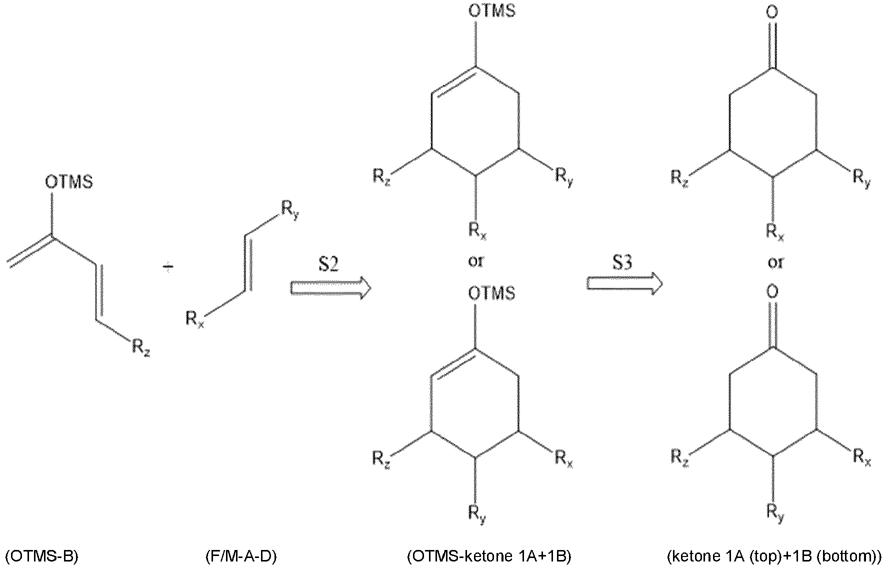 wherein (OTMS-B) is 3 or 2-trimethylsililoxy-substituted diene, preferably is 2-trimethylsililoxy-1,4-butadiene, (F/M-A-D) is a fumaric/maleic acid derivative (F/M-A-D), preferably wherein the 2-Trimethylsililoxy-1,4-butadiene (OTMS-B) and/or the fumaric/maleic acid derivative (F/M-A-D) are synthesized from chemicals, derived from oil- or biomass;
wherein
Rₓ is selected from CN, COOR₄, CONR₅R₆, halogen or NO₂;
R_{y} is selected from CN, COOR₄, CONR₅R₆, halogen or NO₂;
R_{z} is selected from H, substituted or unsubstituted alkyl or substituted, unsubstituted O-alkyl, or substituted or unsubstituted O-aryl
wherein in the reaction step S2
- the temperature is selected between 25° C and 180° C, preferably is 120°C to 140°C, most preferably is 130°C;
- the reaction time is selected from 1 hour to 24 hours, preferably from 3 hours to 5 hours, most preferably is 4 hours;
wherein in the reaction step S3
- acetonitrile, ethanol or methanol, formic acid or sodium carbonate is added,
- the reaction mixture is filtered and evaporated
- and optionally the final product is additionally purified by a vacuum distillation or by chromatography; and
wherein finally, when R_{z} is H, the ketone 2 of claim 6 is provided from ketone 1A in which Rₓ is R₁ and R_{y} is R₃/R₂.

8. A functionalized aliphatic polyester homopolymer of general formula (IV) 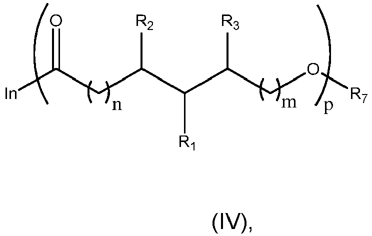 wherein
n is selected from 0, 1 or 2;
m is selected from 0, 1 or 2; and
m + n is 2;
p is selected from 5-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
with the condition that
if n is 0, R₂ is H, and if m is 0, R₃ is H;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl;
preferably wherein one of R₂ or R₃ is H,
with the condition that if R₂ is H, m is not 0; or if R₃ is H, n is not 0; and
In is substituted or unsubstituted aliphatic alkoxide or a primary or secondary substituted or unsubstituted aliphatic amino group, preferably is ethoxide,
wherein alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c',} or a linear or branched, substituted or unsubstituted -OC₁₋₆ alkyl, with R_{c} and R_{c'} being represented by H or by a linear or branched, substituted or unsubstituted C₁₋₆-alkyl; and
wherein aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , -NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

9. A functionalized aliphatic polyester homopolymer according to claim 8
a) of general formula (IVa) 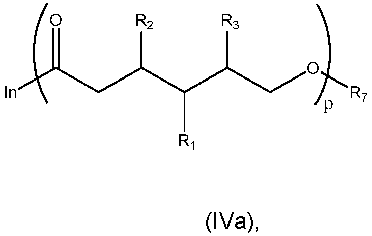 wherein
p is selected from 5-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl,
wherein aliphatic, alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c',} or a linear or branched, substituted or unsubstituted -OC₁₋₆ alkyl, with R_{c} and R_{c'} being represented by H or by a linear or branched, substituted or unsubstituted C₁₋₆-alkyl; and
wherein aromatic, aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , - NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}H_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl; or
b) of general formula (V) or (VI) 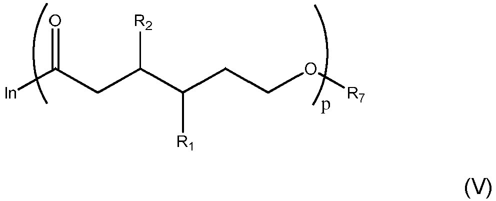 or 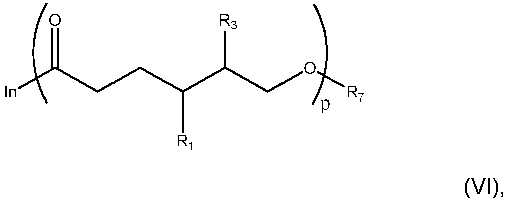 wherein
p is selected from 5-500;
R₁ is selected from CN, COOR₄, CONR₅R₆, halogen or NO₂;
R₂ is selected from CN, COOR₄, CONR₅R₆, halogen or NO₂;
R₃ is selected from CN, COOR₄, CONR₅R₆, halogen or NO₂;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl,
wherein aliphatic, alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R_{c}, -OR_{c}, -C(O)OR_{c}, -CN, -C(O)NR_{c}R_{c',} or a linear or branched, substituted or unsubstituted -OC₁₋₆ alkyl, with R_{c} and R_{c'} being represented by H or by a linear or branched, substituted or unsubstituted C₁₋₆-alkyl; and
wherein aromatic, aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , - NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

10. A functionalized aliphatic polyester random copolymer of general formula (VII) 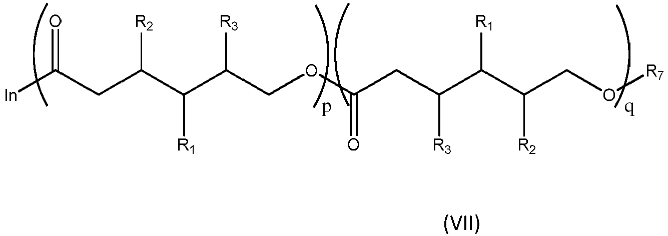
p is selected from 5-500,
q is selected from 5-500,
p+q is 10-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl,
wherein aliphatic, alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c}, -SR_{c}, -S(O)R_{c}, -S(O)₂R,, -OR_{c}, -C(O)OR_{c}, -CN, -C(O)NR_{c}R_{c'}, or a linear or branched, substituted or unsubstituted -OC₁₋₆ alkyl, with R_{c} and R_{c'} being represented by H or by a linear or branched, substituted or unsubstituted C₁₋₆-alkyl; and
wherein aromatic, aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , - NR_{c}S(O)₂R_{c'} , =O, -OCH₂CH₂OR_{c}, -NR_{c}C(O)NR_{c}R_{c"}, -S(O)₂NR_{c}R_{c'}, -NR_{c}S(O)₂NR_{c}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

11. A functionalized aliphatic polyester copolymer of random and block microstructure of general formula (VIII) or (IX) 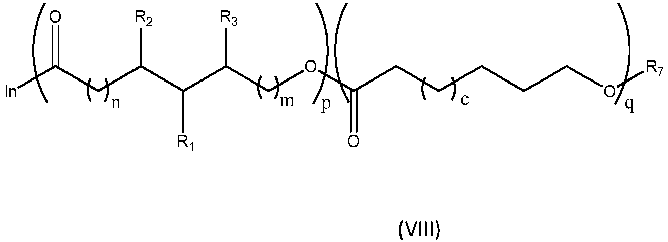 or 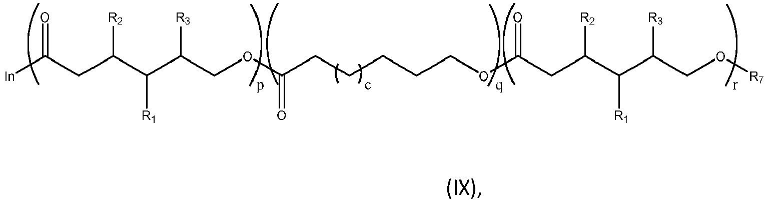 wherein
n is selected from 0, 1 or 2,
m is selected from 0, 1 or 2, and
m + n is 2,
c is selected from 0 or 1;
p is selected from 5-500,
q is selected from 5-500, and
r is selected from 5-500,
p+q+r is 15-500;
R₁ is selected from CN, COOR₄, CONR₅R₆, halogen or NO₂;
one of R₂ and R₃ is selected from CN, COOR₄, CONR₅R₆, halogen or NO₂;, while the other one is selected from H, substituted or unsubstituted alkyl, substituted or unsubstituted O-alkyl, or substituted or unsubstituted O-aryl;
with the condition that
if n is 0, R₂ is H, and if m is 0, R₃ is H;
R₄ is selected from substituted or unsubstituted alkyl, substituted or unsubstituted alkenyl, substituted or unsubstituted alkyn, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
preferably wherein one of R₂ or R₃ is H,
with the condition that if R₂ is H, m is not 0; or if R₃ is H, n is not 0;
R₇ is selected from H, substituted or unsubstituted aliphatic or aromatic acyl, substituted or unsubstituted alkyl,
wherein aliphatic, alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c}, -SR_{c}, -S(O)R_{c}, -S(O)₂R,, -OR_{c}, -C(O)OR_{c}, -CN, -C(O)NR_{c}R_{c'}, or a linear or branched, substituted or unsubstituted -OC₁₋₆ alkyl, with R_{c} and R_{c'} being represented by H or by a linear or branched, substituted or unsubstituted C₁₋₆-alkyl; and
wherein aromatic, aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , - NR_{c}S(O)₂R_{c'} , =O, -OCH₂CH₂OR_{c}, -NR_{c}C(O)NR_{c}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

12. A functionalized aliphatic polyester copolymer of random and block microstructure according to claim 11
a) of general formula (VIIIa) or (IXa) 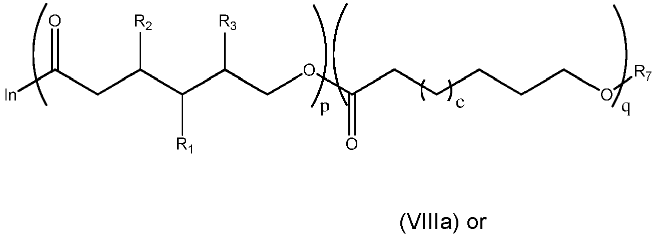 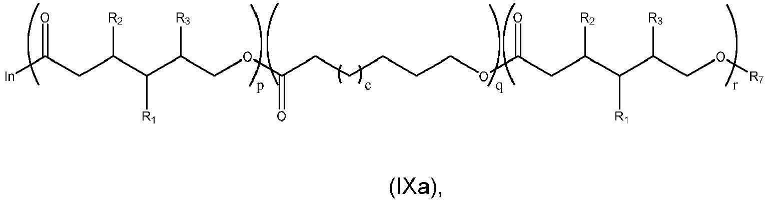 wherein
c is selected from 0 or 1,
p is selected from 5-500,
q is selected from 5-500, and
r is selected from 5-500,
p+q+r is 15-500;
R₁ is selected from CN, COOR₄, CONR₅R₆, halogen or NO₂;
one of R₂ and R₃ is selected from CN, COOR₄, CONR₅R₆, halogen or NO₂, while the other one is selected from H, substituted or unsubstituted alkyl, substituted or unsubstituted O-alkyl, or substituted or unsubstituted O-aryl;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl,
wherein aliphatic, alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R,, -OR_{c}, -C(O)OR_{c}, -CN, -C(O)NR_{c}R_{c'}, or a linear or branched, substituted or unsubstituted -OC₁₋₆ alkyl, with R_{c} and R_{c'} being represented by H or by a linear or branched, substituted or unsubstituted C₁₋₆-alkyl; and
wherein aromatic, aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c'}, -C(O)OR_{c}, NR_{c}C(O)R_{c'} , -C(O)NR_{c}R_{c'} , - NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl or
b) of general formula (X) or (XI) and (XII) or (XIII) 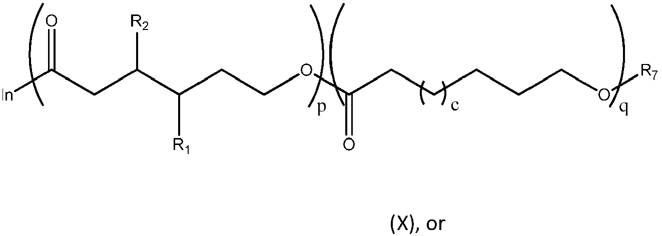 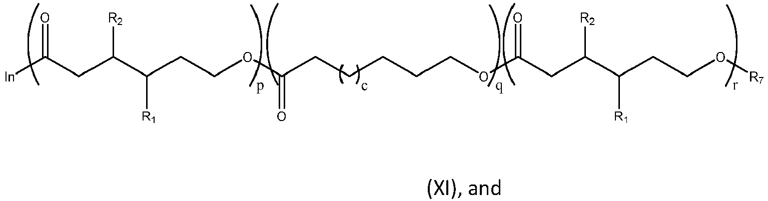 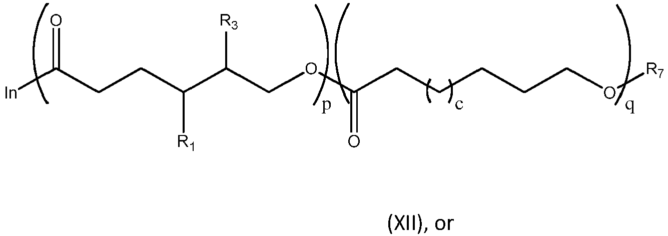 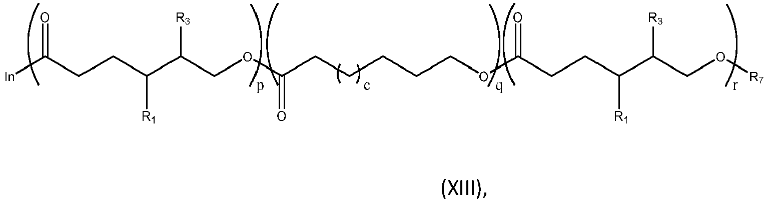 wherein
c is selected from 0 or 1;
p is selected from 5-500,
q is selected from 5.500, and
r is selected from 5-500,
p+q+r is 15-500,
R₁ is selected from CN, COOR₄, CONR₅R₆, halogen or NO₂;
R₂ is selected from CN, COOR₄, CONR₅R₆, halogen or NO₂;
R₃ is selected from CN, COOR₄, CONR₅R₆, halogen or NO₂;
R₄ is selected from substituted or unsubstituted alkyl, substituted or unsubstituted alkenyl, substituted or unsubstituted alkynyl, substituted or unsubstituted aryl, or substituted or unsubstituted alkyl-aryl;
R₅ and R₆ are independently of each other selected from substituted or unsubstituted alkyl, substituted or unsubstituted alkenyl, substituted or unsubstituted alkynyl, substituted or unsubstituted aryl, substituted or unsubstituted alkyl-aryl, substituted or unsubstituted heterocyclyl, or substituted or unsubstituted alkyl-heterocyclyl, alternatively, R₅ and R₆ taken together with the nitrogen atom to which they are attached, may form a substituted or unsubstituted heterocyclyl;
R₇ is selected from H, substituted or unsubstituted aliphatic or aromatic acyl, substituted or unsubstituted alkyl,
wherein aliphatic, alkyl, alkenyl, alkynyl and O-alkyl if substituted, as well as alkylaryl, alkylheterocycly, alkylcycloalkyl if substituted on the alkyl, is substituted by halogen (F, Cl, Br, I), -NR_{c}R_{c'}, -SR_{c}, -S(O)R_{c}, -S(O)₂R,, -OR_{c}, -C(O)OR_{c}, -CN, -C(O)NR_{c}R_{c'}, or a linear or branched, substituted or unsubstituted -OC₁₋₆ alkyl, with R_{c} and R_{c'} being represented by H or by a linear or branched, substituted or unsubstituted C₁₋₆-alkyl; and
wherein aromatic, aryl, cycloalkyl or heterocyclyl if substituted as well as alkyl-aryl, alkyl-cycloalkyl, or alkyl-heterocyclyl, if substituted on the ring-system, substituted by halogen (F, Cl, Br, I), -R_{c} ,-OR_{c}, -CN, -NO₂ , -NR_{c}R_{c}, -C(O)OR_{c}, NR_{c}C(O)R_{c'} , -C(O)NR_{c}R_{c'} , - NR_{c}S(O)₂R_{c'} , =O, -OCH₂CH₂OR_{c}, -NR_{c}C(O)NR_{c'}R_{c"}, -S(O)₂NR_{c}R_{c'}, -NR_{c}S(O)₂NR_{c'}R_{c"}, haloalkyl, haloalkoxy, -SR_{c}, -S(O)R_{c}, -S(O)₂R_{c}, -C(CH₃)OR_{c}; NR_{c}R_{c'}, or linear or branched, substituted or unsubstituted -OC₁₋₆ alkyl, with R_{c}, R_{c'} and R_{c"} being represented by H or by a linear or branched, substituted or unsubstituted C₁₋₆-alkyl.

13. A lactone monomer according to any one of claims 1 to 3, a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a)), wherein
R₄ is selected from substituted or unsubstituted C₁₋₁₀-alkyl, substituted or unsubstituted aryl, or substituted or unsubstituted C₁₋₄-alkyl-aryl;
R₅ and R₆ are independently of each other selected from substituted or unsubstituted C₁₋₁₀-alkyl, substituted or unsubstituted aryl, substituted or unsubstituted C₁₋₄-alkyl-aryl, substituted or unsubstituted mono- or bi-cyclic heterocyclyl, or substituted or unsubstituted mono- or bi-cyclic C₁₋₄-alkyl-heterocyclyl,
alternatively, R₅ and R₆ taken together with the nitrogen atom to which they are attached, may form a substituted or unsubstituted mono- or bi-cyclic heterocyclyl;
preferably wherein
R₄ is selected from substituted or unsubstituted C₁₋₄-alkyl, substituted or unsubstituted aryl, or substituted or unsubstituted C₁₋₄-alkyl-aryl;
R₅ and R₆ are independently of each other selected from substituted or unsubstituted C₁₋₄-alkyl, substituted or unsubstituted phenyl, substituted or unsubstituted benzyl or substituted or unsubstituted mono- or bi-cyclic heterocyclyl,
alternatively, R₅ and R₆ taken together with the nitrogen atom to which they are attached, may form a substituted or unsubstituted morpholine;
more preferably wherein
R₄ is selected from unsubstituted C₁₋₄-alkyl, substituted or unsubstituted phenyl or substituted or unsubstituted benzyl;
R₅ and R₆ are independently of each other selected from substituted or unsubstituted C₁₋₄-alkyl, substituted or unsubstituted phenyl or substituted or unsubstituted benzyl, alternatively, R₅ and R₆ taken together with the nitrogen atom to which they are attached, may form an unsubstituted morpholine;
and/or
wherein in a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a))
R₇ is selected from H, substituted or unsubstituted aliphatic or aromatic acyl, substituted or unsubstituted alkyl;
preferably wherein in a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a))
R₇ is selected from H, acetyl or unsubstituted alkyl;
more preferably wherein in a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a))
R₇ is H.

14. A lactone monomer according to any one of claims 1 to 3 and 13, a functionalized aliphatic polyester homopolymer according to any one of claims 8, 9 (option a)) and 13, a functionalized aliphatic polyester random copolymer according to any one of claims 10 or 13, or a functionalized aliphatic polyester copolymer of random and block microstructure according to any one of claims 11, 12 (option a)) and 13, wherein
R₁ is selected from CN, COOR₄, CONR₅R₆; and
R₂ is selected from CN, COOR₄, CONR₅R₆, while R₃ is selected from H, substituted or unsubstituted alkyl; substituted or unsubstituted O-alkyl or substituted or unsubstituted O-aryl; or
R₃ is selected from CN, COOR₄, CONR₅R₆, while R₂ is selected from H, substituted or unsubstituted alkyl, substituted or unsubstituted O-alkyl or substituted or unsubstituted O-aryl;
preferably wherein
R₁ is selected from CN, COOR₄, CONR₅R₆; and
R₂ is selected from CN, COOR₄, CONR₅R₆, while R₃ is selected from H, substituted or unsubstituted C₁₋₄-alkyl, substituted or unsubstituted O-C₁₋₄-alkyl or substituted or unsubstituted O-aryl; or
R₃ is selected from CN, COOR₄, CONR₅R₆, while R₂ is selected from H, substituted or unsubstituted C₁₋₄-alkyl, substituted or unsubstituted O-C₁₋₄-alkyl or substituted or unsubstituted O-aryl;
more preferably wherein
R₁ is selected from CN, COOR₄, CONR₅R₆; and
R₂ is selected from CN, COOR₄, CONR₅R₆, while R₃ is selected from H, unsubstituted C₁₋₄-alkyl, unsubstituted O-C₁₋₄-alkyl or substituted or unsubstituted O-phenyl; or
R₃ is selected from CN, COOR₄, CONR₅R₆, while R₂ is selected from H, substituted or unsubstituted C₁₋₄-alkyl or substituted or unsubstituted O-C₁₋₄-alkyl or substituted or unsubstituted O-phenyl;
most preferably wherein
R₁ is selected from CN, COOR₄, CONR₅R₆; and
R₂ is selected from CN, COOR₄, CONR₅R₆, while R₃ is H; or
R₃ is selected from CN, COOR₄, CONR₅R₆, while R₂ is H.

15. A lactone monomer of claim 4, a functionalized aliphatic polyester homopolymer according to claim 9 (option b)), a functionalized aliphatic polyester random copolymer of claim 10 or a functionalized aliphatic polyester copolymer of random and block microstructure according to claim 12 (option b)), wherein
R₄ is selected from substituted or unsubstituted C₁₋₁₀-alkyl, substituted or unsubstituted aryl, or substituted or unsubstituted C₁₋₄-alkyl-aryl;
R₅ and R₆ are independently of each other selected from substituted or unsubstituted C₁₋₁₀-alkyl, substituted or unsubstituted aryl, substituted or unsubstituted C₁₋₄-alkyl-aryl, substituted or unsubstituted mono- or bi-cyclic heterocyclyl, or substituted or unsubstituted mono- or bi-cyclic C₁₋₄-alkyl-heterocyclyl,
alternatively, R₅ and R₆ taken together with the nitrogen atom to which they are attached, may form a substituted or unsubstituted mono- or bi-cyclic heterocyclyl;
preferably wherein
R₄ is selected from substituted or unsubstituted C₁₋₄-alkyl, substituted or unsubstituted aryl, or substituted or unsubstituted C₁₋₄-alkyl-aryl;
R₅ and R₆ are independently of each other selected from substituted or unsubstituted C₁₋₄-alkyl, substituted or unsubstituted phenyl, substituted or unsubstituted benzyl or substituted or unsubstituted mono- or bi-cyclic heterocyclyl,
alternatively, R₅ and R₆ taken together with the nitrogen atom to which they are attached, may form a substituted or unsubstituted morpholine;
more preferably wherein
R₄ is selected from unsubstituted C₁₋₄-alkyl, substituted or unsubstituted phenyl or substituted or unsubstituted benzyl;
R₅ and R₆ are independently of each other selected from substituted or unsubstituted C₁₋₄-alkyl, substituted or unsubstituted phenyl or substituted or unsubstituted benzyl, alternatively, R₅ and R₆ taken together with the nitrogen atom to which they are attached, may form an unsubstituted morpholine;
and/or
wherein
R₁ is selected from CN, COOR₄, CONR₅R₆;
R₂ is selected from CN, COOR₄, CONR₅R₆, and
R₃ is selected from CN, COOR₄, CONR₅R₆.
and/or
wherein in a functionalized aliphatic polyester homopolymer according to claim 9 (option b)), in a functionalized aliphatic polyester random copolymer according to claim 10, or in a functionalized aliphatic polyester copolymer of random and block microstructure according to claim 12 (option b))
R₇ is selected from H, substituted or unsubstituted aliphatic or aromatic acyl, substituted or unsubstituted alkyl;
preferably wherein in a functionalized aliphatic polyester homopolymer according to claim 9 (option b)), in a functionalized aliphatic polyester random copolymer according to claim 10, or a functionalized aliphatic polyester copolymer of random and block microstructure according to claim 12 (option b))
R₇ is selected from H, acetyl or unsubstituted alkyl;
more preferably wherein in a functionalized aliphatic polyester homopolymer according to claim 9 (option b)), in a functionalized aliphatic polyester random copolymer according to claim 10, or in a functionalized aliphatic polyester copolymer of random and block microstructure according to claim 12 (option b))
R₇ is H.

16. A process of ROP for the production of a functionalized aliphatic polyester homopolymer according to any of claims 8 or 9, an aliphatic polyester random copolymer of claim 10 or a functionalized aliphatic polyester copolymer of random and block microstructure according to claims 11 or 12, wherein the ROP is executed
in the presence of an basic, acidic, tin or aluminum catalyst; preferably in the presence of an aluminum alkyl and alkoxide catalyst; more preferably in the presence of an aluminum alkyl and aluminum alkoxide catalyst selected from diethylaluminum ethoxide, methylaluminum bis(2,6-di-tert-butyl-4-methylphenolate), or methylaluminum bis(2,6-di-phenylphenolate); most preferably in the presence of methylaluminum bis(2,6-diphenylphenolate),
preferably
in the presence of primary alcohol as initiator; more preferably in the presence of ethanol as initiator.

17. A Iprocess according to claim 5, wherein
R₄ is selected from substituted or unsubstituted C₁₋₁₀-alkyl, substituted or unsubstituted aryl, or substituted or unsubstituted C₁₋₄-alkyl-aryl;
R₅ and R₆ are independently of each other selected from substituted or unsubstituted C₁₋₁₀-alkyl, substituted or unsubstituted aryl, substituted or unsubstituted C₁₋₄-alkyl-aryl, substituted or unsubstituted mono- or bi-cyclic heterocyclyl, or substituted or unsubstituted mono- or bi-cyclic C₁₋₄-alkyl-heterocyclyl,
alternatively, R₅ and R₆ taken together with the nitrogen atom to which they are attached, may form a substituted or unsubstituted mono- or bi-cyclic heterocyclyl;
preferably wherein
R₄ is selected from substituted or unsubstituted C₁₋₄-alkyl, substituted or unsubstituted aryl, or substituted or unsubstituted C₁₋₄-alkyl-aryl;
R₅ and R₆ are independently of each other selected from substituted or unsubstituted C₁₋₄-alkyl, substituted or unsubstituted phenyl, substituted or unsubstituted benzyl or substituted or unsubstituted mono- or bi-cyclic heterocyclyl,
alternatively, R₅ and R₆ taken together with the nitrogen atom to which they are attached, may form a substituted or unsubstituted morpholine;
more preferably wherein
R₄ is selected from unsubstituted C₁₋₄-alkyl, substituted or unsubstituted phenyl or substituted or unsubstituted benzyl;
R₅ and R₆ are independently of each other selected from substituted or unsubstituted C₁₋₄-alkyl, substituted or unsubstituted phenyl or substituted or unsubstituted benzyl, alternatively, R₅ and R₆ taken together with the nitrogen atom to which they are attached, may form an unsubstituted morpholine;
and/or
wherein in a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a)) R₇ is selected from H, substituted or unsubstituted aliphatic or aromatic acyl, substituted or unsubstituted alkyl;
preferably wherein a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a))
R₇ is selected from H, acetyl or unsubstituted alkyl;
more preferably wherein in a functionalized aliphatic polyester homopolymer according to any one of claims 8 and 9 (option a)), a functionalized aliphatic polyester random copolymer according to claim 10, or a functionalized aliphatic polyester copolymer of random and block microstructure according to any one of claims 11 and 12 (option a))
R₇ is H.

18. A process according to any one of claims 5 and 13, wherein
R₁ is selected from CN, COOR₄, CONR₅R₆; and
R₂ is selected from CN, COOR₄, CONR₅R₆, while R₃ is selected from H, substituted or unsubstituted alkyl; substituted or unsubstituted O-alkyl or substituted or unsubstituted O-aryl; or
R₃ is selected from CN, COOR₄, CONR₅R₆, while R₂ is selected from H, substituted or unsubstituted alkyl, substituted or unsubstituted O-alkyl or substituted or unsubstituted O-aryl;
preferably wherein
R₁ is selected from CN, COOR₄, CONR₅R₆; and
R₂ is selected from CN, COOR₄, CONR₅R₆, while R₃ is selected from H, substituted or unsubstituted C₁₋₄-alkyl, substituted or unsubstituted O-C₁₋₄-alkyl or substituted or unsubstituted O-aryl; or
R₃ is selected from CN, COOR₄, CONR₅R₆, while R₂ is selected from H, substituted or unsubstituted C₁₋₄-alkyl, substituted or unsubstituted O-C₁₋₄-alkyl or substituted or unsubstituted O-aryl;
more preferably wherein
R₁ is selected from CN, COOR₄, CONR₅R₆; and
R₂ is selected from CN, COOR₄, CONR₅R₆, while R₃ is selected from H, unsubstituted C₁₋₄-alkyl, unsubstituted O-C₁₋₄-alkyl or substituted or unsubstituted O-phenyl; or
R₃ is selected from CN, COOR₄, CONR₅R₆, while R₂ is selected from H, substituted or unsubstituted C₁₋₄-alkyl or substituted or unsubstituted O-C₁₋₄-alkyl or substituted or unsubstituted O-phenyl;
most preferably wherein
R₁ is selected from CN, COOR₄, CONR₅R₆; and
R₂ is selected from CN, COOR₄, CONR₅R₆, while R₃ is H; or
R₃ is selected from CN, COOR₄, CONR₅R₆, while R₂ is H.

19. A process according to any one of claims 7 or 8, wherein
R₄ is selected from substituted or unsubstituted C₁₋₁₀-alkyl, substituted or unsubstituted aryl, or substituted or unsubstituted C₁₋₄-alkyl-aryl;
R₅ and R₆ are independently of each other selected from substituted or unsubstituted C₁₋₁₀-alkyl, substituted or unsubstituted aryl, substituted or unsubstituted C₁₋₄-alkyl-aryl, substituted or unsubstituted mono- or bi-cyclic heterocyclyl, or substituted or unsubstituted mono- or bi-cyclic C₁₋₄-alkyl-heterocyclyl,
alternatively, R₅ and R₆ taken together with the nitrogen atom to which they are attached, may form a substituted or unsubstituted mono- or bi-cyclic heterocyclyl;
preferably wherein
R₄ is selected from substituted or unsubstituted C₁₋₄-alkyl, substituted or unsubstituted aryl, or substituted or unsubstituted C₁₋₄-alkyl-aryl;
R₅ and R₆ are independently of each other selected from substituted or unsubstituted C₁₋₄-alkyl, substituted or unsubstituted phenyl, substituted or unsubstituted benzyl or substituted or unsubstituted mono- or bi-cyclic heterocyclyl,
alternatively, R₅ and R₆ taken together with the nitrogen atom to which they are attached, may form a substituted or unsubstituted morpholine;
more preferably wherein
R₄ is selected from unsubstituted C₁₋₄-alkyl, substituted or unsubstituted phenyl or substituted or unsubstituted benzyl;
R₅ and R₆ are independently of each other selected from substituted or unsubstituted C₁₋₄-alkyl, substituted or unsubstituted phenyl or substituted or unsubstituted benzyl, alternatively, R₅ and R₆ taken together with the nitrogen atom to which they are attached, may form an unsubstituted morpholine;
and/or
wherein
R₁ is selected from CN, COOR₄, CONR₅R₆;
R₂ is selected from CN, COOR₄, CONR₅R₆, and
R₃ is selected from CN, COOR₄, CONR₅R₆.
and/or
wherein in a functionalized aliphatic polyester homopolymer according to claim 9 (option b)), in a functionalized aliphatic polyester random copolymer according to claim 10, or in a functionalized aliphatic polyester copolymer of random and block microstructure according to claim 12 (option b))
R₇ is selected from H, substituted or unsubstituted aliphatic or aromatic acyl, substituted or unsubstituted alkyl;
preferably wherein in a functionalized aliphatic polyester homopolymer according to claim 9 (option b)), in a functionalized aliphatic polyester random copolymer according to claim 10, or a functionalized aliphatic polyester copolymer of random and block microstructure according to claim 12 (option b))
R₇ is selected from H, acetyl or unsubstituted alkyl;
more preferably wherein in a functionalized aliphatic polyester homopolymer according to claim 9 (option b)), in a functionalized aliphatic polyester random copolymer according to claim 10, or in a functionalized aliphatic polyester copolymer of random and block microstructure according to claim 12 (option b))
R₇ is H.

## Patentansprüche

1. Lactonmonomer mit einer Struktur der allgemeinen Formel (I) 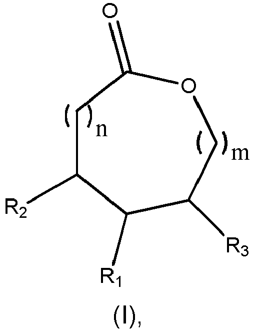 wobei
n ausgewählt ist aus 0, 1 oder 2;
m ausgewählt ist aus 0, 1 oder 2; und
m + n 2 ist;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
mit der Maßgabe, dass
falls n 0 ist, R₂ H ist, und falls m 0 ist, R₃ H ist;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden
wobei Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, -C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'}, -C(O)NR_{c}R_{c'},-NR_{c}S(O)₂R_{c'}, =O, -OCH₂CH₂OR_{c}, -NR_{c}C(O)NR_{c'}R_{c"}, - S(O)₂NR_{c}R_{c'}, -NR_{c}S(O)₂NR_{c'}R_{c"}, Halogenalkyl, Halogenalkoxy, -SR_{c}, -S(O)R_{c}, -S(O)₂R_{c}, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

2. Lactonmonomer nach Anspruch 1, wobei
einer von R₂ oder R₃ H ist
mit der Maßgabe, dass
a) falls R₂ H ist, m nicht 0 ist; oder
b) falls R₃ H ist, n nicht 0 ist.

3. Lactonmonomer nach Anspruch 1 mit einer Struktur der allgemeinen Formel (la) 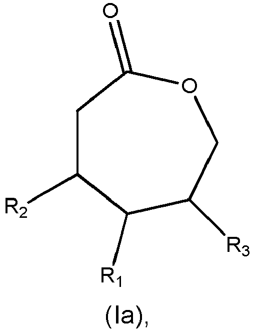 wobei
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden.

4. Lactonmonomer nach Anspruch 1 oder 3 mit einer Struktur der allgemeinen Formel (II) oder (III) 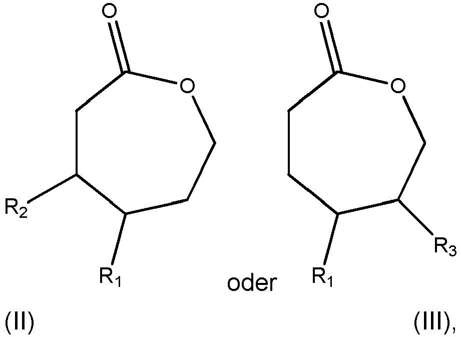 wobei
R₁ ausgewählt ist aus CN, COOR₄, CONR₅R₆, Halogen oder NO₂;
R₂ ausgewählt ist aus CN, COOR₄, CONR₅R₆, Halogen oder NO₂;
R₃ ausgewählt ist aus CN, COOR₄, CONR₅R₆, Halogen oder NO₂;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl
alternativ, R₅ und R₆ zusammen genommen mit dem Stickstoffatom, an das sie gebunden sind, ein substituiertes oder unsubstituiertes Heterocyclyl bilden.

5. Verfahren zum Herstellen eines Lactonmonomers nach Anspruch 1, 2 oder 3, umfassend den letzten Schritt einer Baeyer-Villiger-Oxidation eines Ketons zum Lactonmonomer der Formel (I) nach dem nachstehenden Reaktionsschema: 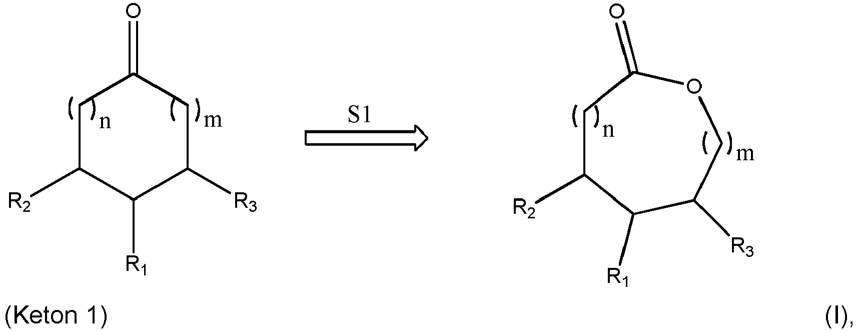 wobei
in dem Reaktionsschritt S1
- ein Oxidationsmittel zugegeben wird, vorzugsweise ein Oxidationsmittel, das ausgewählt ist aus m-Chlorperbenzoesäure (mCPBA), Wasserstoffperoxid, Natriumperborat, Peressigsäure, Trifluorperessigsäure, Perameisensäure, 4-Nitroperbenzoesäure zugegeben wird, mehr bevorzugt mCPBA zugegeben wird;
- das Lösungsmittel ein organisches Lösungsmittel, vorzugsweise CH₂Cl₂ ist, und
- die Temperatur zwischen -5 °C und 90 °C ausgewählt ist, vorzugsweise zwischen 15 °C und 30 °C ausgewählt ist, mehr bevorzugt Raumtemperatur ist;
und wobei in dem Keton 1 und in der Verbindung der Formel (I)
n ausgewählt ist aus 0, 1 oder 2;
m ausgewählt ist aus 0, 1 oder 2; und
m + n 2 ist;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
mit der Maßgabe, dass
falls n 0 ist, R₂ H ist, und falls m 0 ist, R₃ H ist;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
wobei Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, -C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'}, -C(O)NR_{c}R_{c'},-NR_{c}S(O)₂R_{c'}, =O, -OCH₂CH₂OR_{c}, -NR_{c}C(O)NR_{c'}R_{c"}, - S(O)₂NR_{c}R_{c'}, -NR_{c}S(O)₂NR_{c'}R_{c"}, Halogenalkyl, Halogenalkoxy, -SR_{c}, -S(O)R_{c}, -S(O)₂R_{c}, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind;
vorzugsweise
wobei einer von R₂ oder R₃ H ist,
mit der Maßgabe, dass, falls R₂ H ist, m nicht 0 ist; oder falls R₃ H ist, n nicht 0 ist;
und/oder
wobei n und m 1 sind.

6. Verfahren zum Herstellen eines Lactonmonomers nach Anspruch 4, umfassend den letzten Schritt einer Baeyer-Villiger-Oxidation eines Ketons zu dem Lactonmonomer der Formel (II) und (III) nach dem nachstehenden Reaktionsschema: 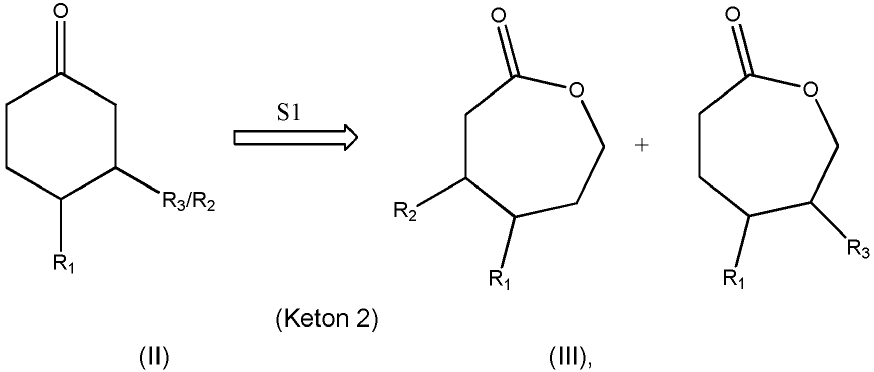 wobei in dem Reaktionsschritt S1
- ein Oxidationsmittel zugegeben wird, vorzugsweise ein Oxidationsmittel, das ausgewählt ist aus mCPBA, Wasserstoffperoxid, Natriumperborat, Peressigsäure, Trifluorperessigsäure, Perameisensäure, 4-Nitroperbenzoesäure zugegeben wird, mehr bevorzugt mCPBA zugegeben wird,
- das Lösungsmittel ein organisches Lösungsmittel, vorzugsweise CH₂Cl₂ ist, und
- die Temperatur zwischen -5 °C und 90 °C ausgewählt ist, vorzugsweise zwischen 15 °C und 30 °C ausgewählt ist, mehr bevorzugt Raumtemperatur ist;
und wobei im Keton 2 und in den Verbindungen der Formeln (II) und (III)
R₁ ausgewählt ist aus CN, COOR₄, CONR₅R₆, Halogen oder NO₂;
R₂ ausgewählt ist aus CN, COOR₄, CONR₅R₆, Halogen oder NO₂;
R₃ ausgewählt ist aus CN, COOR₄, CONR₅R₆, Halogen oder NO₂;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl
alternativ, R₅ und R₆ zusammen genommen mit dem Stickstoffatom, an das sie gebunden sind, ein substituiertes oder unsubstituiertes Heterocyclyl bilden;
wobei Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, -C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'}, -C(O)NR_{c}R_{c'},-NR_{c}S(O)₂R_{c'}, =O, -OCH₂CH₂OR_{c}, -NR_{c}C(O)NR_{c'}R_{c"}, - S(O)₂NR_{c}R_{c'}, -NR_{c}S(O)₂NR_{c'}R_{c"}, Halogenalkyl, Halogenalkoxy, -SR_{c}, -S(O)R_{c}, -S(O)₂R_{c}, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

7. Verfahren nach Anspruch 6, wobei das Keton 2 durch eine Sequenz einer Diels-Alder-Reaktion (S2) gefolgt von TMS-Enol-Spaltung nach dem folgenden Reaktionsschema bereitgestellt wird: 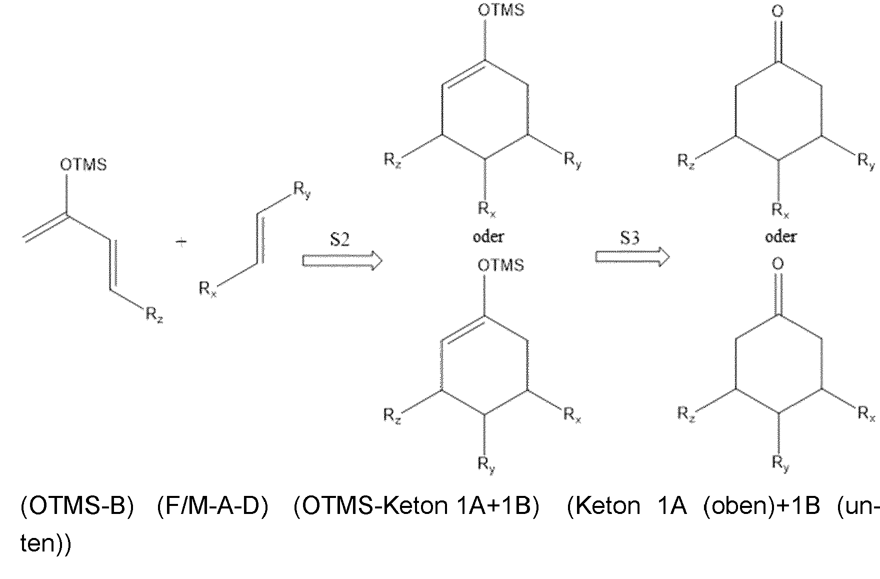
wobei (OTMS-B) 3- oder 2-Trimethylsililoxy-substituiertes Dien ist, vorzugsweise 2-Trimethylsililoxy-1,4-butadien ist, (F/M-A-D) ein Fumarsäure-Maleinsäure-Derivat (F/M-A-D) ist, wobei das 2-Trimethylsililoxy-1,4-butadien (OTMS-B) und/oder das Fumarsäure/Maleinsäure-Derivat (F/M-A-D) vorzugsweise aus Chemikalien synthetisiert werden, die aus ÖI- oder Biomasse abgeleitet sind;
wobei
Rₓ ausgewählt ist aus CN, COOR₄, CONR₅R₆, Halogen oder NO₂;
R_{y} ausgewählt ist aus CN, COOR₄, CONR₅R₆, Halogen oder NO₂;
R_{z} ausgewählt ist aus H, substituiertem oder unsubstituiertem Alkyl oder substituiertem, unsubstituiertem O-Alkyl oder substituiertem oder unsubstituiertem O-Aryl
wobei in dem Reaktionsschritt S2
- die Temperatur zwischen 25 °C und 180 °C ausgewählt ist, vorzugsweise 120 °C bis 140 °C ist, am meisten bevorzugt 130 °C ist;
- die Reaktionszeit von 1 Stunde bis 24 Stunden, vorzugsweise von 3 Stunden bis 5 Stunden ausgewählt ist, am meisten bevorzugt 4 Stunden beträgt;
wobei in dem Reaktionsschritt S3
- Acetonitril, Ethanol oder Methanol, Ameisensäure oder Natriumcarbonat zugegeben wird,
- die Reaktionsmischung filtriert und verdampft wird
- und wahlweise das Endprodukt durch Vakuumdestillation oder durch Chromatographie zusätzlich gereinigt wird; und
wobei schließlich, wenn R_{z} H ist, das Keton 2 nach Anspruch 6 von Keton 1A bereitgestellt wird, in dem Rₓ R₁ ist und R_{y} R₃/R₂ ist.

8. Funktionalisiertes aliphatisches Polyesterhomopolymer der allgemeinen Formel (IV) 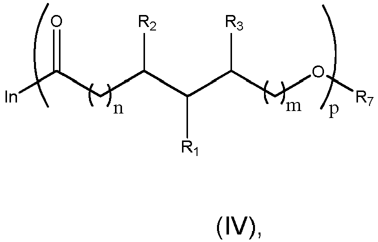 wobei
n ausgewählt ist aus 0, 1 oder 2;
m ausgewählt ist aus 0, 1 oder 2; und
m + n 2 ist;
p ausgewählt ist aus 5-500;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
mit der Maßgabe, dass
falls n 0 ist, R₂ H ist, und falls m 0 ist, R₃ H ist;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
R₇ ausgewählt ist aus H, substituiertem oder unsubstituiertem aliphatischem oder aromatischem Acyl, substituiertem oder unsubstituiertem Alkyl;
wobei vorzugsweise einer von R₂ oder R₃ H ist,
mit der Maßgabe, dass, falls R₂ H ist, m nicht 0 ist; oder falls R₃ H ist, n nicht 0 ist; und
In substituiertes oder unsubstituiertes aliphatisches Alkoxid oder eine primäre oder sekundäre substituierte oder unsubstituierte aliphatische Aminogruppe, vorzugsweise ist Ethoxid ist;
wobei Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, -C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'}, -C(O)NR_{c}R_{c'},-NR_{c}S(O)₂R_{c'}, =O, -OCH₂CH₂OR_{c}, -NR_{c}C(O)NR_{c'}R_{c"}, - S(O)₂NR_{c}R_{c'}, -NR_{c}S(O)₂NR_{c'}R_{c"}, Halogenalkyl, Halogenalkoxy, -SR_{c}, -S(O)R_{c}, -S(O)₂R_{c}, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

9. Funktionalisiertes aliphatisches Polyesterhomopolymer nach Anspruch 8
a) der allgemeinen Formel (IVa) 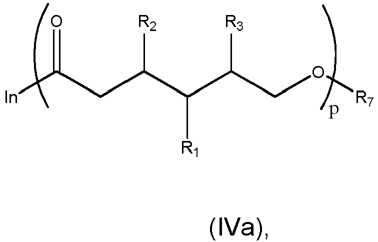 wobei
p ausgewählt ist aus 5-500;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
R₇ ausgewählt ist aus H, substituiertem oder unsubstituiertem aliphatischem oder aromatischem Acyl, substituiertem oder unsubstituiertem Alkyl;
wobei aliphatisches, Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, - C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei aromatisches, Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'} , -C(O)NR_{c}R_{c'},-NR_{c}S(O)₂R_{c'}, =O, -OCH₂CH₂OR_{c}, - NR_{c}C(O)NR_{c'}R_{c"}, -S(O)₂NR_{c}R_{c"}, -NR_{c}S(O)₂NR_{c'}R_{c"}, Halogenalkyl, Halogenalkoxy, -SR_{c}, -S(O)R_{c}, -S(O)₂R,, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind; oder
b) der allgemeinen Formel (V) oder (VI) 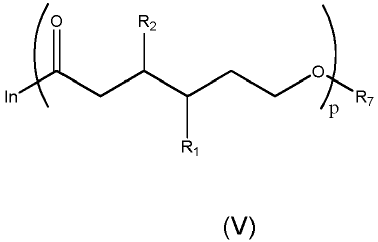 oder 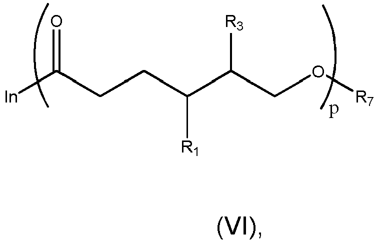 wobei
p ausgewählt ist aus 5-500;
R₁ ausgewählt ist aus CN, COOR₄, CONR₅R₆, Halogen oder NO₂;
R₂ ausgewählt ist aus CN, COOR₄, CONR₅R₆, Halogen oder NO₂;
R₃ ausgewählt ist aus CN, COOR₄, CONR₅R₆, Halogen oder NO₂;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
R₇ ausgewählt ist aus H, substituiertem oder unsubstituiertem aliphatischem oder aromatischem Acyl, substituiertem oder unsubstituiertem Alkyl;
wobei aliphatisches, Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, - C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei aromatisches, Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'} , -C(O)NR_{c}R_{c'},-NR_{c}S(O)₂R_{c'}, =O, -OCH₂CH₂OR_{c}, - NR_{c}C(O)NR_{c'}R_{c"}, -S(O)₂NR_{c}R_{c"}, -NR_{c}S(O)₂NR_{c'}R_{c"}, Halogenalkyl, Halogenalkoxy, -SR_{c}, -S(O)R_{c}, -S(O)₂R,, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

10. Funktionalisiertes aliphatisches statistisches Copolymer der allgemeinen Formel (VII) 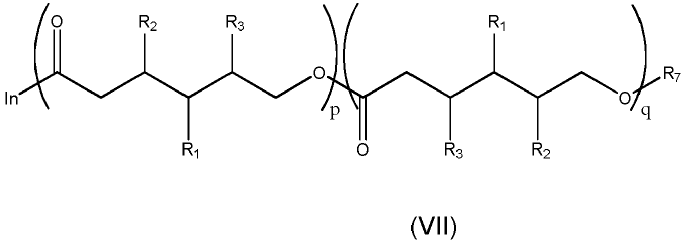
p ausgewählt ist aus 5-500,
q ausgewählt ist aus 5-500,
p+q 10-500 ist;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
R₇ ausgewählt ist aus H, substituiertem oder unsubstituiertem aliphatischem oder aromatischem Acyl, substituiertem oder unsubstituiertem Alkyl;
wobei aliphatisches, Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, - C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei aromatisches, Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'} , -C(O)NR_{c}R_{c'},-NR_{c}S(O)₂R_{c'}, =O, -OCH₂CH₂OR_{c}, - NR_{c}C(O)NR_{c'}R_{c"}, -S(O)₂NR_{c}R_{c"}, -NR_{c}S(O)₂NR_{c'}R_{c"}, Halogenalkyl, Halogenalkoxy, -SR_{c}, -S(O)R_{c}, -S(O)₂R,, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

11. Funktionalisiertes aliphatisches Polyestercopolymer von statistischer und Blockmikrostruktur der allgemeinen Formel (VIII) oder (IX) 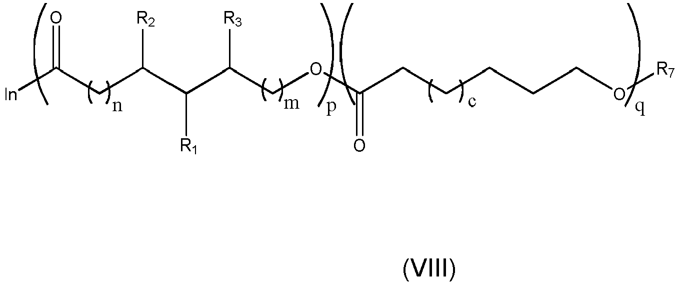 oder 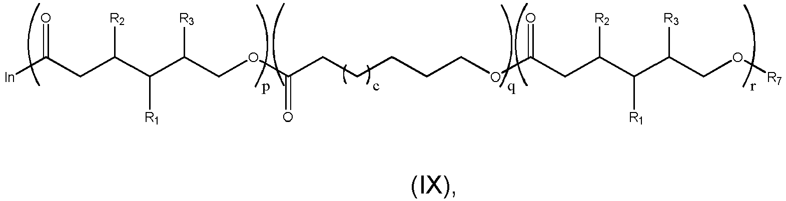 wobei
n ausgewählt ist aus 0, 1 oder 2,
m ausgewählt ist aus 0, 1 oder 2, und
m + n 2 ist,
c ausgewählt ist aus 0 oder 1;
p ausgewählt ist aus 5-500,
q ausgewählt ist aus 5-500; und
r ausgewählt ist aus 5-500,
p+q+r 15-500 ist;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
mit der Maßgabe, dass
falls n 0 ist, R₂ H ist, und falls m 0 ist, R₃ H ist;
R₄ ausgewählt ist aus substituiertem oder unsubstituiertem Alkyl, substituiertem oder unsubstituiertem Alkenyl, substituiertem oder unsubstituiertem Alkin, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
wobei vorzugsweise einer von R₂ oder R₃ H ist,
mit der Maßgabe, dass, falls R₂ H ist, m nicht 0 ist; oder falls R₃ H ist, n nicht 0 ist;
R₇ ausgewählt ist aus H, substituiertem oder unsubstituiertem aliphatischem oder aromatischem Acyl, substituiertem oder unsubstituiertem Alkyl;
wobei aliphatisches, Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R,, -OR_{c}, -C(O)OR_{c}, -CN, - C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei aromatisches, Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂, -NR_{c}R_{c'}, -C(O)OR_{c}, NR_{c}C(O)R_{c'} , -C(O)NR_{c}R_{c'},-NR_{c}S(O)₂R_{c'}, =O, -OCH₂CH₂OR_{c}, - NR_{c}C(O)NR_{c'}R_{c"}, -S(O)₂NR_{c}R_{c"}, -NR_{c}S(O)₂NR_{c'}R_{c"}, Halogenalkyl, Halogenalkoxy, -SR_{c}, -S(O)R_{c}, -S(O)₂R,, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

12. Funktionalisiertes aliphatisches Polyestercopolymer der statistischen und Blockmikrostruktur nach Anspruch 11
a) der allgemeinen Formel (VIIIa) oder (IXa) 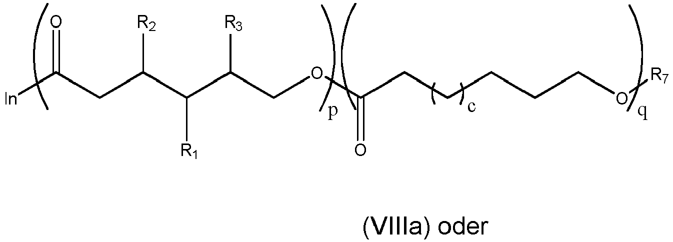 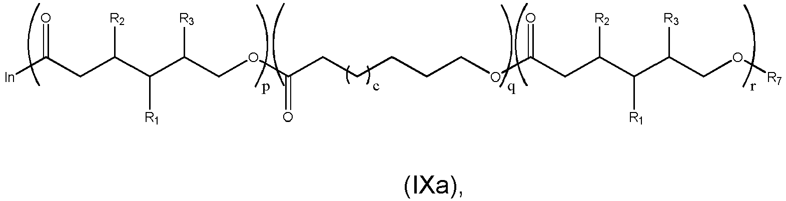 wobei
c ausgewählt ist aus 0 oder 1,
p ausgewählt ist aus 5-500,
q ausgewählt ist aus 5-500; und
r ausgewählt ist aus 5-500,
p+q+r 15-500 ist;
R₁ ausgewählt ist aus CN, COOR₄, CONR₅R₆, Halogen oder NO₂;
einer von R₂ und R₃ ausgewählt ist aus CN, COOR₄, CONR₅R₆, Halogen oder NO₂, während der andere ausgewählt ist aus H, substituiertem oder unsubstituiertem Alkyl, substituiertem oder unsubstituiertem O-Alkyl oder substituiertem oder unsubstituiertem O-Aryl;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
R₇ ausgewählt ist aus H, substituiertem oder unsubstituiertem aliphatischem oder aromatischem Acyl, substituiertem oder unsubstituiertem Alkyl;
wobei aliphatisches, Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NReRe_{'}, -SR_{c}, -S(O)R_{c}, -S(O)₂R_{c}, -OR_{c}, -C(O)OR_{c}, -CN,-C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei aromatisches, Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c}, -CN, -NO₂ , -NR_{c}R_{c'}, -C(O)OR_{c}, NR_{c}C(O)R_{c'} , -C(O)NR_{c}R_{c'}, -NR_{c}S(O)₂R_{c'}, =O, -OCH₂CH₂OR_{c},-NR_{c}C(O)NR_{c'}R_{c"}, -S(O)₂NR_{c}R_{c'}, -NR_{c}S(O)₂NR_{c'}R_{c"}, Halogenalkyl, Halogenalkoxy, -SR_{c}, -S(O)R_{c}, -S(O)₂R_{c}, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind; oder
b) der allgemeinen Formel (X) oder (XI) und (XII) oder (XIII) 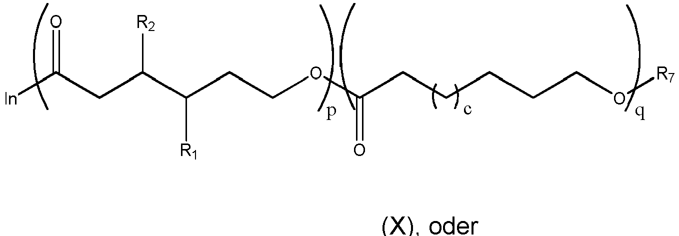 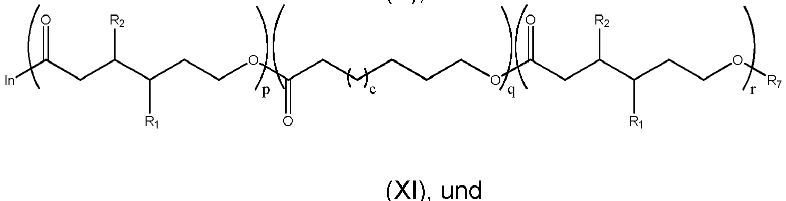 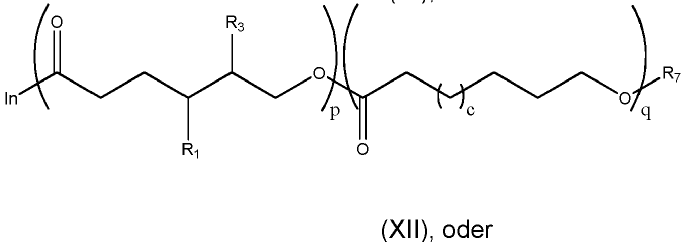 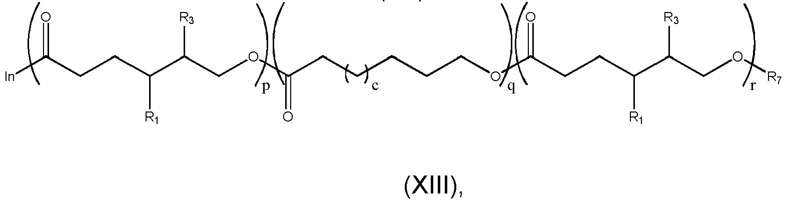 wobei
c ausgewählt ist aus 0 oder 1;
p ausgewählt ist aus 5-500;
q ausgewählt ist aus 5-500 und
r ausgewählt ist aus 5-500,
p+q+r 15-500 ist,
R₁ ausgewählt ist aus CN, COOR₄, CONR₅R₆, Halogen oder NO₂;
R₂ ausgewählt ist aus CN, COOR₄, CONR₅R₆, Halogen oder NO₂;
R₃ ausgewählt ist aus CN, COOR₄, CONR₅R₆, Halogen oder NO₂;
R₄ ausgewählt ist aus substituiertem oder unsubstituiertem Alkyl, substituiertem oder unsubstituiertem Alkenyl, substituiertem oder unsubstituiertem Alkinyl, substituiertem oder unsubstituiertem Aryl oder substituiertem oder unsubstituiertem Alkylaryl;
R₅ und R₆ unabhängig voneinander ausgewählt sind aus substituiertem oder unsubstituiertem Alkyl, substituiertem oder unsubstituiertem Alkenyl, substituiertem oder unsubstituiertem Alkinyl, substituiertem oder unsubstituiertem Aryl, substituiertem oder unsubstituiertem Alkylaryl, substituiertem oder unsubstituiertem Heterocyclyl oder substituiertem oder unsubstituiertem Alkylheterocyclyl,
alternativ, R₅ und R₆ zusammen genommen mit dem Stickstoffatom, an das sie gebunden sind, ein substituiertes oder unsubstituiertes Heterocyclyl bilden;
R₇ ausgewählt ist aus H, substituiertem oder unsubstituiertem aliphatischem oder aromatischem Acyl, substituiertem oder unsubstituiertem Alkyl;
wobei aliphatisches, Alkyl, Alkenyl, Alkinyl und O-Alkyl falls substituiert, sowie Alkylaryl, Alkylheterocycly, Alkylcycloalkyl falls am Alkyl substituiert, durch Halogen (F, Cl, Br, I), -NR_{c}R_{c'}, -SR_{c}, -S(O)R_{c}, -S(O)₂R_{c}, -OR_{c}, -C(O)OR_{c}, -CN,-C(O)NR_{c}R_{c'}, oder ein lineares oder verzweigtes, substituiertes oder unsubstituiertes -OC₁₋₆-Alkyl substituiert ist, wobei R_{c} und R_{c'} durch H oder durch ein lineares oder verzweigtes, substituiertes oder unsubstituiertes C₁₋₆-Alkyl dargestellt sind;
wobei aromatisches, Aryl, Cycloalkyl oder Heterocyclyl, falls substituiert, sowie Alkyl-Aryl, Alkyl-Cycloalkyl oder Alkyl-Heterocyclyl, falls am Ringsystem substituiert, durch Halogen (F, Cl, Br, I), -R_{c}, -OR_{c} , -CN, -NO₂ , -NR_{c}R_{c'}, -C(O)OR_{c}, NR_{c}C(O)R_{c'} , -C(O)NR_{c}R_{c'}, -NR_{c}S(O)₂R_{c'}, =O, -OCH₂CH₂OR_{c},-NR_{c}C(O)NR_{c'}R_{c"}, -S(O)₂NR_{c}R_{c'}, -NR_{c}S(O)₂NR_{c'}R_{c"}, Halogenalkyl, Halogenalkoxy, -SR_{c}, -S(O)R_{c}, -S(O)₂R_{c}, -C(CH₃)OR_{c}; NR_{c}R_{c'} oder ein lineares oder verzweigtes, substituiertes oder unsubstituiertes -OC₁₋₆-Alkyl substituiert ist, wobei R_{c}, R_{c'} und R_{c"} durch H oder durch ein lineares oder verzweigtes, substituiertes oder unsubstituiertes C₁₋₆-Alkyl dargestellt sind.

13. Lactonmonomer nach einem der Ansprüche 1 bis 3, funktionalisiertes aliphatisches Polyesterhomopolymer nach einem der Ansprüche 8 und 9 (Option a)), funktionalisiertes aliphatisches statistisches Polyestercopolymer nach Anspruch 10 oder funktionalisiertes aliphatisches Polyestercopolymer der statistischen und Blockmikrostruktur nach einem der Ansprüche 11 und 12 (Option a)), wobei
R₄ ausgewählt ist aus substituiertem oder unsubstituiertem C₁₋₁₀-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₁₀-Alkyl, substituiertem oder unsubstituiertem Aryl, substituiertem oder unsubstituiertem C₁₋₄-Alkylaryl, substituiertem oder unsubstituiertem mono- oder bicyclischem Heterocyclyl oder substituiertem oder unsubstituiertem mono- oder bicyclischem C₁₋₄-Alkylheterocyclyl, alternativ, R₅ und R₆ zusammen genommen mit dem Stickstoffatom, an das sie gebunden sind, ein substituiertes oder unsubstituiertes mono- oder bicyclisches Heterocyclyl bilden;
vorzugsweise wobei
R₄ ausgewählt ist aus substituiertem oder unsubstituiertem C₁₋₄-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₄-Alkyl, substituiertem oder unsubstituiertem Phenyl, substituiertem oder unsubstituiertem Benzyl oder substituiertem oder unsubstituiertem mono- oder bicyclischem Heterocyclyl,
alternativ, R₅ und R₆ zusammen genommen mit dem Stickstoffatom, an das sie gebunden sind, ein substituiertes oder unsubstituiertes Morpholin bilden;
mehr bevorzugt wobei
R₄ ausgewählt ist aus unsubstituiertem C₁₋₄-Alkyl, substituiertem oder unsubstituiertem Phenyl oder substituiertem oder unsubstituiertem Benzyl;
R₅ und R₆ unabhängig voneinander ausgewählt sind aus substituiertem oder unsubstituiertem C₁₋₄-Alkyl, substituiertem oder unsubstituiertem Phenyl oder substituiertem oder unsubstituiertem Benzyl,
alternativ, R₅ und R₆ zusammen genommen mit dem Stickstoffatom, an das sie gebunden sind, ein unsubstituiertes Morpholin bilden können;
und/oder
wobei in einem funktionalisierten aliphatischen Polyesterhomopolymer nach einem der Ansprüche 8 und 9 (Option a)) einem funktionalisierten aliphatischen statistischen Polyestercopolymer nach Anspruch 10 oder einem funktionalisierten aliphatischen Polyestercopolymer der statistischen und Blockmikrostruktur nach einem der Ansprüche 11 und 12 (Option a))
R₇ ausgewählt ist aus H, substituiertem oder unsubstituiertem aliphatischem oder aromatischem Acyl, substituiertem oder unsubstituiertem Alkyl;
vorzugsweise wobei in einem funktionalisierten aliphatischen Polyesterhomopolymer nach einem der Ansprüche 8 und 9 (Option a)), einem funktionalisierten aliphatischen statistischen Polyestercopolymer nach Anspruch 10 oder einem funktionalisierten aliphatischen Polyestercopolymer der statistischen und Blockmikrostruktur nach einem der Ansprüche 11 und 12 (Option a))
R₇ ausgewählt ist aus H, Acetyl oder unsubstituiertem Alkyl;
mehr bevorzugt wobei in einem funktionalisierten aliphatischen Polyesterhomopolymer nach einem der Ansprüche 8 und 9 (Option a)), einem funktionalisierten aliphatischen statistischen Polyestercopolymer nach Anspruch 10 oder einem funktionalisierten aliphatischen Polyestercopolymer der statistischen und Blockmikrostruktur nach einem der Ansprüche 11 und 12 (Option a)) R₇ H ist.

14. Lactonmonomer nach einem der Ansprüche 1 bis 3 und 13, Verfahren funktionalisiertes aliphatisches Polyesterhomopolymer nach einem der Ansprüche 8, 9 (Option a)) und 13, funktionalisiertes aliphatisches statistisches Polyestercopolymer nach einem der Ansprüche 10 oder 13 oder funktionalisiertes aliphatisches Polyestercopolymer der statistischen und Blockmikrostruktur nach einem der Ansprüche 11, 12 (Option a)) und 13, wobei
R₁ ausgewählt ist aus CN, COOR₄, CONR₅R₆; und
R₂ ausgewählt ist aus CN, COOR₄, CONR₅R₆, während R₃ ausgewählt ist aus H, substituiertem oder unsubstituiertem Alkyl; substituiertem oder unsubstituiertem O-Alkyl oder substituiertem oder unsubstituiertem O-Aryl; oder
R₃ ausgewählt ist aus CN, COOR₄, CONR₅R₆, während R₂ ausgewählt ist aus H, substituiertem oder unsubstituiertem Alkyl, substituiertem oder unsubstituiertem O-Alkyl oder substituiertem oder unsubstituiertem O-Aryl;
vorzugsweise wobei
R₁ ausgewählt ist aus CN, COOR₄, CONR₅R₆; und
R₂ ausgewählt ist aus CN, COOR₄, CONR₅R₆, während R₃ ausgewählt ist aus H, substituiertem oder unsubstituiertem C₁₋₄-Alkyl, substituiertem oder unsubstituiertem O-C₁₋₄-Alkyl oder substituiertem oder unsubstituiertem O-Aryl; oder R₃ ausgewählt ist aus CN, COOR₄, CONR₅R₆, während R₂ ausgewählt ist aus H, substituiertem oder unsubstituiertem C₁₋₄-Alkyl, substituiertem oder unsubstituiertem O-C₁₋₄-Alkyl oder substituiertem oder unsubstituiertem O-Aryl;
mehr bevorzugt wobei
R₁ ausgewählt ist aus CN, COOR₄, CONR₅R₆; und
R₂ ausgewählt ist aus CN, COOR₄, CONR₅R₆, während R₃ ausgewählt ist aus H, unsubstituiertem C₁₋₄-Alkyl, unsubstituiertem O-C₁₋₄-Alkyl oder substituiertem oder unsubstituiertem O-Phenyl; oder
R₃ ausgewählt ist aus CN, COOR₄, CONR₅R₆, während R₂ ausgewählt ist aus H, substituiertem oder unsubstituiertem C₁₋₄-Alkyl oder substituiertem oder unsubstituiertem O-C₁₋₄-Alkyl oder substituiertem oder unsubstituiertem O-Phenyl;
am meisten bevorzugt wobei
R₁ ausgewählt ist aus CN, COOR₄, CONR₅R₆; und
R₂ ausgewählt ist aus CN, COOR₄, CONR₅R₆, während R₃ H ist; oder
R₃ ausgewählt ist aus CN, COOR₄, CONR₅R₆, während R₂ H ist.

15. Lactonmonomer nach Anspruch 4, funktionalisiertes aliphatisches Polyesterhomopolymer nach Anspruch 9 (Option b)), funktionalisiertes aliphatisches statistisches Polyestercopolymer nach Anspruch 10 oder funktionalisiertes aliphatisches Polyestercopolymer der statistischen und Blockmikrostruktur nach Anspruch 12 (Option b)), wobei
R₄ ausgewählt ist aus substituiertem oder unsubstituiertem C₁₋₁₀-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₁₀-Alkyl, substituiertem oder unsubstituiertem Aryl, substituiertem oder unsubstituiertem C₁₋₄-Alkylaryl, substituiertem oder unsubstituiertem mono- oder bicyclischem Heterocyclyl oder substituiertem oder unsubstituiertem mono- oder bicyclischem C₁₋₄-Alkylheterocyclyl, alternativ, R₅ und R₆ zusammen genommen mit dem Stickstoffatom, an das sie gebunden sind, ein substituiertes oder unsubstituiertes mono- oder bicyclisches Heterocyclyl bilden;
vorzugsweise wobei
R₄ ausgewählt ist aus substituiertem oder unsubstituiertem C₁₋₄-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₄-Alkyl, substituiertem oder unsubstituiertem Phenyl, substituiertem oder unsubstituiertem Benzyl oder substituiertem oder unsubstituiertem mono- oder bicyclischem Heterocyclyl,
alternativ, R₅ und R₆ zusammen genommen mit dem Stickstoffatom, an das sie gebunden sind, ein substituiertes oder unsubstituiertes Morpholin bilden;
mehr bevorzugt wobei
R₄ ausgewählt ist aus unsubstituiertem C₁₋₄-Alkyl, substituiertem oder unsubstituiertem Phenyl oder substituiertem oder unsubstituiertem Benzyl;
R₅ und R₆ unabhängig voneinander ausgewählt sind aus substituiertem oder unsubstituiertem C₁₋₄-Alkyl, substituiertem oder unsubstituiertem Phenyl oder substituiertem oder unsubstituiertem Benzyl,
alternativ, R₅ und R₆ zusammen genommen mit dem Stickstoffatom, an das sie gebunden sind, ein unsubstituiertes Morpholin bilden können;
und/oder
wobei
R₁ ausgewählt ist aus CN, COOR₄, CONR₅R₆;
R₂ ausgewählt ist aus CN, COOR₄, CONR₅R₆; und
R₃ ausgewählt ist aus CN, COOR₄, CONR₅R₆
und/oder
wobei in einem funktionalisierten aliphatischen Polyesterhomopolymer nach Anspruch 9 (Option b)), in einem funktionalisierten aliphatischen statistischen Polyestercopolymer nach Anspruch 10 oder in einem funktionalisierten aliphatischen Polyestercopolymer der statistischen und Blockmikrostruktur nach Anspruch 12 (Option b))
R₇ ausgewählt ist aus H, substituiertem oder unsubstituiertem aliphatischem oder aromatischem Acyl, substituiertem oder unsubstituiertem Alkyl;
vorzugsweise wobei in einem funktionalisierten aliphatischen Polyesterhomopolymer nach Anspruch 9 (Option b)), in einem funktionalisierten aliphatischen statistischen Polyestercopolymer nach Anspruch 10 oder einem funktionalisierten aliphatischen Polyestercopolymer der statistischen und Blockmikrostruktur nach Anspruch 12 (Option b))
R₇ ausgewählt ist aus H, Acetyl oder unsubstituiertem Alkyl;
mehr bevorzugt wobei in einem funktionalisierten aliphatischen Polyesterhomopolymer nach Anspruch 9 (Option b)), in einem funktionalisierten aliphatischen statistischen Polyestercopolymer nach Anspruch 10 oder in einem funktionalisierten aliphatischen Polyestercopolymer der statistischen und Blockmikrostruktur nach Anspruch 12 (Option b))
R₇ H ist.

16. Verfahren zur ROP für die Herstellung eines funktionalisierten aliphatischen Polyesterhomopolymer nach einem der Ansprüche 8 oder 9, eines aliphatischen statistischen Polyestercopolymers nach Anspruch 10 oder eines funktionalisierten aliphatischen Polyestercopolymers der statistischen und Blockmikrostruktur nach Anspruch 11 oder 12, wobei die ROP in Gegenwart eines basischen, sauren Zinn- oder Aluminiumkatalysators ausgeführt wird; vorzugsweise in Gegenwart eines Aluminiumalkyl- und -alkoxidkatalysators; mehr bevorzugt in Gegenwart eines Aluminiumalkyl- und Aluminiumalkoxidkatalysators, der ausgewählt ist aus Diethylaluminiumethoxid, Methylaluminium-bis(2,6-di-tert-butyl-4-methylphenolat) oder Methylaluminiumbis(2,6-di-phe-nylphenolat); am meisten bevorzugt in Gegenwart von Methylaluminiumbis(2,6-di-phenylphenolat),
vorzugsweise
in Gegenwart von primärem Alkohol als Initiator; mehr bevorzugt in Gegenwart von Ethanol als Initiator.

17. Verfahren nach Anspruch 5, wobei
R₄ ausgewählt ist aus substituiertem oder unsubstituiertem C₁₋₁₀-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₁₀-Alkyl, substituiertem oder unsubstituiertem Aryl, substituiertem oder unsubstituiertem C₁₋₄-Alkylaryl, substituiertem oder unsubstituiertem mono- oder bicyclischem Heterocyclyl oder substituiertem oder unsubstituiertem mono- oder bicyclischem C₁₋₄-Alkylheterocyclyl, alternativ, R₅ und R₆ zusammen genommen mit dem Stickstoffatom, an das sie gebunden sind, ein substituiertes oder unsubstituiertes mono- oder bicyclisches Heterocyclyl bilden;
vorzugsweise wobei
R₄ ausgewählt ist aus substituiertem oder unsubstituiertem C₁₋₄-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₄-Alkyl, substituiertem oder unsubstituiertem Phenyl, substituiertem oder unsubstituiertem Benzyl oder substituiertem oder unsubstituiertem mono- oder bicyclischem Heterocyclyl,
alternativ, R₅ und R₆ zusammen genommen mit dem Stickstoffatom, an das sie gebunden sind, ein substituiertes oder unsubstituiertes Morpholin bilden;
mehr bevorzugt wobei
R₄ ausgewählt ist aus unsubstituiertem C₁₋₄-Alkyl, substituiertem oder unsubstituiertem Phenyl oder substituiertem oder unsubstituiertem Benzyl;
R₅ und R₆ unabhängig voneinander ausgewählt sind aus substituiertem oder unsubstituiertem C₁₋₄-Alkyl, substituiertem oder unsubstituiertem Phenyl oder substituiertem oder unsubstituiertem Benzyl,
alternativ, R₅ und R₆ zusammen genommen mit dem Stickstoffatom, an das sie gebunden sind, ein unsubstituiertes Morpholin bilden können;
und/oder
wobei in einem funktionalisierten aliphatischen Polyesterhomopolymer nach einem der Ansprüche 8 und 9 (Option a)) einem funktionalisierten aliphatischen statistischen Polyestercopolymer nach Anspruch 10 oder einem funktionalisierten aliphatischen Polyestercopolymer der statistischen und Blockmikrostruktur nach einem der Ansprüche 11 und 12 (Option a))
R₇ ausgewählt ist aus H, substituiertem oder unsubstituiertem aliphatischem oder aromatischem Acyl, substituiertem oder unsubstituiertem Alkyl;
vorzugsweise wobei in einem funktionalisierten aliphatischen Polyesterhomopolymer nach einem der Ansprüche 8 und 9 (Option a)), einem funktionalisierten aliphatischen statistischen Polyestercopolymer nach Anspruch 10 oder einem funktionalisierten aliphatischen Polyestercopolymer der statistischen und Blockmikrostruktur nach einem der Ansprüche 11 und 12 (Option a)) R₇ ausgewählt ist aus H, Acetyl oder unsubstituiertem Alkyl;
mehr bevorzugt wobei in einem funktionalisierten aliphatischen Polyesterhomopolymer nach einem der Ansprüche 8 und 9 (Option a)), einem funktionalisierten aliphatischen statistischen Polyestercopolymer nach Anspruch 10 oder einem funktionalisierten aliphatischen Polyestercopolymer der statistischen und Blockmikrostruktur nach einem der Ansprüche 11 und 12 (Option a))
R₇ H ist.

18. Verfahren nach einem der Ansprüche 5 und 13; wobei
R₁ ausgewählt ist aus CN, COOR₄, CONR₅R₆; und
R₂ ausgewählt ist aus CN, COOR₄, CONR₅R₆, während R₃ ausgewählt ist aus H, substituiertem oder unsubstituiertem Alkyl; substituiertem oder unsubstituiertem O-Alkyl oder substituiertem oder unsubstituiertem O-Aryl; oder
R₃ ausgewählt ist aus CN, COOR₄, CONR₅R₆, während R₂ ausgewählt ist aus H, substituiertem oder unsubstituiertem Alkyl, substituiertem oder unsubstituiertem O-Alkyl oder substituiertem oder unsubstituiertem O-Aryl;
vorzugsweise wobei
R₁ ausgewählt ist aus CN, COOR₄, CONR₅R₆; und
R₂ ausgewählt ist aus CN, COOR₄, CONR₅R₆, während R₃ ausgewählt ist aus H, substituiertem oder unsubstituiertem C₁₋₄-Alkyl, substituiertem oder unsubstituiertem O-C₁₋₄-Alkyl oder substituiertem oder unsubstituiertem O-Aryl; oder R₃ ausgewählt ist aus CN, COOR₄, CONR₅R₆, während R₂ ausgewählt ist aus H, substituiertem oder unsubstituiertem C₁₋₄-Alkyl, substituiertem oder unsubstituiertem O-C₁₋₄-Alkyl oder substituiertem oder unsubstituiertem O-Aryl;
mehr bevorzugt wobei
R₁ ausgewählt ist aus CN, COOR₄, CONR₅R₆; und
R₂ ausgewählt ist aus CN, COOR₄, CONR₅R₆, während R₃ ausgewählt ist aus H, unsubstituiertem C₁₋₄-Alkyl, unsubstituiertem O-C₁₋₄-Alkyl oder substituiertem oder unsubstituiertem O-Phenyl; oder
R₃ ausgewählt ist aus CN, COOR₄, CONR₅R₆, während R₂ ausgewählt ist aus H, substituiertem oder unsubstituiertem C₁₋₄-Alkyl oder substituiertem oder unsubstituiertem O-C₁₋₄-Alkyl oder substituiertem oder unsubstituiertem O-Phenyl;
am meisten bevorzugt wobei
R₁ ausgewählt ist aus CN, COOR₄, CONR₅R₆; und
R₂ ausgewählt ist aus CN, COOR₄, CONR₅R₆, während R₃ H ist; oder
R₃ ausgewählt ist aus CN, COOR₄, CONR₅R₆, während R₂ H ist.

19. Verfahren nach einem der Ansprüche 7 oder 8 wobei
R₄ ausgewählt ist aus substituiertem oder unsubstituiertem C₁₋₁₀-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₁₀-Alkyl, substituiertem oder unsubstituiertem Aryl, substituiertem oder unsubstituiertem C₁₋₄-Alkylaryl, substituiertem oder unsubstituiertem mono- oder bicyclischem Heterocyclyl oder substituiertem oder unsubstituiertem mono- oder bicyclischem C₁₋₄-Alkylheterocyclyl,
alternativ, R₅ und R₆ zusammen genommen mit dem Stickstoffatom, an das sie gebunden sind, ein substituiertes oder unsubstituiertes mono- oder bicyclisches Heterocyclyl bilden;
vorzugsweise wobei
R₄ ausgewählt ist aus substituiertem oder unsubstituiertem C₁₋₄-Alkyl, substituiertem oder unsubstituiertem Aryl oder substituiertem oder unsubstituiertem C₁₋₄-Alkylaryl;
R₅ und R₆ unabhängig voneinander ausgewählt sind aus substituiertem oder unsubstituiertem C₁₋₄-Alkyl, substituiertem oder unsubstituiertem Phenyl, substituiertem oder unsubstituiertem Benzyl oder substituiertem oder unsubstituiertem mono- oder bicyclischem Heterocyclyl,
alternativ, R₅ und R₆ zusammen genommen mit dem Stickstoffatom, an das sie gebunden sind, ein substituiertes oder unsubstituiertes Morpholin bilden;
mehr bevorzugt wobei
R₄ ausgewählt ist aus unsubstituiertem C₁₋₄-Alkyl, substituiertem oder unsubstituiertem Phenyl oder substituiertem oder unsubstituiertem Benzyl;
R₅ und R₆ unabhängig voneinander ausgewählt sind aus substituiertem oder unsubstituiertem C₁₋₄-Alkyl, substituiertem oder unsubstituiertem Phenyl oder substituiertem oder unsubstituiertem Benzyl,
alternativ, R₅ und R₆ zusammen genommen mit dem Stickstoffatom, an das sie gebunden sind, ein unsubstituiertes Morpholin bilden können;
und/oder
wobei
R₁ ausgewählt ist aus CN, COOR₄, CONR₅R₆;
R₂ ausgewählt ist aus CN, COOR₄, CONR₅R₆; und
R₃ ausgewählt ist aus CN, COOR₄, CONR₅R₆
und/oder
wobei in einem funktionalisierten aliphatischen Polyesterhomopolymer nach Anspruch 9 (Option b)), in einem funktionalisierten aliphatischen statistischen Polyestercopolymer nach Anspruch 10 oder in einem funktionalisierten aliphatischen Polyestercopolymer der statistischen und Blockmikrostruktur nach Anspruch 12 (Option b))
R₇ ausgewählt ist aus H, substituiertem oder unsubstituiertem aliphatischem oder aromatischem Acyl, substituiertem oder unsubstituiertem Alkyl;
vorzugsweise wobei in einem funktionalisierten aliphatischen Polyesterhomopolymer nach Anspruch 9 (Option b)), in einem funktionalisierten aliphatischen statistischen Polyestercopolymer nach Anspruch 10 oder einem funktionalisierten aliphatischen Polyestercopolymer der statistischen und Blockmikrostruktur nach Anspruch 12 (Option b))
R₇ ausgewählt ist aus H, Acetyl oder unsubstituiertem Alkyl;
mehr bevorzugt wobei in einem funktionalisierten aliphatischen Polyesterhomopolymer nach Anspruch 9 (Option b)), in einem funktionalisierten aliphatischen statistischen Polyestercopolymer nach Anspruch 10 oder in einem funktionalisierten aliphatischen Polyestercopolymer der statistischen und Blockmikrostruktur nach Anspruch 12 (Option b))
R₇ H ist.

## Revendications

1. Monomère lactone ayant une structure de formule générale (I) 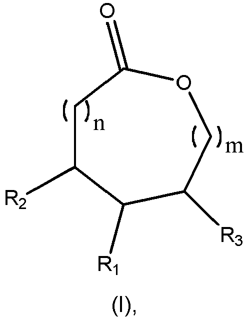 où
n est choisi parmi 0, 1 ou 2 ;
m est choisi parmi 0, 1 ou 2 ; et
m + n est égal à 2 ;
R₁ est choisi parmi CN, COOR₄, CONR₅R₆, halogène ou NO₂ ;
l'un de R₂ et R₃ est choisi parmi CN, COOR₄, CONR₅R₆ , un halogène ou NO₂ , tandis que l'autre est choisi parmi H, un alkyle substitué ou non, un O-alkyle substitué ou non substitué, ou un O-aryle substitué ou non substitué;
à la condition que
si n est égal à 0, R₂ est H, et si m est égal à 0, R₃ est H ;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué,
dans laquel l'alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} , -C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquel l'aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'}, -C(O)OR_{c}, NR_{c}C(O)R_{c'}, -C(O)NR_{c} R_{c'}, -NR_{c}S(O)₂R_{c'}, =O, -OCH₂CH₂OR_{c} , -NR_{c}C(O)NR_{c'}R_{c"},-S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c},-C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆ -alkyle linéaire ou ramifié, substitué ou non substitué.

2. Monomère lactone selon la revendication 1, dans lequel
l'un de R₂ ou R₃ est H
à la condition que
a) si R₂ est H, m n'est pas 0 ; ou
b) si R₃ est H, n n'est pas 0.

3. Monomère lactone selon la revendication 1, ayant une structure de formule générale (la) 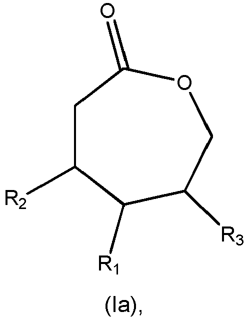 où
R₁ est choisi parmi CN, COOR₄, CONR₅R₆, halogène ou NO₂;
l'un de R₂ et R₃ est choisi parmi CN, COOR₄, CONR₅R₆ , halogène ou NO₂, tandis que l'autre est choisi parmi H, un alkyle substitué ou non substitué ou un O-alkyle substitué ou non substitué, ou un O-aryle substitué ou non substitué;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitués ou non substitué, alcényle substitué ou non substitué, alcynyle substitué ou non substitué, aryle substitués ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué.

4. Monomère lactone selon la revendication 1 ou 3, ayant une structure de formule générale (II) ou (III) 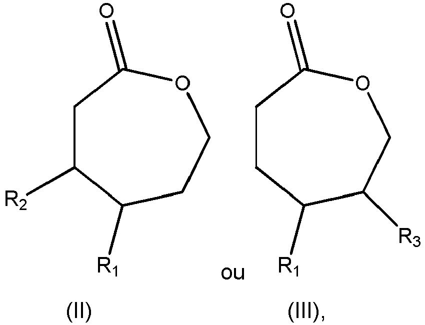 où
R₁ est choisi parmi CN, COOR₄, CONR₅R₆ , halogène ou NO₂;
R₂ est choisi parmi CN, COOR₄, CONR₅R₆, halogène ou NO₂;
R₃ est choisi parmi CN, COOR₄, CONR₅R₆ , halogène ou NO₂;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkylaryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué.

5. Procédé de production d'un monomère lactone selon les revendications 1, 2 ou 3, comprenant l'étape finale d'une oxydation de Baeyer-Villiger d'une cétone en monomère lactone de formule (I) suivant le schéma réactionnel ci-dessous : 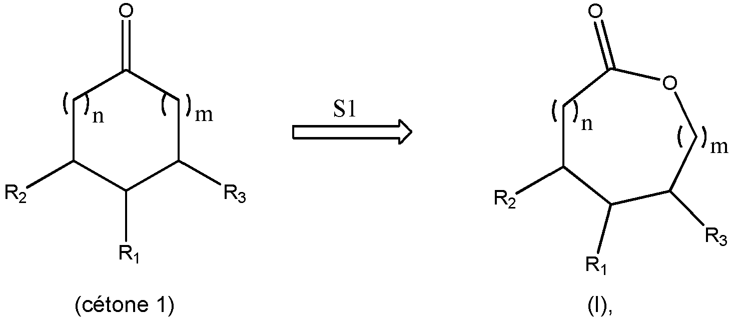 où
dans l'étape de réaction S1
- un oxydant est ajouté, de préférence un oxydant choisi parmi l'acide m-chloroperbenzoïque (mCPBA), le peroxyde d'hydrogène, le perborate de sodium, l'acide peracétique, l'acide trifluoroperacétique, l'acide performique, l'acide 4-nitroperbenzoïque est ajouté, plus préférablement le mCPBA est ajouté ;
- le solvant est un solvant organique, de préférence CH₂Cl₂, et
- la température est choisie entre -5° C et 90° C, de préférence entre 15° C et 30° C, plus préférablement à la température ambiante ;
et où dans la cétone 1 et dans le composé de formule (I)
n est choisi parmi 0, 1 ou 2 ;
m est choisi parmi 0, 1 ou 2 ; et
m + n est égal à 2;
R₁ est choisi parmi CN, COOR₄, CONR₅R₆ , halogène ou NO₂;
l'un de R₂ et R₃ est choisi parmi CN, COOR₄, CONR₅R₆ , halogène ou NO₂, tandis que l'autre est choisi parmi H, un alkyle substitué ou non substitué, un O-alkyle substitué ou non substitué, ou un O-aryle substitué ou non substitué;
à la condition que
si n est égal à 0, R₂ est H, et si m est égal à 0, R₃ est H ;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué,
dans laquel l'alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} , -C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquel l'aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'}, -C(O)OR_{c}, NR_{c}C(O)R_{c'}, -C(O)NR_{c} R_{c'}, -NR_{c}S(O)₂R_{c'}, =O, -OCH₂CH₂OR_{c} , -NR_{c}C(O)NR_{c'}R_{c",}-S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c},-C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆ -alkyle linéaire ou ramifié, substitué ou non substitué;
de préférence
dans laquelle l'un de R₂ ou R₃ est H,
avec la condition que si R₂ est H, m n'est pas 0 ; ou si R₃ est H, n n'est pas 0;
et/ou
dans laquelle n et m sont égaux à 1.

6. Procédé de production d'un monomère lactone selon la revendication 4, comprenant l'étape finale d'une oxydation de Baeyer-Villiger d'une cétone en monomère lactone de formule (II) et (III) suivant le schéma réactionnel ci-dessous : 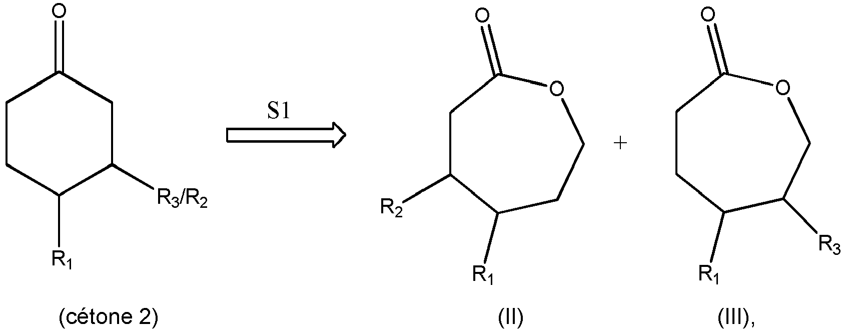 dans laquelle, dans l'étape de réaction S1
- un oxydant est ajouté, de préférence un oxydant choisi parmi le mCPBA, le peroxyde d'hydrogène, le perborate de sodium, l'acide peracétique, l'acide trifluoroperacétique, l'acide performique, l'acide 4-nitroperbenzoïque est ajouté, plus préférablement le mCPBA est ajouté,
- le solvant est un solvant organique, de préférence CH₂Cl₂, et
- la température est choisie entre -5° C et 90° C, de préférence entre 15° C et 30° C, plus préférablement à la température ambiante ;
et où dans la cétone 2 et dans les composés de formules (II) et (III)
R₁ est choisi parmi CN, COOR₄, CONR₅R₆ , halogène ou NO₂;
R₂ est choisi parmi CN, COOR₄, CONR₅R₆, halogène ou NO₂;
R₃ est choisi parmi CN, COOR₄, CONR₅R₆ , halogène ou NO₂;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkylaryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué,
dans laquel l'alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} , -C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquel l'aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'}, -C(O)OR_{c}, NR_{c}C(O)R_{c'}, -C(O)NR_{c} R_{c'}, -NR_{c}S(O)₂R_{c'}, =O, -OCH₂CH₂OR_{c} , -NR_{c}C(O)NR_{c'}R_{c"},-S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c},-C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆ -alkyle linéaire ou ramifié, substitué ou non substitué.

7. Procédé selon la revendication 6, dans lequel la cétone 2 est fournie par une séquence d'une réaction de Diels-Alder (S2) suivie d'un clivage TMS-énol, suivant le schéma de réaction ci-dessous: 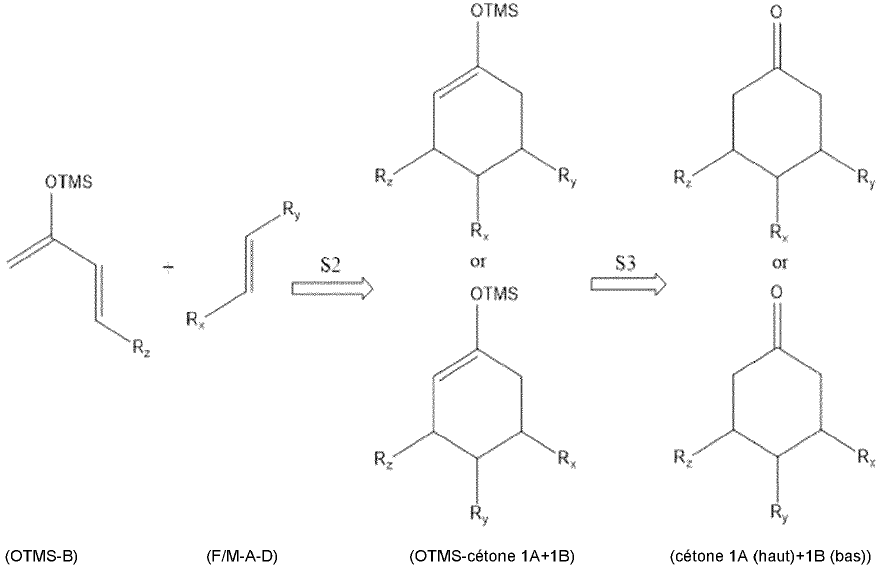
dans laquelle (OTMS-B) est un diène substitué par un 3 ou 2-triméthylsiloxy, de préférence le 2-triméthylsiloxy-1,4-butadiène, (F/M-A-D) est un dérivé d'acide fumarique/maléique (F/M-A-D), de préférence dans laquelle le 2-triméthylsiloxy-1,4-butadiène (OTMS-B) et/ou le dérivé d'acide fumarique/maléique (F/M-A-D) sont synthétisés à partir de produits chimiques, dérivés de pétrole ou de biomasse ;
où
Rₓ est choisi parmi CN, COOR₄, CONR₅R₆, halogène ou NO₂;
R_{y} est choisi parmi CN, COOR₄, CONR₅R₆, halogène ou NO₂;
R_{z} est choisi parmi H, un alkyle substitué ou non substitué ou un O-alkyle substitué ou non substitué, ou un O-aryle substitué ou non substitué
dans laquelle, dans l'étape de réaction S2
- la température est choisie entre 25° C et 180° C, de préférence entre 120°C et 140°C, le plus préférable étant 130°C;
- le temps de réaction est choisi entre 1 heure et 24 heures, de préférence entre 3 heures et 5 heures, le plus préférable étant 4 heures;
dans laquelle, dans l'étape de réaction S3
- acétonitrile, éthanol ou méthanol, acide formique ou carbonate de sodium est ajouté,
- le mélange réactionnel est filtré et évaporé
- et éventuellement le produit final est en outre purifié par une distillation sous vide ou par chromatographie; et
où enfin, lorsque R_{z} est H, la cétone 2 de la revendication 6 est fournie à partir de la cétone 1A dans laquelle Rₓ est R₁ et R_{y} est R₃ /R₂ .

8. Homopolymère de polyester aliphatique fonctionnalisé de formule générale (IV) 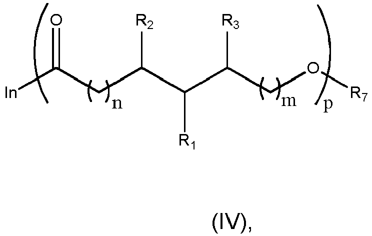 où
n est choisi parmi 0, 1 ou 2;
m est choisi parmi 0, 1 ou 2; et
m + n est égal à 2;
p est choisi parmi 5-500 ;
R₁ est choisi parmi CN, COOR₄, CONR₅R₆, halogène ou NO₂;
l'un de R₂ et R₃ est choisi parmi CN, COOR₄, CONR₅R₆, halogène ou NO₂, tandis que l'autre est choisi parmi H, un alkyle substitué ou non substitué, un O-alkyle substitué ou non substitué, ou un O-aryle substitué ou non substitué;
à la condition que
si n est égal à 0, R₂ est H, et si m est égal à 0, R₃ est H;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué ;
R₇ est choisi parmi H, un acyle aliphatique ou aromatique substitué ou non substitué, un alkyle substitué ou non substitué;
de préférence dans laquelle l'un de R₂ ou R₃ est H,
avec la condition que si R₂ est H, m n'est pas 0; ou si R₃ est H, n n'est pas 0 ; et
In est un alcoxyde aliphatique substitué ou non substitué ou un groupe amino aliphatique primaire ou secondaire substitué ou non substitué, de préférence un éthoxyde,
dans laquel l'alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} , -C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquel l'aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'}, -C(O)OR_{c}, NR_{c}C(O)R_{c'}, -C(O)NR_{c} R_{c'}, -NR_{c}S(O)₂R_{c'}, =O, -OCH₂CH₂OR_{c} , -NR_{c}C(O)NR_{c'}R_{c"},-S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c},-C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆ -alkyle linéaire ou ramifié, substitué ou non substitué.

9. Homopolymère de polyester aliphatique fonctionnalisé selon la revendication 8
a) de formule générale (IVa) 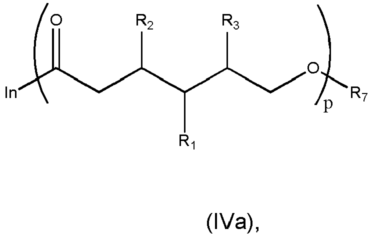 où
p est choisi parmi 5-500 ;
R₁ est choisi parmi CN, COOR₄, CONR₅R₆, halogène ou NO₂;
l'un de R₂ et R₃ est choisi parmi CN, COOR₄, CONR₅R₆, halogène ou NO₂, tandis que l'autre est choisi parmi H, un alkyle substitué ou non substitué, un O-alkyle substitué ou non substitué, ou un O-aryle substitué ou non substitué;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkylaryle substitué ou non substitué, hétérocyclyle substitué ou non substitué, ou alkyl-hétérocyclyle substitué ou non substitué,
alternativement, R₅ et R₆ pris ensemble avec l'atome d'azote auquel ils sont attachés, peuvent former un hétérocyclyle substitué ou non substitué ;
R₇ est choisi parmi H, un acyle aliphatique ou aromatique substitué ou non substitué, un alkyle substitué ou non substitué,
dans laquel l'aliphatique, alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} ,-C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquelle l'aromatique, aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'},-C(O)OR_{c}, NR_{c}C(O)R_{c'}, -C(O)NR_{c} R_{c'}, -NR_{c}S(O)₂R_{c'}, =O, -OCH₂CH₂OR_{c} ,-NR_{c}C(O)NR_{c'}R_{c"}, -S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c}, -C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆-alkyle linéaire ou ramifié, substitué ou non substitué; ou
b) de formule générale (V) ou (VI) 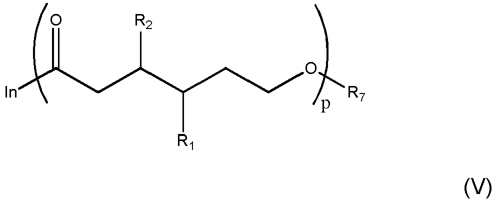 ou 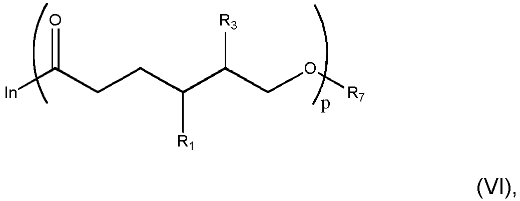 où
p est choisi parmi 5-500 ;
R₁ est choisi parmi CN, COOR₄, CONR₅R₆ , halogène ou NO₂;
R₂ est choisi parmi CN, COOR₄, CONR₅R₆, halogène ou NO₂;
R₃ est choisi parmi CN, COOR₄, CONR₅R₆, halogène ou NO₂;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué;
R₇ est choisi parmi H, un acyle aliphatique ou aromatique substitué ou non substitué, un alkyle substitué ou non substitué,
dans laquel l'aliphatique, alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} ,-C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquelle l'aromatique, aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'},-C(O)OR_{c}, NR_{c}C(O)R_{c'}, -C(O)NR_{c} R_{c'}, -NR_{c}S(O)₂R_{c'}, =O, -OCH₂CH₂OR_{c} ,-NR_{c}C(O)NR_{c'}R_{c"}, -S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c}, -C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆-alkyle linéaire ou ramifié, substitué ou non substitué.

10. Copolymère statistique de polyester aliphatique fonctionnalisé de formule générale (VII) 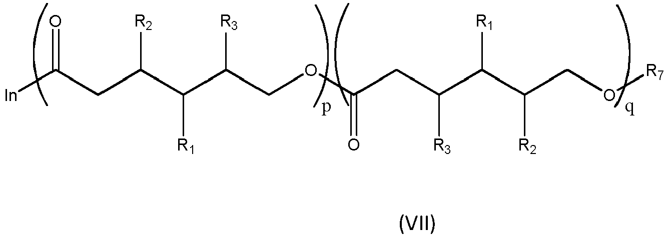
p est choisi parmi 5-500,
q est choisi parmi 5-500,
p+q est de 10-500 ;
R₁ est choisi parmi CN, COOR₄, CONR₅R₆, halogène ou NO₂;
l'un de R₂ et R₃ est choisi parmi CN, COOR₄, CONR₅R₆, halogène ou NO₂, tandis que l'autre est choisi parmi H, un alkyle substitué ou non substitué, un O-alkyle substitué ou non substitué, ou un O-aryle substitué ou non substitué;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué;
R₇ est choisi parmi H, un acyle aliphatique ou aromatique substitué ou non substitué, un alkyle substitué ou non substitué,
dans laquel l'aliphatique, alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} ,-C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquelle l'aromatique, aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'},-C(O)OR_{c}, NR_{c}C(O)R_{c'}, -C(O)NR_{c} R_{c'}, -NR_{c}S(O)₂R_{c'}, =O, -OCH₂CH₂OR_{c} ,-NR_{c}C(O)NR_{c'}R_{c"}, -S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c}, -C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆-alkyle linéaire ou ramifié, substitué ou non substitué.

11. Copolymère statistique et de microstructure aléatoire de polyester aliphatique fonctionnalisé de formule générale (VIII) ou (IX) 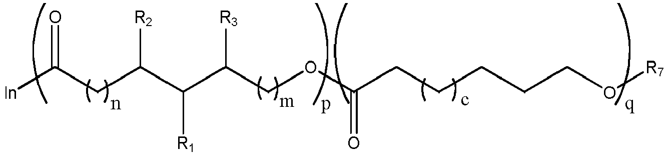 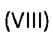 ou 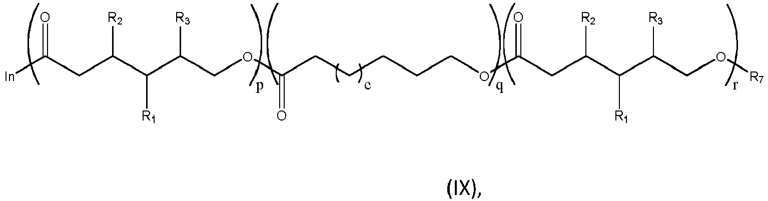 où
n est choisi parmi 0, 1 ou 2,
m est choisi parmi 0, 1 ou 2, et
m + n est égal à 2,
c est choisi parmi 0 ou 1 ;
p est choisi parmi 5-500,
q est choisi parmi 5-500, et
r est choisi parmi 5-500,
p+q+r est de 15-500 ;
R₁ est choisi parmi CN, COOR₄, CONR₅R₆, halogène ou NO₂;
l'un de R₂ et R₃ est choisi parmi CN, COOR₄, CONR₅R₆, halogène ou NO₂; tandis que l'autre est choisi parmi H, un alkyle substitué ou non substitué, un O-alkyle substitué ou non substitué, ou un O-aryle substitué ou non substitué;
à la condition que
si n est égal à 0, R₂ est H, et si m est égal à 0, R₃ est H ;
R₄ est choisi parmi un alkyle substitué ou non substitué, un alcényle substitué ou non substitué, un alcyn substitué ou non substitué, un aryle substitué ou non substitué, ou un alkyl-aryle substitué ou non substitué;
R₅ et R₆ sont choisis indépendamment l'un de l'autre parmi un alkyle substitué ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué ;
de préférence dans laquelle l'un de R₂ ou R₃ est H,
avec la condition que si R₂ est H, m n'est pas 0 ; ou si R₃ est H, n n'est pas 0;
R₇ est choisi parmi H, un acyle aliphatique ou aromatique substitué ou non substitué, un alkyle substitué ou non substitué,
dans laquel l'aliphatique, alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} , - C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquelle l'aromatique, aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'},-C(O)OR_{c}, NR_{c}C(O)R_{c'}, -C(O)NR_{c} R_{c'}, -NR_{c}S(O)₂R_{c'}, =O, -OCH₂CH₂OR_{c} ,-NR_{c}C(O)NR_{c'}R_{c"}, -S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c}, -C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆-alkyle linéaire ou ramifié, substitué ou non substitué.

12. Copolymère statistique et de microstructure aléatoire de polyester aliphatique fonctionnalisé selon la revendication 11
a) de formule générale (VIIIa) ou (IXa) 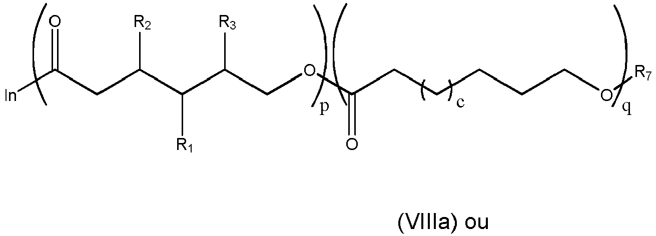 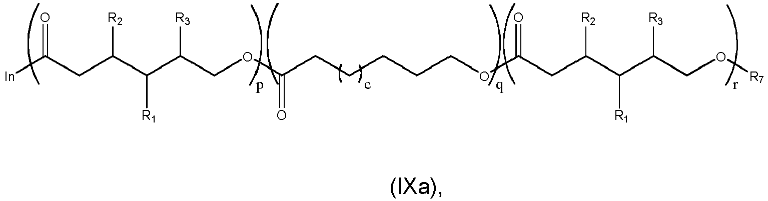 où
c est choisi parmi 0 ou 1,
p est choisi parmi 5-500,
q est choisi parmi 5-500, et
r est choisi parmi 5-500,
p+q+r est de 15-500 ;
R₁ est choisi parmi CN, COOR₄, CONR₅R₆, halogène ou NO₂;
l'un de R₂ et R₃ est choisi parmi CN, COOR₄, CONR₅R₆, un halogène ou NO₂, tandis que l'autre est choisi parmi H, un alkyle substitué ou non substitué, un O-alkyle substitué ou non substitué, ou un O-aryle substitué ou non substitué;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitués ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué ;
R₇ est choisi parmi H, un acyle aliphatique ou aromatique substitué ou non substitué, un alkyle substitué ou non substitué,
dans laquel l'aliphatique, alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} ,-C(O)OR_{c}, -CN, -C(O)NR_{c}R_{c'}, ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquelle l'aromatique, aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'},-C(O)OR_{c}, NR_{c}C(O)R_{c'}, -C(O)NR_{c} R_{c'}, -NR_{c}S(O)₂R_{c'}, =O, -OCH₂CH₂OR_{c} ,-NR_{c}C(O)NR_{c'}R_{c"}, -S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c}, -C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆-alkyle linéaire ou ramifié, substitué ou non substitué;
ou
b) de formule générale (X) ou (XI) et (XII) ou (XIII) 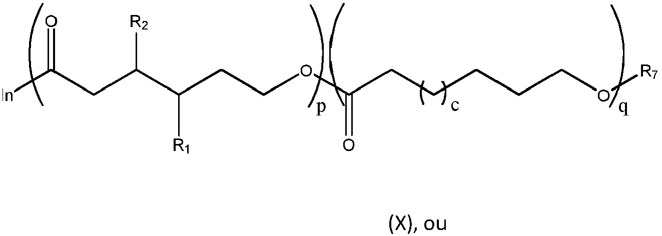 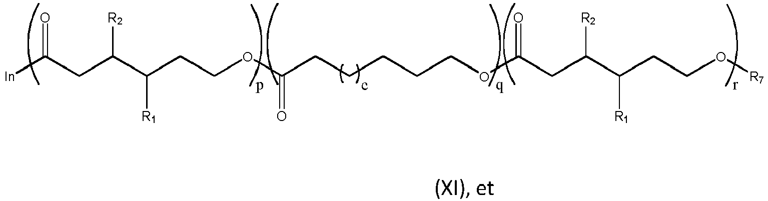 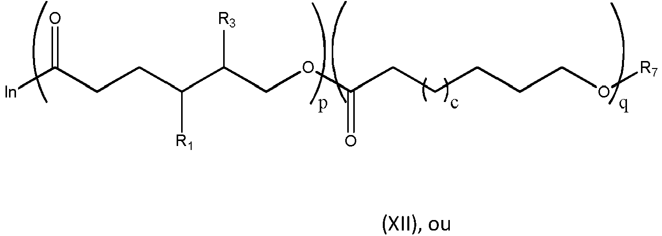 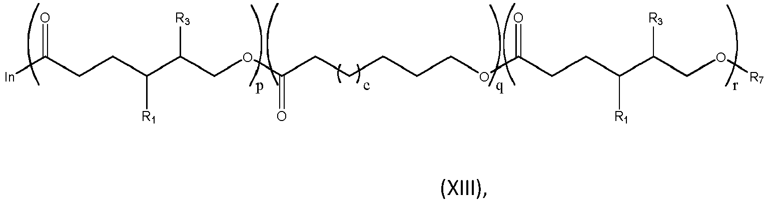 où
c est choisi parmi 0 ou 1 ;
p est choisi parmi 5-500,
q est choisi parmi 5-500, et
r est choisi parmi 5-500,
p+q+r est de 15-500,
R₁ est choisi parmi CN, COOR₄, CONR₅R₆, halogène ou NO₂;
R₂ est choisi parmi CN, COOR₄, CONR₅R₆, halogène ou NO₂;
R₃ est choisi parmi CN, COOR₄, CONR₅R₆ , halogène ou NO₂;
R₄ est choisi parmi un alkyle substitué ou non substitué, un alcényle substitué ou non substitué, un alcynyle substitué ou non substitué, un aryle substitué ou non substitué, ou un alkyl-aryle substitué ou non substitué;
R₅ et R₆ sont choisis indépendamment l'un de l'autre parmi un alkyle substitués ou non substitué, alcényle substitué ou non substitué, alcynyle substitué ou non substitué, aryle substitué ou non substitué, alkyl-aryle substitué ou non substitué, hétérocyclyle substitué ou non substitué, ou alkyl-hétérocyclyle substitué ou non substitué, alternativement, R₅ et R₆ pris ensemble avec l'atome d'azote auquel ils sont attachés, peuvent former un hétérocyclyle substitué ou non substitué ;
R₇ est choisi parmi H, un acyle aliphatique ou aromatique substitué ou non substitué, un alkyle substitué ou non substitué,
dans laquel l'aliphatique, alkyle, alcényle, alcynyle et O-alkyle s'il est substitué, ainsi que alkylaryle, alkylhétérocyclique, alkylcycloalkyle s'il est substitué sur le groupe alkyle, est substitué par un halogène (F, Cl, Br, I), -NR_{c}R_{c'} , -SR_{c} , -S(O)R_{c}, -S(O)₂ R_{c} , -OR_{c} ,-C(O)OR_{c}, -CN, -C(O)NR_{c}R_{c',} ou un groupe -OC₁₋₆ alkyle linéaire ou ramifié, substitué ou non substitué, avec R_{c} et R_{c'} étant représentés par H ou par un groupe C₁₋₆ alkyle linéaire ou ramifié, substitué ou non substitué; et
dans laquelle l'aromatique, aryle, cycloalkyle ou hétérocyclyle, s'il est substitué, ainsi que alkyl-aryle, alkyl-cycloalkyle ou alkyl-hétérocyclyle, s'il est substitué sur le système cyclique, est substitué par un halogène (F, Cl, Br, I), -R_{c} ,-OR_{c} , -CN, -NO₂, -NR_{c} R_{c'},-C(O)OR_{c}, NR_{c}C(O)R_{c'}, -C(O)NR_{c} R_{c'}, -NR_{c}S(O)₂R_{c'}, =O, -OCH₂CH₂OR_{c} ,-NR_{c}C(O)NR_{c'}R_{c",}-S(O)₂NR_{c}R_{c'}, -NR_{c}S(O)₂NR_{c'}R_{c"}, haloalkyle, haloalcoxy, -SR_{c}, -S(O)R_{c}, -S(O)₂R_{c}, -C(CH₃)OR_{c}; NR_{c} R_{c'}, ou un groupe -OC₁₋₆ alkyle linéaire ou ramifié, substitué ou non substitué, avec R_{c} , R_{c'} et R_{c"} étant représentés par H ou par un groupe C₁₋₆-alkyle linéaire ou ramifié, substitué ou non substitué.

13. Monomère de lactone selon l'une quelconque des revendications 1 à 3, homopolymère de polyester aliphatique fonctionnalisé selon l'une quelconque des revendications 8 et 9 (option a)), copolymère statistique de polyester aliphatique fonctionnalisé selon la revendication 10, ou copolymère statistique et de microstructure aléatoire de polyester aliphatique fonctionnalisé selon l'une quelconque des revendications 11 et 12 (option a)), où
R₄ est choisi parmi un alkyle en C₁₋₁₀ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₁₀ substitué ou non substitué, aryle substitué ou non substitué, alkylaryle en C₁₋₄ substitué ou non substitué, hétérocyclyle mono- ou bi-cyclique substitué ou non substitué, ou alkyl-hétérocyclyle en C₁₋₄ mono- ou bi-cyclique substitué ou non substitué, alternativement, R₅ et R₆ pris ensemble avec l'atome d'azote auquel ils sont attachés, peuvent former un hétérocyclyle mono- ou bi-cyclique substitué ou non substitué ;
de préférence dans laquelle
R₄ est choisi parmi un alkyle en C₁₋₄ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₄ substitué ou non substitué, phényle substitué ou non substitué, benzyle substitué ou non substitué ou hétérocyclyle mono- ou bi-cyclique substitué ou non substitué,
alternativement, R₅ et R₆ pris ensemble avec l'atome d'azote auquel ils sont attachés, peuvent former une morpholine substituée ou non substituée ;
plus préférablement dans lequel
R₄ est choisi parmi un alkyle en C₁₋₄ non substitué, phényle substitué ou non substitué ou benzyle substitué ou non substitué;
R₅ et R₆ sont choisis indépendamment l'un de l'autre parmi un alkyle en C₁₋₄ substitué ou non substitué, phényle substitué ou non substitué ou benzyle substitué ou non substitué, alternativement, R₅ et R₆ pris ensemble avec l'atome d'azote auquel ils sont attachés, peuvent former une morpholine non substituée;
et/ou
dans lequel dans un homopolymère de polyester aliphatique fonctionnalisé selon l'une quelconque des revendications 8 et 9 (option a)), copolymère statistique de polyester aliphatique fonctionnalisé selon la revendication 10, ou copolymère statistique et de microstructure aléatoire de polyester aliphatique fonctionnalisé selon l'une quelconque des revendications 11 et 12 (option a))
R₇ est choisi parmi H, un acyle aliphatique ou aromatique substitué ou non substitué, un alkyle substitué ou non substitué ;
de préférence, dans un homopolymère de polyester aliphatique fonctionnalisé selon l'une quelconque des revendications 8 et 9 (option a)), copolymère statistique de polyester aliphatique fonctionnalisé selon la revendication 10, ou copolymère statistique et de microstructure aléatoire de polyester aliphatique fonctionnalisé selon l'une quelconque des revendications 11 et 12 (option a))
R₇ est choisi parmi H, acétyle ou alkyle non substitué ;
plus préférablement dans lequel, dans un homopolymère de polyester aliphatique fonctionnalisé selon l'une quelconque des revendications 8 et 9 (option a)), copolymère statistique de polyester aliphatique fonctionnalisé selon la revendication 10, ou copolymère statistique et de microstructure aléatoire de polyester aliphatique fonctionnalisé selon l'une quelconque des revendications 11 et 12 (option a))
R₇ est H.

14. Monomère lactone selon l'une quelconque des revendications 1 à 3 et 13, homopolymère de polyester aliphatique fonctionnalisé selon l'une quelconque des revendications 8, 9 (option a)) et 13, copolymère statistique de polyester aliphatique fonctionnalisé selon l'une quelconque des revendications 10 ou 13, ou copolymère statistique et de microstructure aléatoire de polyester aliphatique fonctionnalisé selon l'une quelconque des revendications 11, 12 (option a)) et 13, où
R₁ est choisi parmi CN, COOR₄, CONR₅R₆; et
R₂ est choisi parmi CN, COOR₄, CONR₅R₆, tandis que R₃ est choisi parmi H, un alkyle substitué ou non substitué, un O-alkyle substitué ou non substitué ou un O-aryle substitué ou non substitué ; ou
R₃ est choisi parmi CN, COOR₄, CONR₅R₆, tandis que R₂ est choisi parmi H, un alkyle substitué ou non substitué, un O-alkyle substitué ou non substitué ou un O-aryle substitué ou non substitué;
de préférence dans laquelle
R₁ est choisi parmi CN, COOR₄, CONR₅R₆; et
R₂ est choisi parmi CN, COOR₄, CONR₅R₆, tandis que R₃ est choisi parmi H, un alkyle en C₁₋₄ substitué ou non substitué, un O-alkyle en C₁₋₄ substitué ou non substitué ou un O-aryle substitué ou non substitué; ou
R₃ est choisi parmi CN, COOR₄, CONR₅R₆, tandis que R₂ est choisi parmi H, un alkyle en C₁₋₄ substitué ou non substitué, un O-alkyle en C₁₋₄ substitué ou non substitué ou un O-aryle substitué ou non substitué;
plus préférablement dans lequel
R₁ est choisi parmi CN, COOR₄, CONR₅R₆; et
R₂ est choisi parmi CN, COOR₄, CONR₅R₆ , tandis que R₃ est choisi parmi H, un alkyle en C₁₋₄ non substitué, un O-alkyle en C₁₋₄ non substitué ou un O-phényle substitué ou non substitué; ou
R₃ est choisi parmi CN, COOR₄, CONR₅R₆, tandis que R₂ est choisi parmi H, un alkyle en C₁₋₄ substitué ou non substitué ou un O-alkyle en C₁₋₄ substitué ou non substitué ou un O-phényle substitué ou non substitué;
de préférence dans laquelle
R₁ est choisi parmi CN, COOR₄, CONR₅R₆; et
R₂ est choisi parmi CN, COOR₄, CONR₅R₆ , tandis que R₃ est H; ou
R₃ est choisi parmi CN, COOR₄, CONR₅R₆, tandis que R₂ est H.

15. Monomère de lactone selon la revendication 4, homopolymère de polyester aliphatique fonctionnalisé selon l'une quelconque des revendication 9 (option b)), copolymère statistique de polyester aliphatique fonctionnalisé selon la revendication 10, ou copolymère statistique et de microstructure aléatoire de polyester aliphatique fonctionnalisé selon l'une quelconque des revendication 12 (option b)), où
R₄ est choisi parmi un alkyle en C₁₋₁₀ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₁₀ substitués ou non substitué, aryle substitué ou non substitué, alkylaryle en C₁₋₄ substitué ou non substitué, hétérocyclyle mono- ou bi-cyclique substitué ou non substitué, ou alkyl-hétérocyclyle en C₁₋₄ mono- ou bi-cyclique substitué ou non substitué, alternativement, R₅ et R₆ pris ensemble avec l'atome d'azote auquel ils sont attachés, peuvent former un hétérocyclyle mono- ou bi-cyclique substitué ou non substitué ;
de préférence dans laquelle
R₄ est choisi parmi un alkyle en C₁₋₄ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₄ substitué ou non substitué, phényle substitué ou non substitué, benzyle substitué ou non substitué ou hétérocyclyle mono- ou bi-cyclique substitué ou non substitué,
alternativement, R₅ et R₆ pris ensemble avec l'atome d'azote auquel ils sont attachés, peuvent former une morpholine substituée ou non substituée;
plus préférablement dans lequel
R₄ est choisi parmi les groupes alkyle en C₁₋₄ non substitués, phényle substitués ou non substitués ou benzyle substitués ou non substitués ;
R₅ et R₆ sont choisis indépendamment l'un de l'autre parmi les groupes alkyle en C₁₋₄ substitués ou non substitués, phényle substitué ou non substitué ou benzyle substitué ou non substitué,
alternativement, R₅ et R₆ pris ensemble avec l'atome d'azote auquel ils sont attachés, peuvent former une morpholine non substituée;
et/ou
où
R₁ est choisi parmi CN, COOR₄, CONR₅R₆;
R₂ est choisi parmi CN, COOR₄, CONR₅R₆, et
R₃ est choisi parmi CN, COOR₄, CONR₅R₆
et/ou
dans lequel dans un homopolymère de polyester aliphatique fonctionnalisé selon la revendication 9 (option b)), dans un copolymère statistique de polyester aliphatique fonctionnalisé selon la revendication 10, ou dans un copolymère statistique et de microstructure aléatoire de polyester aliphatique fonctionnalisé selon la revendication 12 (option b))
R₇ est choisi parmi H, un acyle aliphatique ou aromatique substitué ou non substitué, un alkyle substitué ou non substitué;
de préférence dans un homopolymère de polyester aliphatique fonctionnalisé selon la revendication 9 (option b)), dans un copolymère statistique de polyester aliphatique fonctionnalisé selon la revendication 10, ou dans un copolymère statistique et de microstructure aléatoire de polyester aliphatique fonctionnalisé selon la revendication 12 (option b))
R₇ est choisi parmi H, acétyle ou alkyle non substitué;
plus préférablement dans un homopolymère de polyester aliphatique fonctionnalisé selon la revendication 9 (option b)), dans un copolymère statistique de polyester aliphatique fonctionnalisé selon la revendication 10, ou dans un copolymère statistique et de microstructure aléatoire de polyester aliphatique fonctionnalisé selon la revendication 12 (option b))
R₇ est H.

16. Procédé de ROP pour la production d'un homopolymère de polyester aliphatique fonctionnalisé selon l'une quelconque des revendications 8 ou 9, un copolymère statistique de polyester aliphatique selon la revendication 10 ou un copolymère statistique et de microstructure aléatoire de polyester aliphatique fonctionnalisé selon les revendications 11 ou 12, dans lequel le ROP est exécuté
en présence d'un catalyseur basique, acide, à base d'étain ou d'aluminium ; de préférence en présence d'un catalyseur à base d'alkyle et d'alkoxyde d'aluminium; plus préférablement en présence d'un catalyseur à base d'alkyle et d'alkoxyde d'aluminium choisi parmi l'éthoxyde de diéthylaluminium, le bis(2,6-di-tert-butyl-4-méthylphénolate) de méthylaluminium ou le bis(2,6-di-phénylphénolate) de méthylaluminium; plus préférablement en présence du bis(2,6-di-phénylphénolate) de méthylaluminium,
de préférence
en présence d'un alcool primaire comme initiateur; plus préférablement en présence d'éthanol comme initiateur.

17. Procédé selon la revendication 5, dans lequel
R₄ est choisi parmi un alkyle en C₁₋₁₀ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₁₀ substitué ou non substitué, aryle substitué ou non substitué, alkylaryle en C₁₋₄ substitué ou non substitué, hétérocyclyle mono- ou bi-cyclique substitué ou non substitué, ou alkyl-hétérocyclyle en C₁₋₄ mono- ou bi-cyclique substitué ou non substitué,
alternativement, R₅ et R₆ pris ensemble avec l'atome d'azote auquel ils sont attachés, peuvent former un hétérocyclyle mono- ou bi-cyclique substitué ou non substitué ;
de préférence dans laquelle
R₄ est choisi parmi un alkyle en C₁₋₄ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₄ substitué ou non substitué, phényle substitué ou non substitué, benzyle substitué ou non substitué ou hétérocyclyle mono- ou bi-cyclique substitué ou non substitué,
alternativement, R₅ et R₆ pris ensemble avec l'atome d'azote auquel ils sont attachés, peuvent former une morpholine substituée ou non substituée ;
plus préférablement dans lequel
R₄ est choisi parmi un alkyle en C₁₋₄ non substitué, phényle substitué ou non substitué ou benzyle substitué ou non substitué;
R₅ et R₆ sont choisis indépendamment l'un de l'autre parmi un alkyle en C₁₋₄ substitué ou non substitué, phényle substitué ou non substitué ou benzyle substitué ou non substitué, alternativement, R₅ et R₆ pris ensemble avec l'atome d'azote auquel ils sont attachés, peuvent former une morpholine non substituée ;
et/ou
dans lequel, dans un homopolymère de polyester aliphatique fonctionnalisé selon l'une quelconque des revendications 8 et 9 (option a)), dans un copolymère statistique de polyester aliphatique fonctionnalisé selon la revendication 10, ou dans un copolymère statistique et de microstructure aléatoire de polyester aliphatique fonctionnalisé selon l'une quelconque des revendications 11 et 12 (option a))
R₇ est choisi parmi H, un acyle aliphatique ou aromatique substitué ou non substitué, un alkyle substitué ou non substitué ;
de préférence dans laquelle un homopolymère de polyester aliphatique fonctionnalisé selon l'une quelconque des revendications 8 et 9 (option a)), copolymère statistique de polyester aliphatique fonctionnalisé selon la revendication 10, ou un copolymère statistique et de microstructure aléatoire de polyester aliphatique fonctionnalisé selon l'une quelconque des revendications 11 et 12 (option a))
R₇ est choisi parmi H, acétyle ou alkyle non substitué ;
plus préférablement dans lequel, dans un homopolymère de polyester aliphatique fonctionnalisé selon l'une quelconque des revendications 8 et 9 (option a)), copolymère statistique de polyester aliphatique fonctionnalisé selon la revendication 10, ou un copolymère statistique et de microstructure aléatoire de polyester aliphatique fonctionnalisé selon l'une quelconque des revendications 11 et 12 (option a))
R₇ est H.

18. Procédé selon l'une quelconque des revendications 5 et 13, dans lequel
R₁ est choisi parmi CN, COOR₄, CONR₅R₆; et
R₂ est choisi parmi CN, COOR₄, CONR₅R₆, tandis que R₃ est choisi parmi H, un alkyle substitué ou non substitué, un O-alkyle substitué ou non substitué ou un O-aryle substitué ou non substitué; ou
R₃ est choisi parmi CN, COOR₄, CONR₅R₆, tandis que R₂ est choisi parmi H, un alkyle substitué ou non substitué, un O-alkyle substitué ou non substitué ou un O-aryle substitué ou non substitué;
de préférence dans laquelle
R₁ est choisi parmi CN, COOR₄, CONR₅R₆; et
R₂ est choisi parmi CN, COOR₄, CONR₅R₆, tandis que R₃ est choisi parmi H, un alkyle en C₁₋₄ substitué ou non substitué, un O-alkyle en C₁₋₄ substitué ou non substitué ou un O-aryle substitué ou non substitué; ou
R₃ est choisi parmi CN, COOR₄, CONR₅R₆, tandis que R₂ est choisi parmi H, un alkyle en C₁₋₄ substitué ou non substitué, un O-alkyle en C₁₋₄ substitué substitué ou non ou un O-aryle substitué ou non substitué;
plus préférablement dans lequel
R₁ est choisi parmi CN, COOR₄, CONR₅R₆; et
R₂ est choisi parmi CN, COOR₄, CONR₅R₆, tandis que R₃ est choisi parmi H, un alkyle en C₁₋₄ non substitué, un O-alkyle en C₁₋₄ non substitué ou un O-phényle substitué ou non substitué; ou
R₃ est choisi parmi CN, COOR₄, CONR₅R₆, tandis que R₂ est choisi parmi H, un alkyle en C₁₋₄ substitué ou non substitué ou un O-alkyle en C₁₋₄ substitué ou non substitué ou un O-phényle substitué ou non substitué;
de préférence dans laquelle
R₁ est choisi parmi CN, COOR₄, CONR₅R₆; et
R₂ est choisi parmi CN, COOR₄, CONR₅R₆, tandis que R₃ est H ; ou
R₃ est choisi parmi CN, COOR₄, CONR₅R₆, tandis que R₂ est H.

19. Procédé selon l'une quelconque des revendications 7 ou 8, dans lequel
R₄ est choisi parmi un alkyle en C₁₋₁₀ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₁₀ substitué ou non substitué, aryle substitué ou non substitué, alkylaryle en C₁₋₄ substitué ou non substitué, hétérocyclyle mono- ou bi-cyclique substitué ou non substitué, ou alkyl-hétérocyclyle en C₁₋₄ mono- ou bi-cyclique substitué ou non substitué, alternativement, R₅ et R₆ pris ensemble avec l'atome d'azote auquel ils sont attachés, peuvent former un hétérocyclyle mono- ou bi-cyclique substitué ou non substitué;
de préférence dans laquelle
R₄ est choisi parmi un alkyle en C₁₋₄ substitué ou non substitué, aryle substitué ou non substitué, ou alkylaryle en C₁₋₄ substitué ou non substitué;
R₅ et R₆ sont choisis indépendamment l'un de l'autre parmi un alkyle en C₁₋₄ substitué ou non substitué, phényle substitué ou non substitué, benzyle substitué ou non substitué ou hétérocyclyle mono- ou bi-cyclique substitué ou non substitué,
alternativement, R₅ et R₆ pris ensemble avec l'atome d'azote auquel ils sont attachés, peuvent former une morpholine substituée ou non substituée;
plus préférablement dans lequel
R₄ est choisi parmi un alkyle en C₁₋₄ non substitué, phényle substitué ou non substitué ou benzyle substitué ou non substitué;
R₅ et R₆ sont choisis indépendamment l'un de l'autre parmi un alkyle en C₁₋₄ substitué ou non substitué, phényle substitué ou non substitué ou benzyle substitué ou non substitué, alternativement, R₅ et R₆ pris ensemble avec l'atome d'azote auquel ils sont attachés, peuvent former une morpholine non substituée ;
et/ou
où
R₁ est choisi parmi CN, COOR₄, CONR₅R₆;
R₂ est choisi parmi CN, COOR₄, CONR₅R₆, et
R₃ est choisi parmi CN, COOR₄, CONR₅R₆
et/ou
dans lequel dans un homopolymère de polyester aliphatique fonctionnalisé selon la revendication 9 (option b)), dans un copolymère statistique de polyester aliphatique fonctionnalisé selon la revendication 10, ou dans un copolymère statistique et de microstructure aléatoire de polyester aliphatique fonctionnalisé selon la revendication 12 (option b))
R₇ est choisi parmi H, un acyle aliphatique ou aromatique substitué ou non substitué, un alkyle substitué ou non substitué;
de préférence dans un homopolymère de polyester aliphatique fonctionnalisé selon la revendication 9 (option b)), dans un copolymère statistique de polyester aliphatique fonctionnalisé selon la revendication 10, ou dans un copolymère statistique et de microstructure aléatoire de polyester aliphatique fonctionnalisé selon la revendication 12 (option b))
R₇ est choisi parmi H, acétyle ou alkyle non substitué;
plus préférablement dans un homopolymère de polyester aliphatique fonctionnalisé selon la revendication 9 (option b)), dans un copolymère statistique de polyester aliphatique fonctionnalisé selon la revendication 10, ou dans un copolymère statistique et de microstructure aléatoire de polyester aliphatique fonctionnalisé selon la revendication 12 (option b))
R₇ est H.
